(19) Europäisches Patentamt
European Patent Office
Office européen des brevets

(11) EP 1 149 583 A2

(12) EUROPEAN PATENT APPLICATION

(43) Date of publication:
**31.10.2001 Bulletin 2001/44**

(51) Int Cl.[7]: **A61K 38/27**, A61K 45/06, A61P 9/00, A61P 19/10

(21) Application number: **01303033.3**

(22) Date of filing: **30.03.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.04.2000 US 196698 P**

(71) Applicant: **Pfizer Products Inc.**
**Groton, Connecticut 06340 (US)**

(72) Inventor: **Fossa, Anthony A.**
**Pfizer Global Research and Dev.**
**Groton, Connecticut 06340 (US)**

(74) Representative: **Ruddock, Keith Stephen et al**
**Pfizer Limited,**
**European Patent Department,**
**Ramsgate Road**
**Sandwich, Kent CT13 9NJ (GB)**

(54) **Combinations of corticotropin releasing factor antagonists and growth hormone secretagogues**

(57) This invention is directed to pharmaceutical compositions comprising corticotropin releasing factor antagonist and growth hormone or growth hormone secretagogues, prodrugs thereof, or pharmaceutically acceptable salts of said compounds or said prodrugs. The invention is also directed to the use of such compositions in the treatment or prevention of osteoporosis and heart-related diseases (including congestive heart failure) in mammals, particularly humans.

EP 1 149 583 A2

## Description

BACKGROUND OF THE INVENTION

This invention relates to pharmaceutical compositions comprising combinations of corticotropin releasing factor (CRF) antagonists and growth hormone or growth hormone secretagogues, prodrugs thereof, and pharmaceutically acceptable salts of said compounds and said prodrugs. These compositions have utility, *inter alia,* in the treatment of osteoporosis or frailty associated with aging or obesity, in the treatment of cardiovascular or heart related diseases including hypertension, tachycardia, and in particular congestive heart failure, as well as in accelerating bone fracture repair, attenuating protein catabolic response after a major operation, reducing cachexia and protein loss due to chronic illness, accelerating wound healing or accelerating the recovery of bum patients or of patients having undergone major surgery. These utilities are most relevant to mammals, and particularly to humans. Accordingly, this invention also relates to methods of using such compositions for the treatment of the above diseases in mammals, particularly humans.

CRF antagonists are disclosed in U.S. Patents 4,605,642 and 5,063,245. Other CRF antagonists are disclosed in International patent publications WO 95/33750; WO 95/34563; WO 94/13661; WO 94/13644; WO 94/13643; WO 94/13676; WO 94/13677; WO 95/33727; WO 98/05661; WO 98/08847; WO 98/08846; and European patent publications EP 778277 and EP 773023. Yet other CRF antagonists are disclosed in the following patent publications: EP 576350; EP 659747; EP 812831; WO 95/10506; WO 96/35689; WO 96/39400; WO 97/00868; WO 97/14684; WO 97/29109; WO 97/29110; WO 97/35539; WO 97/35580; WO 97/35846; WO 97/44038; WO 97/45421; WO 98/03510; WO 98/08821; WO 98/11075; WO 98/15543; WO 98/21200; WO 98/27066; WO 98/29397; WO 98/29413; WO 98/42699; WO 98/35967; WO 98/42706; WO 98/45295; WO 98/47874; WO 98/47903; WO 98/51312; WO 99/01454; WO 99/01439; WO 99/10350; WO 99/12908; WO 99/00373; WO 99/38868; WO 99/51597; WO 99/51599; WO 99/40089; WO 99/51598; and WO 99/51600. Still more CRF antagonists are disclosed in United States Patents 5,109,111; 5,132,111; 5,245,009; 5,464,847; 5,493,006; 5,510,458; 5,644,057; 5,663,292; 5,668,145; 5,705,646; 5,712,303; and 5,723,608. An overview of the patent literature on CRF antagonists is provided in T.E. Christos and A. Arvanitis, Exp. Opin. Ther. Patents (1998) 8(2):143-152. Many of the above cited publications include information on how to make the CRF antagonists described therein.

The importance of CRF antagonists is set out in the literature, e.g., P. Black, Scientific American: "Science & Medicine," 1995, 2:16-25; T. Lovenberg, et al., Current Pharmaceutical Design, 1995, 1: 305-316; D.T. Chalmers et al., Trends in Pharmacological Sciences, April 1996, pages 166-172; and United States Patent 5,063,245. An outline of the activities possessed by CRF antagonists is found in M. J. Owens et al., 1991, Pharm. Rev., 43:425-473. CRF antagonists are described in the art as being effective in the treatment of stress-related illnesses, mood disorders such as depression, major depressive disorder, single episode depression, recurrent depression, child abuse induced depression, postpartum depression, dysthemia, bipolar disorders, and cyclothymia; chronic fatigue syndrome; eating disorders such as anorexia and bulimia nervosa; generalized anxiety disorder; panic disorder; phobias; obsessive-compulsive disorder; post-traumatic stress disorder; pain perception such as fibromyalgia; headache; gastrointestinal diseases; hemorrhagic stress; ulcers; stress-induced psychotic episodes; fever; diarrhea; post-operative ileus; colonic hypersensitivity; irritable bowel syndrome; Crohn's disease; spastic colon; inflammatory disorders such as rheumatoid arthritis and osteoarthritis; pain; asthma; psoriasis; allergies; osteoporosis; premature birth; hypertension, congestive heart failure; sleep disorders; neurodegenerative diseases such as Alzheimer's disease, senile dementia of the Alzheimer's type, multiinfarct dementia, Parkinson's disease, and Huntington's disease; head trauma; ischemic neuronal damage; excitotoxic neuronal damage; epilepsy; stroke; spinal cord trauma; psychosocial dwarfism; euthyroid sick syndrome; syndrome of inappropriate antidiuretic hormone; obesity; chemical dependencies and addictions; drug and alcohol withdrawal symptoms; infertility; cancer; muscular spasms; urinary incontinence; hypoglycemia and immune dysfunctions including stress induced immune dysfunctions, immune suppression and human immunodeficiency virus infections; and stress-induced infections in humans and animals.

PCT publication WO 97/24369, which is incorporated herein by reference, discloses growth hormone secretagogues of formula III:

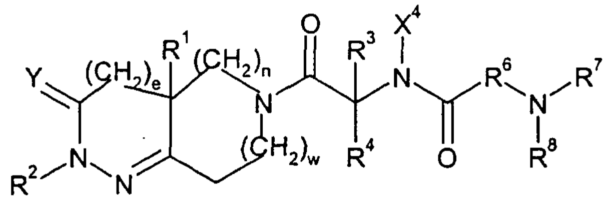

III

wherein the variables are as defined in WO 97/24369.

PCT publication WO 98/58947, which is incorporated herein by reference, discloses growth hormone secretagogues of formula IV:

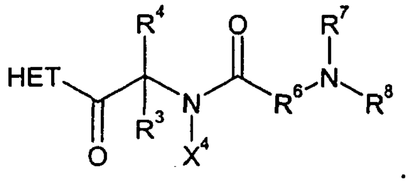

IV

wherein the variables are as defined in WO 98/58947.

Other growth hormones and growth hormone secretagogues that can be used to treat the disorders recited in the methods and compositions of this invention are referred to in PCT international patent application numbers PCT/US97/07516 (published as WO 97/41879) and PCT/DK98/00249 (published as WO 98/58950), as well as in United States patents 5,206,235; 5,283,241; and 5,492,916. Many of the above-cited publications disclose how to make or obtain the growth hormone or growth hormone secretagogue described therein. All of the above-cited patent applications and United States patents are incorporated herein by reference in their entirety. Any growth hormone and growth hormone secretagogue, either presently known or yet to be discovered, may be used in the present invention.

SUMMARY

This invention is directed to pharmaceutical compositions comprising a CRF antagonist, a growth hormone secretagogue or growth hormone, and preferably additionally a pharmaceutically acceptable carrier, vehicle, or diluent.

This invention is also directed to methods for treating or preventing osteoporosis or frailty associated with aging or obesity, cardiovascular or heart related disease, in particular hypertension, tachycardia, and congestive heart failure, accelerating bone fracture repair, attenuating protein catabolic response after a major operation, reducing cachexia and protein loss due to chronic illness, accelerating wound healing, or accelerating the recovery of bum patients or of patients having undergone major surgery, wherein said methods comprise administering to a human or other mammal an amount of a pharmaceutical composition as defined herein, which is effective in treating or preventing the stated disease or condition. This invention is also directed to methods for treating or preventing the diseases or conditions described herein by the co-administration of two separate pharmaceutical compositions. In this latter embodiment, a first composition comprises a CRF antagonist, and a second composition comprises a growth hormone or growth hormone secretagogue. These first and second compositions are preferably co-administered either simultaneously, or in a specifically timed manner.

This invention is also directed to kits comprising a) an amount of a CRF antagonist, in a first unit dosage form; b) an amount of a growth hormone secretagogue or growth hormone in a second unit dosage form; and c) a container.

This invention is also directed to kits comprising a) a pharmaceutical composition comprising an amount of a growth hormone or growth hormone secretagogue, b) a package containing the above composition, and c) a package insert (which may be integral with the package), wherein it is stated on the package insert that the pharmaceutical composition is to be administered simultaneously or in a specifically timed manner with a separate pharmaceutical composition containing at least one CRF antagonist.

This invention is also directed to kits, comprising a) a pharmaceutical composition comprising an amount of a CRF antagonist, b) a package containing the above composition, and c) a package insert that may be integral with the package, wherein it is stated on the package insert that the pharmaceutical composition is to be administered simultaneously or in a specifically timed manner with a pharmaceutical composition containing at least one growth hormone or growth hormone secretagogue.

A group of preferred CRF antagonists for use in the compositions, methods, and kits of the present invention are those wherein the CRF antagonist is a compound of formula:

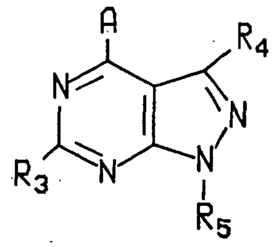

or a pharmaceutically acceptable acid addition salt thereof, wherein

A is $NR_1R_2$, $CR_1R_2R_{11}$, or $C(=CR_1R_{12})R_2$, $NHCR_1R_2R_{11}$, $OCR_1R_2R_{11}$, $SCR_1R_2R_{11}$, $NHNR_1R_2$, $CR_2R_{11}NHR_1$, $CR_2R_{11}OR_1$, $CR_2R_{11}SR_1$ or $C(O)R_2$;

$R_1$ is hydrogen, or $C_1$-$C_6$ alkyl which may be substituted by one or two substituents $R_6$ independently selected from the group consisting of hydroxy, fluoro, chloro, bromo, iodo, $C_1$-$C_6$ alkoxy, O-C(O)-($C_1$-$C_6$ alkyl), O-C(O)-N($C_1$-$C_4$ alkyl)($C_1$-$C_2$ alkyl); amino, NH($C_1$-$C_4$ alkyl), S($C_1$-$C_6$ alkyl), OC(O)NH($C_1$-$C_4$alkyl), N($C_1$-$C_2$ alkyl)C(O)($C_1$-$C_4$ alkyl), NHC(O)($C_1$-$C_4$ alkyl), COOH, CO($C_1$-$C_4$ alkyl), C(O)NH($C_1$-$C_4$ alkyl), C(O)N($C_1$-$C_4$ alkyl)($C_1$-$C_2$ alkyl), SH, CN, $NO_2$, SO($C_1$-$C_4$ alkyl); $SO_2$($C_1$-$C_4$ alkyl), $SO_2$NH($C_1$-$C_4$ alkyl), $SO_2$N($C_1$-$C_4$ alkyl)($C_1$-$C_2$ alkyl), and said $C_1$-$C_6$ alkyl may have one or two double or triple bonds;

$R_2$ is $C_1$-$C_{12}$ alkyl, aryl or ($C_1$-$C_{10}$ alkylene)aryl wherein said aryl is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl; 3- to 8-membered cycloalkyl or ($C_1$-$C_6$ alkylene) cycloalkyl, wherein said cycloalkyl may have one or two of O, S or N-Z, wherein Z is hydrogen, substituted , independently, for one or two carbons of said cycloalkyl, $C_1$-$C_4$ alkyl, benzyl or $C_1$-$C_4$ alkanoyl, wherein $R^2$ may be substituted independently by from one to three of chloro, fluoro, or $C_1$-$C_4$ alkyl, or one of hydroxy, bromo, iodo, $C_1$-$C_6$ alkoxy, OC(O)($C_1$-$C_6$ alkyl), O-C-N($C_1$-$C_4$ alkyl)($C_1$-$C_2$ alkyl), S($C_1$-$C_6$ alkyl), $NH_2$, NH($C_1$-$C_2$ alkyl), N($C_1$-$C_4$ alkyl) C(O)($C_1$-$C_4$ alkyl), NHC(O)($C_1$-$C_4$ alkyl), COOH, C(O)O($C_1$-$C_4$ alkyl), C(O)NH($C_1$-$C_4$ alkyl), C(O)N($C_1$-$C_4$ alkyl)($C_1$-$C_2$ alkyl), SH, CN, $NO_2$, SO($C_1$-$C_4$ alkyl), $SO_2$($C_1$-$C_4$ alkyl), $SO_2$NH($C_1$-$C_4$ alkyl), $SO_2$N($C_1$-$C_4$ alkyl)($C_1$-$C_2$ alkyl), and wherein said $C_1$-$C_{12}$ alkyl or $C_1$-$C_{10}$ alkylene may have one to three double or triple bonds; or

$NR_1R_2$ or $CR_1R_2R_{11}$ may form a 4- to 8-membered ring optionally having one or two double bonds or one or two of O, S or N-Z wherein Z is hydrogen, $C_1$-$C_4$ alkyl, benzyl, or $C_1$-$C_4$ alkanoyl;

$R_3$ is hydrogen, $C_1$-$C_6$ alkyl, fluoro, chloro, bromo, iodo, hydroxy, amino, O($C_1$-$C_6$ alkyl), NH($C_1$-$C_6$ alkyl), N($C_1$-$C_4$ alkyl)($C_1$-$C_2$ alkyl), SH, S($C_1$-$C_4$ alkyl), SO($C_1$-$C_4$ alkyl), or $SO_2$($C_1$-$C_4$ alkyl), wherein said $C_1$-$C_4$ alkyl and $C_1$-$C_6$ alkyl may have one or two double or triple bonds and may be substituted by from 1 to 3 $R_7$ substituents independently selected from the group consisting of hydroxy, amino, $C_1$-$C_3$ alkoxy, dimethylamino, diethylamino, methylamino, ethylamino, $NHC(O)CH_3$, fluoro, chloro or $C_1$-$C_3$ thioalkyl;

$R_4$ is hydrogen, $C_1$-$C_6$ alkyl, fluoro, chloro, bromo, iodo, $C_1$-$C_6$ alkoxy, amino, NH($C_1$-$C_6$ alkyl), N($C_1$-$C_6$ alkyl) ($C_1$-$C_2$ alkyl), $SO_n$($C_1$-$C_6$ alkyl), wherein n is 0, 1 or 2, cyano, hydroxy, carboxy, or amido, wherein said $C_1$-$C_6$ alkyls may be substituted by one to three of hydroxy, amino, carboxy, amido, NHC(O)($C_1$-$C_4$ alkyl), NH($C_1$-$C_4$ alkyl), N($C_1$-$C_4$ alkyl)($C_1$-$C_2$ alkyl), C(O)O($C_1$-$C_4$ alkyl), $C_1$-$C_3$ alkoxy, $C_1$-$C_3$ thioalkyl, fluoro, bromo, chloro, iodo, cyano or nitro;

$R_5$ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl benzoxazolyl, oxazolyl, pyrrolidinyl, thiazolidinyl, piperazinyl, piperidinyl, or tetrazolyl, wherein each one of the above groups may be substituted independently by from one to three of fluoro, chloro, bromo, formyl, $C_1$-$C_6$ alkyl, $C_1$-$C_6$ alkoxy or trifluoromethyl, or one of hydroxy, iodo, cyano, nitro, amino, cyclopropyl, NH($C_1$-$C_4$ alkyl), N($C_1$-$C_4$ alkyl)($C_1$-$C_2$ alkyl), COO($C_1$-$C_4$ alkyl), CO($C_1$-$C_4$ alkyl), $SO_2$NH($C_1$-$C_4$ alkyl), $SO_2$N($C_1$-$C_4$ alkyl)($C_1$-$C_2$ alkyl), $SO_2NH_2$, $NHSO_2$($C_1$-$C_4$ alkyl), S($C_1$-$C_6$ alkyl), $SO_2$($C_1$-$C_6$ alkyl), wherein said $C_1$-$C_4$ alkyl and $C_1$-$C_6$ alkyl may have one double or triple bond and may be substituted by one or two of fluoro, chloro, hydroxy, amino, methylamino, dimethylamino or acetyl; with the proviso that $R_5$ is not unsubstituted phenyl;

$R_{11}$ is hydrogen, hydroxy, fluoro, chloro, COO($C_1$-$C_2$ alkyl), cyano, or CO($C_1$-$C_2$ alkyl); and

$R_{12}$ is hydrogen or $C_1$-$C_4$ alkyl;

with the provisos that:

(a) A is not straight chain $C_1$-$C_{12}$ alkyl;
(b) when $R_3$ is hydrogen, A is benzyl or phenethyl, and $R_4$ is fluoro, chloro, bromo or iodo, then $R_5$ is not 5'-deoxy-ribofuranosyl or 5'-amino-5'-deoxy-ribofuranosyl; and
(c) when $R^5$ is phenyl, said phenyl is substituted by two or three substituents.

Another group of preferred CRF antagonists for use in the compositions, methods, and kits of the present invention are those wherein the CRF antagonist is a compound of formula:

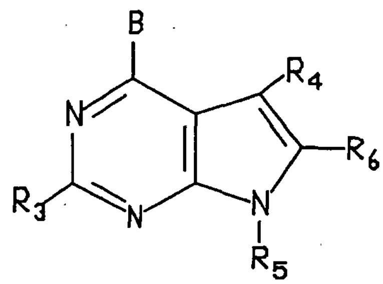

or a pharmaceutically acceptable acid addition salt thereof, wherein

B is $NR_1R_2$, $CR_1R_2R_{11}$, $C(=CR_2R_{12})R_1$, $NHR_1R_2R_{11}$, $OCR_1R_2R_{11}$, $SCR_1R_2R_{11}$, $NHNR_1R_2$, $CR_2R_{11}NHR_1$, $CR_2R_{11}OR_1$, $CR_2R_{11}SR_1$, or $C(O)R_2$;
$R_1$ is hydrogen, or $C_1$-$C_6$ alkyl which may be substituted by one or two substituents $R_7$ independently selected from the group consisting of hydroxy, fluoro, chloro, bromo, iodo, $C_1$-$C_8$ alkoxy, O-C(=O)-($C_1$-$C_6$ alkyl), O-C(=O)NH($C_1$-$C_4$ alkyl), O-C(=O)-N($C_1$-$C_4$ alkyl)($C_1$-$C_2$ alkyl), amino, NH($C_1$-$C_4$ alkyl), N($C_1$-$C_2$ alkyl)($C_1$-$C_4$ alkyl), S($C_1$-$C_6$ alkyl), N($C_1$-$C_4$alkyl)C(=O)($C_1$-$C_4$ alkyl), NH($C_1$-$C_4$ alkyl), COOH, C(=O)O($C_1$-$C_4$ alkyl), C(=O)NH($C_1$-$C_4$ alkyl), C(=O)N($C_1$-$C_4$ alkyl)($C_1$-$C_2$ alkyl), SH, CN, $NO_2$, SO($C_1$-$C_4$ alkyl), $SO_2$($C_1$-$C_4$ alkyl), $SO_2$NH($C_1$-$C_4$ alkyl), $SO_2$N($C_1$-$C_4$ alkyl)($C_1$-$C_2$ alkyl), and said $C_1$-$C_6$ alkyl may contain one or two double or triple bonds;
$R_2$ is $C_1$-$C_{12}$ alkyl, aryl or ($C_1$-$C_{10}$ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl, or benzoxazolyl; 3- to 8-membered cycloalkyl or ($C_1$-$C_6$ alkylene) cycloalkyl, wherein said cycloalkyl may contain one or two of O, S or N-Z wherein Z is hydrogen, $C_1$-$C_4$ alkyl, benzyl or $C_1$-$C_4$ alkanoyl, wherein $R_2$ may be substituted independently by from one to three of chloro, fluoro, or $C_1$-$C_4$ alkyl, or one of hydroxy, bromo, iodo, $C_1$-$C_6$ alkoxy, O-C(=O)-($C_1$-$C_6$ alkyl), O-C-N($C_1$-$C_4$ alkyl)($C_1$-$C_2$ alkyl), S($C_1$-$C_6$ alkyl), $NH_2$, NH($C_1$-$C_2$ alkyl), N($C_1$-$C_2$ alkyl) ($C_1$-$C_4$ alkyl), N($C_1$-$C_4$)-C(=O)($C_1$-$C_4$ alkyl), NHC(=O)($C_1$-$C_4$), COOH, C(=O)O($C_1$-$C_4$ alkyl), C(=O)NH($C_1$-$C_4$ alkyl), C(=O)N($C_1$-$C_4$ alkyl)($C_1$-$C_2$ alkyl), SH, CN, $NO_2$, SO($C_1$-$C_4$ alkyl); $SO_2$($C_1$-$C_4$ alkyl), $SO_2$NH($C_1$-$C_4$ alkyl), $SO_2$N($C_1$-$C_4$ alkyl)($C_1$-$C_2$ alkyl), and wherein said $C_1$-$C_{12}$ alkyl or $C_1$-$C_{10}$ alkylene may contain one to three double or triple bonds; or
$NR_1R_2$ or $CR_1R_2R_{11}$ may form a saturated 3- to 8 membered carbocyclic ring of which the 5- to 8-membered ring contain one or two double bonds or one or two of O, S or N-Z wherein Z is hydrogen, $C_1$-$C_4$ alkyl, benzyl or $C_1$-$C_4$ alkanoyl;
$R_3$ is hydrogen, $C_1$-$C_6$ alkyl, fluoro, chloro, bromo, iodo, hydroxy, amino, O($C_1$-$C_6$ alkyl), NH($C_1$-$C_6$ alkyl), N($C_1$-$C_4$ alkyl)($C_1$-$C_2$ alkyl), SH, S($C_1$-$C_4$ alkyl), SO($C_1$-$C_4$ alkyl), or $SO_2$($C_1$-$C_4$ alkyl), wherein said $C_1$-$C_4$ alkyl and $C_1$-$C_6$ alkyl may contain from one or two double or triple bonds and may be substituted by from 1 to 3 substituents $R_8$ independently selected from the group consisting of hydroxy, amino, $C_1$-$C_3$ alkoxy, dimethylamino, diethylamino, methylamino, ethylamino, $NHCH_3$, fluoro, chloro or $C_1$-$C_3$ thioalkyl;
$R_4$ and $R_6$ are each independently hydrogen, $C_1$-$C_6$ alkyl, fluoro, chloro, bromo, iodo, $C_1$-$C_6$ alkoxy, amino, NH($C_1$-$C_6$ alkyl), N($C_1$-$C_6$ alkyl)($C_1$-$C_2$ alkyl), $SO_n$($C_1$-$C_6$ alkyl), wherein n is 0, 1 or 2, cyano, hydroxy, carboxy, or amido, wherein said $C_1$-$C_6$ alkyls may be substituted by one to three of hydroxy, amino, carboxy, amido, NHC(=O)($C_1$-$C_4$ alkyl), NH($C_1$-$C_4$ alkyl), N($C_1$-$C_4$ alkyl)($C_1$-$C_2$ alkyl), C(=O)O($C_1$-$C_4$ alkyl), $C_1$-$C_3$ alkoxy, $C_1$-$C_3$ thioalkyl, fluoro, bromo, chloro, iodo, cyano or nitro;
$R_5$ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, piperidinyl, piperazinyl, tetrazolyl, or 3- to 8-membered cycloalkyl or 9- to 12-membered bicycloalkyl, optionally containing one to three of O, S or N-Z wherein Z is hydrogen, $C_1$-$C_4$ alkyl, $C_1$-$C_4$ alkanoyl, phenyl or phenylmethyl, wherein each

one of the above groups may be substituted independently by from one to four of fluoro, chloro, $C_1$-$C_6$ alkyl, $C_1$-$C_6$ alkoxy or trifluoromethyl, or one of bromo, iodo, cyano, nitro, amino, NH($C_1$-$C_4$ alkyl), N($C_1$-$C_4$)($C_1$-$C_2$ alkyl), COO($C_1$-$C_4$ alkyl), CO($C_1$-$C_4$ alkyl), $SO_2$NH($C_1$-$C_4$ alkyl), $SO_2$N($C_1$-$C_4$ alkyl)($C_1$-$C_2$ alkyl), $SO_2NH_2$, $NHSO_2$($C_1$-$C_4$ alkyl), S($C_1$-$C_6$ alkyl), $SO_2$($C_1$-$C_6$ alkyl), wherein said $C_1$-$C_4$ alkyl and $C_1$-$C_6$ alkyl may be substituted by one or two of fluoro, chloro, hydroxy, amino, methylamino, dimethylamino or acetyl; with the proviso that $R_5$ is not unsubstituted phenyl;

$R_{11}$ is hydrogen, hydroxy, fluoro, chloro, COO($C_1$-$C_2$ alkyl), cyano, or CO($C_1$-$C_2$ alkyl); and

$R_{12}$ is hydrogen or $C_1$-$C_4$ alkyl; with the proviso that (1) when $R_5$ is 4-bromophenyl, $R_3$ is hydrogen, and $R_4$ and $R_6$ are methyl, then B is not methylamino or ethyl, and (2) when $R_5$ is 4-bromophenyl, and $R_3$, $R_4$ and $R_6$ are methyl, then B is not 2-hydroxyethylamino.

Another group of preferred CRF antagonists for use in the compositions, methods, and kits of the present invention are those wherein the CRF antagonist is a compound of formula:

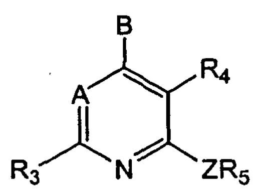
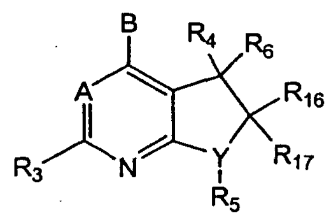
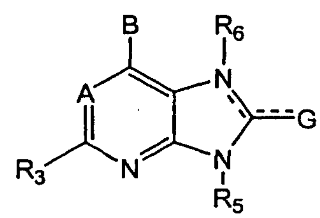

or a pharmaceutically acceptable acid addition salt thereof, wherein

A is $CR_7$ or N;

B is $NR_1R_2$, $CR_1R_2R_{11}$, C(=$CR_2R_{12}$)$R_1$, $NHCHR_1R_2$, $OCHR_1R_2$, $SCHR_1R_2$, $CHR_2OR_{12}$, $CHR_2SR_{12}$, C(S)$R_2$ or C(O)$R_2$;

G is oxygen, sulfur, NH, $NH_3$, hydrogen, methoxy, ethoxy, trifluoromethoxy, methyl, ethyl, thiomethoxy, $NH_2$, $NHCH_3$, $N(CH_3)_2$ or trifluromethyl;

Y is CH or N;

Z is NH, O, S, N ($C_1$-$C_2$ alkyl), or $CR_{13}R_{14}$, wherein $R_{13}$ and $R_{14}$ are each independently hydrogen, trifluoromethyl, or $C_1$-$C_4$ alkyl, or one of $R_{13}$ and $R_{14}$ may be cyano, chloro, bromo, iodo, fluoro, hydroxy, O($C_1$-$C_2$ alkyl), amino, NH($C_1$-$C_2$ alkyl), or $CR_{13}R_{14}$ may be C=O or cyclopropyl;

$R_1$ is $C_1$-$C_6$ alkyl which may be substituted by one or two substituents $R_8$ independently selected from the group consisting of hydroxy, fluoro, chloro, bromo, iodo, $C_1$-$C_4$ alkoxy, O-CO-($C_1$-$C_4$ alkyl), O-CO-NH($C_1$-$C_4$ alkyl), O-CO-N($C_1$-$C_4$ alkyl)($C_1$-$C_2$ alkyl), NH($C_1$-$C_4$ alkyl), N($C_1$-$C_2$ alkyl)($C_1$-$C_4$ alkyl), S($C_1$-$C_4$ alkyl), N($C_1$-$C_4$alkyl)CO($C_1$-$C_4$ alkyl), NHCO($C_1$-$C_4$ alkyl), COO($C_1$-$C_4$ alkyl), CONH($C_1$-$C_4$ alkyl), CON($C_1$-$C_4$ alkyl)($C_1$-$C_2$ alkyl), S($C_1$-$C_4$ alkyl), CN, $NO_2$, SO($C_1$-$C_4$ alkyl), $SO_2$($C_1$-$C_4$ alkyl), and said $C_1$-$C_6$ alkyl or $C_1$-$C_4$ alkyl may contain one double or triple bond;

$R_2$ is $C_1$-$C_{12}$ alkyl, aryl or ($C_1$-$C_4$ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, or benzoxazolyl; 3- to 8-membered cycloalkyl or ($C_1$-$C_6$ alkylene)cycloalkyl, wherein said cycloalkyl may contain one or two of O, S or N-$R_9$ wherein $R_9$ is hydrogen, or $C_1$-$C_4$ alkyl, wherein the above defined $R_2$ may be substituted independently by from one to three of chloro, fluoro, or $C_1$-$C_4$ alkyl, or one of bromo, iodo, $C_1$-$C_6$ alkoxy, O-CO-($C_1$-$C_6$ alkyl), O-CO-N($C_1$-$C_4$ alkyl)($C_1$-$C_2$ alkyl), S($C_1$-$C_6$ alkyl), CN, $NO_2$, SO($C_1$-$C_4$ alkyl), or $SO_2$($C_1$-$C_4$ alkyl), and wherein said $C_1$-$C_{12}$ alkyl or $C_1$-$C_4$ alkylene may contain one double or triple bond; or

$NR_1R_2$ or $CR_1R_2R_{11}$ may form a saturated 5- to 8-membered carbocyclic ring which may contain one or two double bonds or one or two of O or S;

$R_3$ is methyl, ethyl, fluoro, chloro, bromo, iodo, cyano, methoxy, $OCF_3$, methylthio, methylsulfonyl, $CH_2OH$ or $CH_2OCH_3$;

$R_4$ is hydrogen, $C_1$-$C_4$ alkyl, fluoro, chloro, bromo, iodo, $C_1$-$C_4$ alkoxy, amino, nitro, NH($C_1$-$C_4$ alkyl), N($C_1$-$C_4$ alkyl)($C_1$-$C_2$ alkyl), $SO_n$($C_1$-$C_4$ alkyl), wherein n is 0, 1 or 2, cyano, hydroxy, CO($C_1$-$C_4$ alkyl), CHO, or COO($C_1$-$C_4$ alkyl), wherein said $C_1$-$C_4$ alkyl may contain one or two double or triple bonds and may be substituted by one or two of hydroxy, amino, carboxy, $NHCOCH_3$, NH($C_1$-$C_2$ alkyl), N($C_1$-$C_2$ alkyl)$_2$, COO($C_1$-$C_4$ alkyl), CO($C_1$-$C_4$ alkyl), $C_1$-$C_3$ alkoxy, $C_1$-$C_3$ thioalkyl, fluoro, chloro, cyano or nitro;

$R_5$ is phenyl, naphthyl, thienyl, benzothienyl, pyridyl, quinolyl, pyrazinyl, pyrimidyl, furanyl, benzofuranyl, benzothiazolyl, or indolyl, wherein each one of the above groups $R_5$ is substituted independently by from one to three of fluoro, chloro, $C_1$-$C_6$ alkyl, or $C_1$-$C_6$ alkoxy, or one of hydroxy, iodo, bromo, formyl, cyano, nitro, trifluoromethyl, amino, NH($C_1$-$C_4$ alkyl), N($C_1$-$C_6$)($C_1$-$C_2$ alkyl), COOH, COO($C_1$-$C_4$ alkyl), CO($C_1$-$C_4$ alkyl), $SO_2$NH($C_1$-$C_4$ alkyl), $SO_2$N($C_1$-$C_4$ alkyl)($C_1$-$C_2$ alkyl), $SO_2NH_2$, $NHSO_2$($C_1$-$C_4$ alkyl), S($C_1$-$C_6$ alkyl), or $SO_2$($C_1$-$C_6$ alkyl), wherein said $C_1$-$C_4$ alkyl and $C_1$-$C_6$ alkyl may be substituted by one or two of fluoro, hydroxy, amino, methylamino, dimethylamino or acetyl;

$R_6$ is hydrogen, or $C_1$-$C_6$ alkyl, wherein said $C_1$-$C_6$ alkyl may be substituted by one hydroxy, methoxy, ethoxy or fluoro;

$R_7$ is hydrogen, $C_1$-$C_4$ alkyl, fluoro, chloro, bromo, iodo, cyano, hydroxy, O($C_1$-$C_4$ alkyl), C(O)($C_1$-$C_4$ alkyl), or C (O)O($C_1$-$C_4$ alkyl), wherein the $C_1$-$C_4$ alkyl groups may be substituted with one hydroxy, chloro or bromo, or one to three fluoro;

$R_{11}$ is hydrogen, hydroxy, fluoro, or methoxy;

$R_{12}$ is hydrogen or $C_1$-$C_4$ alkyl; and

$R_{16}$ and $R_{17}$ are each independently hydrogen, hydroxy, methyl, ethyl, methoxy, or ethoxy, except that they are not both methoxy or ethoxy, and $CR_4R_6$ and $CR_{16}R_{17}$ each independently may be C=O.

Another group of preferred CRF antagonists for use in the compositions, methods, and kits of the present invention are those wherein the CRF antagonist is a compound of formula:

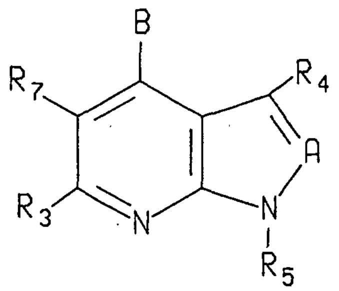

or a pharmaceutically acceptable acid addition salt thereof, wherein

A is N or -$CR_6$;

B is -$NR_1R_2$, -$CR_1R_2R_{11}$, -C(=$CR_2R_{12}$)$R_1$, -$NHCHR_1R_2$, -$OCHR_1R_2$, -$SCHR_1R_2$, -$CHR_2OR_{12}$, -$CHR_2SR_{12}$, -C(S) $R_1$ or -C(O)$R_1$;

$R_1$ is $C_1$-$C_6$ alkyl which may optionally be substituted with one or two substituents independently selected from the group consisting of hydroxy, fluoro, chloro, bromo, iodo, $C_1$-$C_4$ alkoxy, -O-CO-($C_1$-$C_4$ alkyl), -O-CO-NH($C_1$-$C_4$ alkyl), -O-CO-N($C_1$-$C_4$ alkyl)($C_1$-$C_2$ alkyl), -NH($C_1$-$C_4$ alkyl), -N($C_1$-$C_2$ alkyl)($C_1$-$C_4$ alkyl), -S($C_1$-$C_4$ alkyl), -N($C_1$-$C_4$alkyl)CO($C_1$-$C_4$ alkyl), -NHCO($C_1$-$C_4$ alkyl), -COO($C_1$-$C_4$ alkyl), -CONH($C_1$-$C_4$ alkyl), -CON($C_1$-$C_4$ alkyl)($C_1$-$C_2$ alkyl); CN, $NO_2$, -SO($C_1$-$C_4$ alkyl), -$SO_2$($C_1$-$C_4$ alkyl), and wherein any of the foregoing $C_1$-$C_4$ alkyl and $C_1$-$C_6$ alkyl groups may optionally contain one carbon-carbon double or triple bond;

$R_2$ is $C_1$-$C_{12}$ alkyl, aryl, -($C_1$-$C_4$ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, oxazolyl, or benzoxazolyl; or 3- to 8- membered cycloalkyl or -($C_1$-$C_6$ alkylene)cycloalkyl, wherein one or two of the ring carbons of said cycloalkyl having at least 4 ring members and the cycloalkyl moiety of said -($C_1$-$C_6$ alkylene)cycloalkyl having at least 4 ring members may optionally be replaced by an oxygen or sulfur atom or by N-Z wherein Z is hydrogen; or $C_1$-$C_4$ alkyl, and wherein each of said groups $R_2$ may optionally be substituted with from one to three substituents independently selected from chloro, fluoro, and $C_1$-$C_4$ alkyl, or by one substituent selected from bromo, iodo, $C_1$-$C_6$ alkoxy, -O-CO-($C_1$-$C_6$ alkyl), -S($C_1$-$C_6$ alkyl), -COO($C_1$-$C_4$ alkyl), CN, $NO_2$, -SO($C_1$-$C_4$ alkyl), and -$SO_2$($C_1$-$C_4$ alkyl), and wherein said $C_1$-$C_{12}$ alkyl and the $C_1$-$C_4$ alkylene moiety of said -($C_1$-$C_4$ alkylene)aryl may optionally contain one carbon-carbon double or triple bond;

or-$NR_1R_2$ may form a saturated 5- to 8-membered heterocyclic ring, or -$CHR_1R_2$ may form a saturated 5- to 8-membered carbocyclic ring, wherein each of these rings may optionally contain one or two carbon-carbon double bonds and wherein one or two of the carbon atoms of each of these rings may optionally be replaced with a sulfur or oxygen atom;

$R_3$ is $C_1$-$C_4$ alkyl, fluoro, chloro, bromo, iodo, -$CH_2OH$, -$CH_2OCH_3$, -O($C_1$-$C_3$ alkyl), -S($C_1$-$C_3$ alkyl), or -$SO_2$($C_1$-$C_3$ alkyl), wherein said $C_1$-$C_3$ alkyl may optionally contain one carbon-carbon double or triple bond;

$R_4$ is hydrogen, $C_1$-$C_6$ alkyl, fluoro, chloro, bromo, iodo, $C_1$-$C_4$ alkoxy, amino, $-NHCH_3$, $-N(CH_3)_2$, $-CH_2OH$, $-CH_2OCH_3$, or $-SO_n(C_1$-$C_4$ alkyl), wherein n is 0, 1 or 2, cyano, hydroxy, -CO($C_1$-$C_4$ alkyl), -CHO, or -COO($C_1$-$C_4$ alkyl) wherein the $C_1$-$C_4$ alkyl moieties in the foregoing $R_4$ groups may optionally contain one carbon-carbon double or triple bond;

$R_5$ is phenyl, naphthyl, thienyl, benzothienyl, pyridyl, pyrimidyl, benzofuranyl, pyrazinyl or benzothiazolyl, wherein each one of said groups $R_5$ may optionally be substituted with from one to three substituents independently selected from fluoro, chloro, $C_1$-$C_6$ alkyl and $C_1$-$C_6$ alkoxy, or by one substituent selected from iodo, hydroxy, bromo, formyl, cyano, nitro, amino, trifluoromethyl, -NH($C_1$-$C_4$ alkyl), -N($C_1$-$C_6$)($C_1$-$C_2$ alkyl), -COO($C_1$-$C_4$ alkyl), -CO($C_1$-$C_4$ alkyl), -COOH, $-SO_2NH(C_1$-$C_4$ alkyl), $-SO_2N(C_1$-$C_4$ alkyl)($C_1$-$C_2$ alkyl), $-SO_2NH_2$, $-NHSO_2(C_1$-$C_4$ alkyl), -S($C_1$-$C_6$ alkyl) and $-SO_2(C_1$-$C_6$ alkyl), wherein each of said $C_1$-$C_4$ alkyl and $C_1$-$C_6$ alkyl moieties in the foregoing $R^5$ groups may optionally be substituted with one to three fluorine atoms;

$R_6$ is hydrogen, $C_1$-$C_4$ alkyl, fluoro, chloro, bromo, iodo, $-CH_2OH$, $-CH_2OCH_3$, or $C_1$-$C_4$ alkoxy;

$R_7$ is hydrogen, $C_1$-$C_4$ alkyl, fluoro, chloro, bromo, iodo, -O($C_1$-$C_4$ alkyl), cyano, $-CH_2OH$, $-CH_2O(C_1$-$C_2$ alkyl), -CO ($C_1$-$C_2$ alkyl), or -COO($C_1$-$C_2$ alkyl);

$R_{11}$ is hydrogen, hydroxy, fluoro, or methoxy; and

$R_{12}$ is hydrogen or $C_1$-$C_4$ alkyl;

with the proviso that when A is N, then: (a) B is not unsubstituted alkyl; (b) $R_5$ is not unsubstituted phenyl or monosubstituted phenyl; and (c) $R_3$ is not unsubstituted alkyl.

Another group of preferred CRF antagonists for use in the compositions, methods, and kits of the present invention are those wherein the CRF antagonist is a compound of formula:

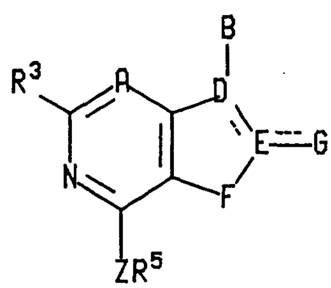

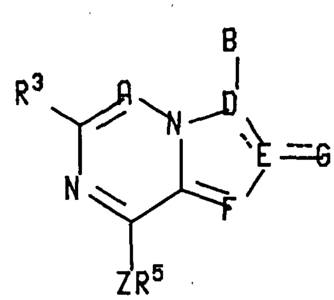

or

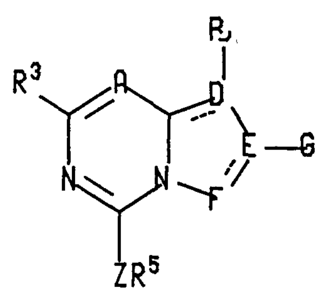

or a pharmaceutically acceptable salt thereof, wherein

the dashed lines represent optional double bonds;

A is nitrogen or $CR^7$;

B is $-NR^1R^2$, $-CR^1R^2R^{10}$, $-C(=CR^2R^{11})R^1$, $-NHCR^1R^2R^{10}$, $-OCR^1R^2R^{10}$, $-SCR^1R^2R^{10}$,$-CR^2R^{10}NHR^1$, $-CR^2R^{10}OR^1$, $-CR^2R^{10}SR^1$ or $-COR^2$;

D is nitrogen and is single bonded to all atoms to which it is attached, or D is carbon and is either double bonded to E in formulas I and II or double bonded to the adjacent carbon atom common to both fused rings in formula III, or D is CH and is single bonded to E in formulas I and II;

E is nitrogen, CH or carbon;
F is oxygen, sulfur, $CHR^4$ or $NR^4$ when it is single bonded to E and F is nitrogen or $CR^4$ when it is double bonded to E;
G, when single bonded to E, is hydrogen, $C_1$-$C_4$ alkyl, -S($C_1$-$C_4$ alkyl), -O($C_1$-$C_4$ alkyl), $NH_2$, -NH($C_1$-$C_4$ alkyl) on -N($C_1$-$C_2$ alkyl)($C_1$-$C_4$ alkyl), wherein each of the $C_1$-$C_4$ alkyl groups of G may optionally be substituted with one hydroxy, -O($C_1$-$C_2$ alkyl) or fluoro group; G, when double bonded to E, is oxygen, sulfur or NH; and G, when E is nitrogen and double bonded to D or F, is absent;
$R^1$ is hydrogen, $C_1$-$C_6$ alkyl optionally substituted with one or two substituents $R^8$ independently selected from hydroxy, fluoro, chloro, bromo, iodo, $C_1$-$C_4$ alkoxy, $CF_3$, -C(=O)O-($C_1$-$C_4$)alkyl, -OC(=O)($C_1$-$C_4$ alkyl), -OC(=O)N ($C_1$-$C_4$ alkyl)($C_1$-$C_2$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, -$NO_2$, -SO($C_1$-$C_4$ alkyl), -$SO_2$($C_1$-$C_4$ alkyl), -$SO_2$NH($C_1$-$C_4$ alkyl) and -$SO_2$N($C_1$-$C_4$ alkyl)($C_1$-$C_2$ alkyl), wherein each of the $C_1$-$C_4$ alkyl groups in the foregoing $R^1$ groups may optionally contain one or two double or triple bonds;
$R^2$ is $C_1$-$C_{12}$ alkyl which may optionally contain from one to three double or triple bonds, aryl or ($C_1$-$C_4$ alkylene) aryl, wherein said aryl and the aryl moiety of said ($C_1$-$C_4$ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; $C_3$-$C_8$ cycloalkyl or ($C_1$-$C_6$ alkylene)($C_3$-$C_8$ cycloalkyl), wherein one or two of the carbon atoms of said cycloalkyl and the 5 to 8 membered cycloalkyl moieties of said ($C_1$-$C_6$ alkylene)($C_3$-$C_8$ cycloalkyl) may optionally and independently be replaced by an oxygen or sulfur atom or by $NZ^2$ wherein $Z^2$ is selected from hydrogen, $C_1$-$C_4$ alkyl, benzyl and $C_1$-$C_4$ alkanoyl, and wherein each of the foregoing $R^2$ groups may optionally be substituted with from one to three substituents independently selected from chloro, fluoro, hydroxy and $C_1$-$C_4$ alkyl, or with one substituent selected from bromo, iodo, $C_1$-$C_6$ alkoxy, -OC (=O)($C_1$-$C_6$ alkyl), -OC(=O)N($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl) ($C_1$-$C_4$ alkyl), -N($C_1$-$C_4$ alkyl)-CO-($C_1$-$C_4$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -SH, -CN, -$NO_2$, -SO($C_1$-$C_4$ alkyl), -$SO_2$($C_1$-$C_4$ alkyl), -$SO_2$NH($C_1$-$C_4$ alkyl) and -$SO_2$N($C_1$-$C_4$ alkyl)($C_1$-$C_2$ alkyl);
-$NR^1R^2$ or $CR^1R^2R^{10}$ may form a saturated 3 to 8 membered carbocyclic ring which may optionally contain from one to three double bonds and-wherein one or two of the ring carbon atoms of such 5 to 8 membered rings may optionally and independently be replaced by an oxygen or sulfur atom or by $NZ^3$ wherein $Z^3$ is hydrogen, $C_1$-$C_4$ alkyl, benzyl or $C_1$-$C_4$ alkanoyl;
$R^3$ is hydrogen, $C_1$-$C_4$ alkyl, -O($C_1$-$C_4$ alkyl), chloro, fluoro, bromo, iodo, -CN, -S($C_1$-$C_4$ alkyl) or -$SO_2$($C_1$-$C_4$ alkyl) wherein each of the ($C_1$-$C_4$ alkyl) moieties in the foregoing $R^3$ groups may optionally be substituted with one substituent $R^9$ selected from hydroxy, fluoro and ($C_1$-$C_2$ alkoxy);
each $R^4$ is, independently, hydrogen, ($C_1$-$C_6$ alkyl), fluoro, chloro, bromo, iodo, hydroxy, cyano, amino, nitro, -O ($C_1$-$C_4$ alkyl), -N($C_1$-$C_4$ alkyl)($C_1$-$C_2$ alkyl), -S($C_1$-$C_4$ alkyl), -SO($C_1$-$C_4$ alkyl), -$SO_2$($C_1$-$C_4$)alkyl, -CO($C_1$-$C_4$ alkyl), -C(=O)H or -C(=O)O($C_1$-$C_4$alkyl), wherein each of the ($C_1$-$C_6$ alkyl) and ($C_1$-$C_4$ alkyl) moieties in the foregoing $R^4$ groups may optionally contain one or two double or triple bonds and may optionally be substituted with one or two substituents independently selected from hydroxy, amino, $C_1$-$C_3$ alkoxy, dimethylamino, methylamino, ethylamino, -NHC(=O)$CH_3$, fluoro, chloro, $C_1$-$C_3$ thioalkyl, -CN, -COOH, -C(=O)O($C_1$-$C_4$ alkyl),-C(=O)($C_1$-$C_4$ alkyl) and -$NO_2$;
$R^5$ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, benzoxazolyl or $C_3$-$C_8$ cycloalkyl wherein one or two of the carbon atoms of said cycloalkyl rings that contain at least 5 ring members may optionally and independently be replaced by an oxygen or sulfur atom or by $NZ^4$ wherein $Z^4$ is hydrogen, $C_1$-$C_4$ alkyl or benzyl; and wherein each of the foregoing $R^5$ groups is substituted with from one to four substituents $R^{12}$ wherein one to three of said substituents may be selected, independently, from chloro, $C_1$-$C_6$ alkyl and -O($C_1$-$C_6$ alkyl) and one of said substituents may be selected from bromo, iodo, formyl, -CN, - $CF_3$, -$NO_2$, -$NH_2$, -NH($C_1$-$C_4$ alkyl), -N($C_1$-$C_2$ alkyl)($C_1$-$C_6$ alkyl), -C(=O)O($C_1$-$C_4$ alkyl), -C(=O)($C_1$-$C_4$ alkyl), -COOH, -$SO_2$NH($C_1$-$C_4$ alkyl), -$SO_2$N($C_1$-$C_2$ alkyl)($C_1$-$C_4$ alkyl),-$SO_2NH_2$, -$NHSO_2$($C_1$-$C_4$ alkyl), -S($C_1$-$C_6$ alkyl) and -$SO_2$($C_1$-$C_6$ alkyl), and wherein each of the $C_1$-$C_4$ alkyl and $C_1$-$C_6$ alkyl moieties in the foregoing $R^5$ groups may optionally be substituted with one or two substituents independently selected from fluoro, hydroxy, amino, methylamino, dimethylamino and acetyl;
$R^7$ is hydrogen, $C_1$-$C_4$ alkyl, halo, cyano, hydroxy, -O($C_1$-$C_4$ alkyl) -C(=O)($C_1$-$C_4$ alkyl), -C(=O)O($C_1$-$C_4$alkyl), -$OCF_3$, -$CF_3$, -$CH_2OH$, -$CH_2O$($C_1$-$C_4$ alkyl);
$R^{10}$ is hydrogen, hydroxy, methoxy or fluoro;
$R^{11}$ is hydrogen or $C_1$-$C_4$ alkyl; and
Z is NH, oxygen, sulfur, -N($C_1$-$C_4$ alkyl), -NC(=O)($C_1$-$C_2$ alkyl), NC(=O)O($C_1$-$C_2$alkyl) or $CR^{13}R^{14}$ wherein $R^{13}$ and $R^{14}$ are independently selected from hydrogen, trifluoromethyl and methyl with the exception that one of $R^{13}$ and $R^{14}$ can be cyano;

with the proviso that: (a) in the five membered rings of structures I, II and III, there can not be two double bonds

adjacent to each other; and (b) when $R^4$ is attached to nitrogen, it is not halo, cyano or nitro.

Another group of preferred CRF antagonists for use in the compositions, methods, and kits of the present invention are those wherein the CRF antagonist is a compound of formula:

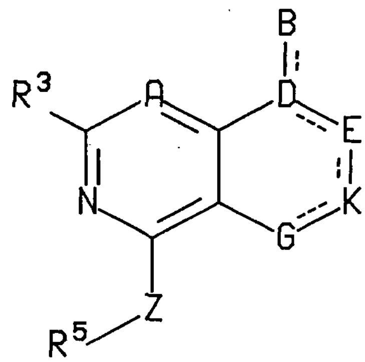

wherein the dashed lines represent optional double bonds;

or a pharmaceutically acceptable salt thereof, wherein

A is nitrogen or $CR^7$;

B is $-NR^1R^2$, $-CR^1R^2R^{10}$, $-C(=CR^2R^{11})R^1$, $-NHCR^1R^2R^{10}$, $-OCR^1R^2R^{10}$, $-SCR^1R^2R^{10}$, $-CR^2R^{10}NHR^1$, $-CR^2R^{10}OR^1$, $-CR^2R^{10}SR^1$ or $-COR^2$, and is single bonded to D; or B is $-CR^1R^2$, and is double bonded to D and D is carbon;

D is nitrogen or $CR^4$ and is single bonded to all atoms to which it is attached, or D is carbon and is double bonded to E or double bonded to B;

E is oxygen, nitrogen, sulfur, C=O, C=S, $CR^6R^{12}$, $NR^6$ or $CR^6$; or E is a two atom spacer, wherein one of the atoms is oxygen, nitrogen, sulfur, C=O, C=S, $CR^6R^{12}$, $NR^6$ or $CR^6$, and the other is $CR^6R^{12}$ or $CR^9$;

K and G are each, independently, C=O, C=S, sulfur, oxygen, $CHR^8$ or $NR^8$ when single bonded to both adjacent ring atoms, or nitrogen or $CR^8$ when it is double bonded to an adjacent ring atom;

the 6- or 7-membered ring that contains D, E, K and G may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;

$R^1$ is $C_1$-$C_6$ alkyl optionally substituted with from one or two substituents independently selected from hydroxy, fluoro, chloro, bromo, iodo, $C_1$-$C_4$ alkoxy, $CF_3$, -C(=O)($C_1$-$C_4$alkyl), -C(=O)-O-($C_1$-$C_4$)alkyl, -OC(=O)($C_1$-$C_4$ alkyl), -OC(=O)N($C_1$-$C_4$ alkyl)($C_1$-$C_2$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl), wherein each of the $C_1$-$C_4$ alkyl groups in the foregoing $R^1$ groups may optionally contain one or two double or triple bonds;

$R^2$ is $C_1$-$C_{12}$ alkyl which may optionally contain from one to three double or triple bonds, aryl or ($C_1$-$C_4$ alkylene) aryl, wherein said aryl and the aryl moiety of said ($C_1$-$C_4$ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; $C_3$-$C_8$ cycloalkyl or ($C_1$-$C_6$ alkylene)($C_3$-$C_8$ cycloalkyl), wherein one or two of the carbon atoms of said cycloalkyl and the 5 to 8 membered cycloalkyl moieties of said ($C_1$-$C_6$ alkylene)($C_3$-$C_8$ cycloalkyl may optionally and independently be replaced by an oxygen or sulfur and wherein each of the foregoing $R^2$ groups may optionally be substituted with from one to three substituents independently selected from chloro, fluoro, hydroxy and $C_1$-$C_4$ alkyl, or with one substituent selected from $C_1$-$C_6$ alkoxy, -OC(=O)($C_1$-$C_6$ alkyl), -OC(=O)N($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl)($C_1$-$C_4$ alkyl), -N($C_1$-$C_4$ alkyl)-CO-($C_1$-$C_4$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -SH, -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl);

$-NR^1R^2$ or $CR^1R^2R^{10}$ may form a ring selected from saturated 3 to 8 membered rings, the 5 to 8 membered rings of which may optionally contain one or two double bonds, and wherein one or two of the ring carbon atoms of such 5 to 8 membered rings may optionally and independently be replaced by an oxygen or sulfur atom or by $NZ^3$ wherein $Z^3$ is hydrogen or $C_1$-$C_4$ alkyl;

$R^3$ is hydrogen, $C_1$-$C_4$ alkyl, -O($C_1$-$C_4$ alkyl), chloro, fluoro, bromo, iodo, -S($C_1$-$C_4$ alkyl) or $-SO_2$($C_1$-$C_4$ alkyl);

$R^4$ is hydrogen, $C_1$-$C_2$ alkyl, hydroxy or fluoro;

each $R^6$, $R^8$ and $R^9$ that is attached to a carbon atom is selected, independently, from hydrogen, $C_1$-$C_2$ alkyl, fluoro, chloro, bromo, iodo, hydroxy, hydroxymethyl, formyl, trifluoromethyl, cyano, amino, nitro, -O($C_1$-$C_2$ alkyl), -N($C_1$-$C_2$ alkyl)($C_1$-$C_2$ alkyl), -S($C_1$-$C_2$ alkyl), -CO($C_1$-$C_2$ alkyl), -C(=O)H or -C(=O)O($C_1$-$C_2$alkyl), wherein each of the $C_1$-$C_2$ alkyl moieties in the foregoing $R^6$, $R^8$, and $R^9$ groups may optionally contain one double or triple bond; and each $R^6$, $R^8$, and $R^9$ that is attached to a nitrogen atom is selected, independently, from hydrogen and $C_1$-$C_4$ alkyl;

$R^5$ is substituted phenyl, naphthyl, pyridyl or pyrimidyl, wherein each of the foregoing $R^5$ groups is substituted with from two to four substituents $R^{15}$, wherein from one to three of said substituents may be selected, independently, from chloro, $C_1$-$C_6$ alkyl, -O($C_1$-$C_6$ alkyl) and -($C_1$-$C_6$alkylene)O($C_1$-$C_6$alkyl), and wherein one of said substituents may be selected, independently, from bromo, iodo, formyl, cyano, trifluoromethyl, nitro, amino, -NH($C_1$-$C_4$ alkyl), -N($C_1$-$C_2$ alkyl)($C_1$-$C_6$ alkyl), -C(=O)O($C_1$-$C_4$ alkyl), -C(=O)($C_1$-$C_4$ alkyl), -COOH, -$SO_2$NH($C_1$-$C_4$ alkyl), -$SO_2$N($C_1$-$C_2$ alkyl)($C_1$-$C_4$ alkyl), -$SO_2NH_2$, -NH$SO_2$($C_1$-$C_4$ alkyl), -S($C_1$-$C_6$ alkyl) and -$SO_2$($C_1$-$C_6$ alkyl), and wherein each of the $C_1$-$C_4$ alkyl and $C_1$-$C_6$ alkyl moieties in the foregoing $R^5$ groups may optionally be substituted with one or two substituents independently selected from fluoro, hydroxy, amino, methylamino, dimethylamino and acetyl;

$R^7$ is hydrogen, methyl, halo, hydroxy, methoxy, -C(=O)($C_1$-$C_2$ alkyl), -C(=O)O($C_1$-$C_2$ alkyl), trifluoromethoxy, hydroxymethyl, trifluoromethyl or formyl;

$R^{10}$ is hydrogen, hydroxy, methoxy or fluoro;

$R^{11}$ is hydrogen or $C_1$-$C_4$ alkyl;

$R^{12}$ is hydrogen or methyl; and

Z is NH, oxygen, sulfur, -N($C_1$-$C_4$ alkyl), or $CR^{13}R^{14}$ wherein $R^{13}$ and $R^{14}$ are independently selected from hydrogen, and methyl with the exception that one of $R^{13}$ and $R^{14}$ may optionally be cyano;

with the proviso that: (a) in the six or seven membered rings of structures in formula I, there can not be two double bonds adjacent to each other; and (b) when D is carbon and is double bonded to B, then B is $CR^1R^2$.

Another group of preferred CRF antagonists for use in the compositions, methods, and kits of the present invention are those wherein the CRF antagonist is a compound of formula:

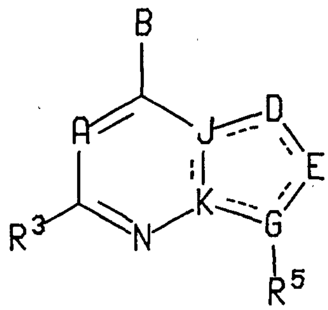

or a pharmaceutically acceptable salt thereof, wherein

the dashed lines represent optional double bonds;

A is nitrogen or $CR^7$;

B is -$NR^1R^2$, -$CR^1R^2R^{10}$ -C(=$CR^2R^{11}$)$R^1$, -NH$CR^1R^2R^{10}$, -O$CR^1R^2R^{10}$, -S$CR^1R^2R^{10}$, -$CR^2R^{10}$N$HR^1$, -$CR^2R^{10}OR^1$, -$CR^2R^{10}SR^1$ or -$COR^2$;

J and K are each independently nitrogen or carbon and both J and K are not nitrogens;

D and E are each selected, independently, from nitrogen, $CR^4$, C=O, C=S, sulfur, oxygen, $CR^4R^6$ and $NR^8$;

G is nitrogen or carbon;

the ring containing D, E, G, K, and J in formula I may be a saturated or unsaturated 5-membered ring and may optionally contain one or two double bonds and may optionally contain from one to three heteroatoms in the ring and may optionally have one or two C=O or C=S groups;

$R^1$ is $C_1$-$C_6$ alkyl optionally substituted with one or two substituents independently selected from hydroxy, fluoro, chloro, bromo, iodo, -O-($C_1$-$C_4$ alkyl), $CF_3$, -C(=O)O-($C_1$-$C_4$alkyl), -OC(=O)($C_1$-$C_4$ alkyl), -OC(=O)N($C_1$-$C_4$ alkyl)($C_1$-$C_2$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, -$NO_2$, -SO($C_1$-$C_4$ alkyl), -$SO_2$($C_1$-$C_4$ alkyl), -$SO_2$NH($C_1$-$C_4$ alkyl) and -$SO_2$N($C_1$-$C_4$ alkyl)($C_1$-$C_2$ alkyl), wherein each of the $C_1$-$C_4$ alkyl groups in the foregoing $R^1$ groups may optionally contain one or two double or triple bonds;

$R^2$ is $C_1$-$C_{12}$ alkyl which may optionally contain from one to three double or triple bonds, aryl or ($C_1$-$C_4$ alkylene) aryl, wherein said aryl and the aryl moiety of said ($C_1$-$C_4$ alkylene)aryl is selected from phenyl, naphthyl, thienyl,

benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; $C_3$-$C_8$ cycloalkyl or ($C_1$-$C_6$ alkylene)($C_3$-$C_8$ cycloalkyl), wherein one or two of the carbon atoms of said cycloalkyl and the 5 to 8 membered cycloalkyl moieties of said ($C_1$-$C_6$ alkylene)($C_3$-$C_8$ cycloalkyl) may optionally and independently be replaced by an oxygen or sulfur atom or by $NZ^2$ wherein $Z^2$ is selected from hydrogen, $C_1$-$C_4$ alkyl, benzyl and $C_1$-$C_4$ alkanoyl, and wherein each of the foregoing $R^2$ groups may optionally be substituted with from one to three substituents independently selected from chloro, fluoro, hydroxy and $C_1$-$C_4$ alkyl, or with one substituent selected from bromo, iodo, $C_1$-$C_6$ alkoxy, -OC(=O)($C_1$-$C_6$ alkyl), -OC(=O)N($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl)($C_1$-$C_4$ alkyl), -N($C_1$-$C_4$ alkyl)-CO-($C_1$-$C_4$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -SH, -CN, -$NO_2$, -SO($C_1$-$C_4$ alkyl), -$SO_2$($C_1$-$C_4$ alkyl), -$SO_2$NH($C_1$-$C_4$ alkyl) and -$SO_2$N($C_1$-$C_4$ alkyl)($C_1$-$C_2$ alkyl);

-$NR^1R^2$ or $CR^1R^2R^{10}$ may form a saturated 3 to 8 membered carbocyclic ring which may optionally contain from one to three double bonds and wherein one or two of the ring carbon atoms of such 5 to 8 membered rings may optionally and independently be replaced by an oxygen or sulfur atom or by $NZ^3$ wherein $Z^3$ is hydrogen, $C_1$-$C_4$ alkyl, benzyl or $C_1$-$C_4$ alkanoyl;

$R^3$ is hydrogen, $C_1$-$C_4$ alkyl, -O($C_1$-$C_4$ alkyl), chloro, fluoro, bromo, iodo, ($C_1$-$C_2$ alkylene)-O-($C_1$-$C_2$ alkyl), ($C_1$-$C_2$ alkylene)-OH, or -S($C_1$-$C_4$ alkyl);

each $R^4$ is, independently, hydrogen, ($C_1$-$C_6$ alkyl), fluoro, chloro, bromo, iodo, hydroxy, cyano, amino, ($C_1$-$C_2$ alkylene)-OH, $CF_3$, $CH_2SCH_3$, nitro, -O($C_1$-$C_4$ alkyl), -N($C_1$-$C_4$ alkyl)($C_1$-$C_2$ alkyl), -S($C_1$-$C_4$ alkyl), -CO($C_1$-$C_4$ alkyl), -C(=O)H or -C(=O)O($C_1$-$C_4$alkyl);

$R^6$ is hydrogen, methyl or ethyl;

$R^8$ is hydrogen or $C_1$-$C_4$ alkyl;

$R^5$ is phenyl, pyridyl, pyrazinyl, pyrimidyl, pyridazinyl and wherein each of the foregoing $R^5$ groups is substituted with from one to four substituents $R^{13}$ wherein one to three of said substituents may be selected, independently, from fluoro, chloro, $C_1$-$C_6$ alkyl and -O($C_1$-$C_6$ alkyl) and one of said substituents may be selected from bromo, iodo, formyl, OH, ($C_1$-$C_4$ alkylene)-OH, ($C_1$-$C_4$alkylene)-O-($C_1$-$C_2$ alkyl), -CN, -$CF_3$, -$NO_2$, -$NH_2$, -NH($C_1$-$C_4$ alkyl), -N($C_1$-$C_2$ alkyl)($C_1$-$C_6$ alkyl), -OCO($C_1$-$C_4$ alkyl), ($C_1$-$C_4$ alkylene)-O-($C_1$-$C_4$ alkyl), -S($C_1$-$C_6$ alkyl), ($C_1$-$C_4$ alkylene)-S-($C_1$-$C_4$ alkyl), -C(=O)O($C_1$-$C_4$ alkyl), -C(=O)($C_1$-$C_4$ alkyl), -COOH, -$SO_2$NH($C_1$-$C_4$ alkyl), -$SO_2$N($C_1$-$C_2$ alkyl)($C_1$-$C_4$ alkyl), -$SO_2NH_2$, -$NHSO_2$($C_1$-$C_4$ alkyl), -S($C_1$-$C_6$ alkyl) and -$SO_2$($C_1$-$C_6$ alkyl), and wherein each of the $C_1$-$C_4$ alkyl and $C_1$-$C_6$ alkyl moieties in the foregoing $R^5$ groups may optionally have one or two double bonds;

$R^7$ is hydrogen, $C_1$-$C_4$ alkyl, halo (e.g., chloro, fluoro, iodo or bromo), hydroxy, -O($C_1$-$C_4$ alkyl), -C(=O)($C_1$-$C_4$ alkyl), -C(=O)O($C_1$-$C_4$ alkyl), -$OCF_3$, -$CF_3$, -$CH_2OH$ or -$CH_2O$($C_1$-$C_2$ alkyl);

$R^{10}$ is hydrogen, hydroxy, methoxy or fluoro;

$R^{11}$ is hydrogen or $C_1$-$C_4$ alkyl; and

with the proviso that: a) when both J and K are carbons and D is $CR^4$ and E is nitrogen, then G can not be nitrogen; (b) when both J and K are carbons and D and G are nitrogens, then E can not be $CR^4$ or C=O or C=S; (c) when both J and K are carbons and D and E are carbons, then G can not be nitrogen; (d) when G is carbon, it must be double banded to E; and (e) in the ring containing J, K, D, E and G, there can not be two double bonds adjacent to each other.

Another group of preferred CRF antagonists for use in the compositions, methods, and kits of the present invention are those wherein the CRF antagonist is a compound of formula:

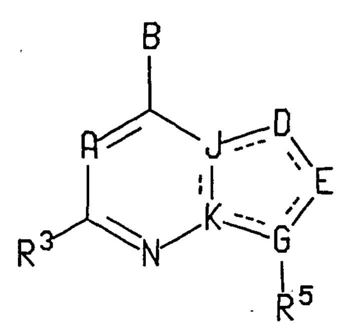

or a pharmaceutically acceptable salt thereof, wherein

the dashed lines represent optional double bonds;

A is nitrogen or $CR^7$;

B is $-NR^1R^2$, $-CR^1R^2R^{10}$ $-C(=CR^2R^{11})R^1$, $-NHCR^1R^2R^{10}$, $-OCR^1R^2R^{10}$, $-SCR^1R^2R^{10}$, $-CR^2R^{10}NHR^1$, $-CR^2R^{10}OR^1$, $-CR^2R^{10}SR^1$ or $-COR^2$;

J and K are each independently nitrogen or carbon and both J and K are not nitrogens;

D and E are each selected, independently, from nitrogen, $CR^4$, C=O, C=S, sulfur, oxygen, $CR^4R^6$ and $NR^8$;

G is nitrogen or carbon;

the ring containing D, E, G, K, and J in formula I may be a saturated or unsaturated 5-membered ring and may optionally contain one or two double bonds and may optionally contain from one to three heteroatoms in the ring and may optionally have one or two C=O or C=S groups;

$R^1$ is $C_1$-$C_6$ alkyl optionally substituted with one or two substituents independently selected from hydroxy, fluoro, chloro, bromo, iodo, -O-($C_1$-$C_4$ alkyl), $CF_3$, -C(=O)O-($C_1$-$C_4$alkyl), -OC(=O)($C_1$-$C_4$ alkyl), -OC(=O)N($C_1$-$C_4$ alkyl)($C_1$-$C_2$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl), wherein each of the $C_1$-$C_4$ alkyl groups in the foregoing $R^1$ groups may optionally contain one or two double or triple bonds;

$R^2$ is $C_1$-$C_{12}$ alkyl which may optionally contain from one to three double or triple bonds, aryl or ($C_1$-$C_4$ alkylene) aryl, wherein said aryl and the aryl moiety of said ($C_1$-$C_4$ alkylene)aryl is selected from phenyl, naphthyl, thienyl, benzothienyl, pyridyl; quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; $C_3$-$C_8$ cycloalkyl or ($C_1$-$C_6$ alkylene)($C_3$-$C_8$ cycloalkyl), wherein one or two of the carbon atoms of said cycloalkyl and the 5 to 8 membered cycloalkyl moieties of said ($C_1$-$C_6$ alkylene)($C_3$-$C_8$ cycloalkyl) may optionally and independently be replaced by an oxygen or sulfur atom or by $NZ^2$ wherein $Z^2$ is selected from hydrogen, $C_1$-$C_4$ alkyl, benzyl and $C_1$-$C_4$ alkanoyl, and wherein each of the foregoing $R^2$ groups may optionally be substituted with from one to three substituents independently selected from chloro, fluoro, hydroxy and $C_1$-$C_4$ alkyl, or with one substituent selected from bromo, iodo, $C_1$-$C_6$ alkoxy, -OC(=O)($C_1$-$C_6$ alkyl), -OC(=O)N($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl)($C_1$-$C_4$ alkyl), -N($C_1$-$C_4$ alkyl)-CO-($C_1$-$C_4$ alkyl), -NHCO($C_1$-$C_4$ alkyl), -COOH, -COO($C_1$-$C_4$ alkyl), -CONH($C_1$-$C_4$ alkyl), -CON($C_1$-$C_4$ alkyl)($C_1$-$C_2$ alkyl), -SH, -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl);

$-NR^1R^2$ or $CR^1R^2R^{10}$ may form a saturated 3 to 8 membered carbocyclic ring which may optionally contain from one to three double bonds and wherein one or two of the ring carbon atoms of such 5 to 8 membered rings may optionally and independently be replaced by an oxygen or sulfur atom or by $NZ^3$ wherein $Z^3$ is hydrogen, $C_1$-$C_4$ alkyl, benzyl or $C_1$-$C_4$ alkanoyl;

$R^3$ is hydrogen, $C_1$-$C_4$ alkyl, -O($C_1$-$C_4$ alkyl), chloro, fluoro, bromo, iodo, ($C_1$-$C_2$ alkylene)-O-($C_1$-$C_2$ alkyl), ($C_1$-$C_2$ alkylene)-OH, or -S($C_1$-$C_4$ alkyl);

each $R^4$ is, independently, hydrogen, ($C_1$-$C_6$ alkyl), fluoro, chloro, bromo, iodo, hydroxy, cyano, amino, ($C_1$-$C_2$ alkylene)-OH, $CF_3$, $CH_2SCH_3$, nitro, -O($C_1$-$C_4$ alkyl), - N($C_1$-$C_4$ alkyl)($C_1$-$C_2$ alkyl), -S($C_1$-$C_4$ alkyl), -CO($C_1$-$C_4$ alkyl), -C(=O)H or -C(=O)O($C_1$-$C_4$alkyl);

$R^6$ is hydrogen, methyl or ethyl;

$R^8$ is hydrogen or $C_1$-$C_4$ alkyl;

$R^5$ is phenyl, pyridyl, pyrazinyl, pyrimidyl, pyridazinyl and wherein each of the foregoing $R^5$ groups is substituted with from one to four substituents $R^{13}$ wherein one to three of said substituents may be selected, independently, from fluoro, chloro, $C_1$-$C_6$ alkyl and -O($C_1$-$C_6$ alkyl) and one of said substituents may be selected from bromo, iodo, formyl, OH, ($C_1$-$C_4$ alkylene)-OH, ($C_1$-$C_4$alkylene)-O-($C_1$-$C_2$ alkyl), -CN, $-CF_3$, $-NO_2$, $-NH_2$, -NH($C_1$-$C_4$ alkyl), -N($C_1$-$C_2$ alkyl)($C_1$-$C_6$ alkyl), -OCO($C_1$-$C_4$ alkyl), ($C_1$-$C_4$ alkylene)-O-($C_1$-$C_4$ alkyl), -S($C_1$-$C_6$ alkyl), ($C_1$-$C_4$ alkylene)-S-($C_1$-$C_4$ alkyl), -C(=O)O($C_1$-$C_4$ alkyl), -C(=O)($C_1$-$C_4$ alkyl), -COOH, $-SO_2NH$($C_1$-$C_4$ alkyl), $-SO_2N$($C_1$-$C_2$ alkyl)($C_1$-$C_4$ alkyl), $-SO_2NH_2$, $-NHSO_2$($C_1$-$C_4$ alkyl), -S($C_1$-$C_6$ alkyl) and $-SO_2$($C_1$-$C_6$ alkyl), and wherein each of the $C_1$-$C_4$ alkyl and $C_1$-$C_6$ alkyl moieties in the foregoing $R^5$ groups may optionally have one or two double bonds;

$R^7$ is hydrogen, $C_1$-$C_4$ alkyl, halo (e.g., chloro, fluoro, iodo or bromo), hydroxy, -O($C_1$-$C_4$ alkyl), -C(=O)($C_1$-$C_4$ alkyl), -C(=O)O($C_1$-$C_4$ alkyl), $-OCF_3$, $-CF_3$, $-CH_2OH$ or $-CH_2O$($C_1$-$C_2$ alkyl);

$R^{10}$ is hydrogen, hydroxy, methoxy or fluoro;

$R^{11}$ is hydrogen or $C_1$-$C_4$ alkyl; and

with the proviso that: a) when both J and K are carbons and D is $CR^4$ and E is nitrogen, then G can not be nitrogen; (b) when both J and K are carbons and D and G are nitrogens, then E can not be $CR^4$ or C=O or C=S; (c) when both J and K are carbons and D and E are carbons, then G can not be nitrogen; (d) when G is carbon, it must be double banded to E; and (e) in the ring containing J, K, D, E and G, there can not be two double bonds adjacent to each other.

Another group of preferred CRF antagonists for use in the compositions, methods, and kits of the present invention are those wherein the CRF antagonist is a compound of formula:

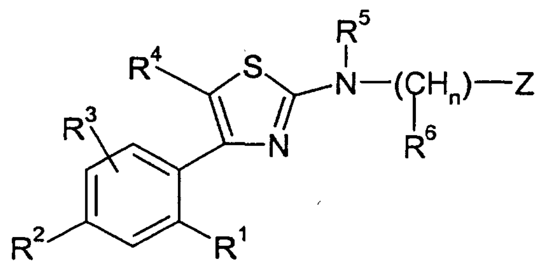

wherein each of $R^1$ and $R^2$ is independently a halogen atom; a $C_1$-$C_5$ hydroxyalkyl radical; $C_1$-$C_5$ alkyl; $C_7$-$C_{10}$ aralkyl; $C_1$-$C_5$ alkoxy; trifluoromethyl; nitro; nitrile; a group - SR where R is hydrogen, a $C_1$-$C_5$ alkyl radical or a $C_7$-$C_{10}$ aralkyl radical; a group S-CO-R where R is a $C_1$-$C_5$ alkyl radical or aralkyl in which the aryl portion is $C_6$-$C_8$ and the alkyl portion is $C_1$-$C_4$; a group -COOR' where R' is hydrogen or $C_1$-$C_5$ alkyl; a group -CONR'R" where R' and R" are as defined above for R'; a group -NR'R" where R' and R" are as previously defined for R'; a group -CONRaRb or NRaRb, where Ra and Rb, taken together with the nitrogen atom to which they are attached, form a 5-to 7-membered heterocyclic ring; or a group -NHCO-NR'R", where R' and R" are as defined above for R'; $R^3$ is hydrogen or as defined for $R^1$ and $R^2$ is a hydrogen atom; $C_{1-5}$ alkyl; halogen; a hydroxymethyl group; or a formyl group; $R^5$ is $C_1$-$C_5$ alkyl; a $C_3$-$C_7$ cycloalkyl group; a cycloalkylalkyl group in which the cycloalkyl portion is $C_3$-$C_7$ and the alkyl portion is $C_1$-$C_5$; or $C_5$-$C_6$ alkenyl; n is 0 or 1; $R^6$ is $C_{1-5}$ alkyl; alkoxyalkyl in which the alkyl portions are $C_1$-$C_5$; $C_3$-$C_7$ cycloalkyl; a cycloalkylalkyl group in which the cycloalkyl portion is $C_3$-$C_7$ and the alkyl portion is $C_1$-$C_5$; a cycloalkyloxyalkyl radical in which the cycloalkyl is $C_3$-$C_7$ and the alkyl is $C_1$-$C_4$; a hydroxyalkyloxyalkyl radical in which the alkyls are $C_2$-$C_{10}$; or an alkoxyalkyloxyalkyl radical in which the alkyls are $C_3$-$C_{12}$; and Z is an optionally substituted bi- or tricyclic aromatic or heteroaromatic group; or a stereoisomer or addition salt thereof.

Another group of preferred CRF antagonists for use in the compositions, methods, and kits of the present invention are those wherein the CRF antagonist is a compound of formula:

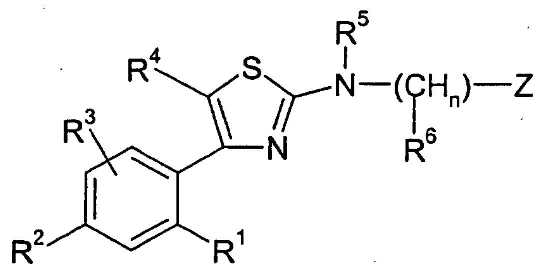

wherein each of $R^1$ and $R^2$ is independently a halogen atom; a $C_1$-$C_5$ hydroxyalkyl radical; $C_1$-$C_5$ alkyl; $C_7$-$C_{10}$ aralkyl; $C_1$-$C_5$ alkoxy; trifluoromethyl; nitro; nitrile; a group - SR where R is hydrogen, a $C_1$-$C_5$ alkyl radical or a $C_7$-$C_{10}$ aralkyl radical; a group S-CO-R where R is a $C_1$-$C_5$ alkyl radical or aralkyl in which the aryl portion is $C_6$-$C_8$ and the alkyl portion is $C_1$-$C_4$; a group -COOR' where R' is hydrogen or $C_1$-$C_5$ alkyl; a group -CONR'R" where R' and R" are as defined above for R'; a group -NR'R" where R' and R" are as previously defined for R'; a group -CONRaRb or NRaRb, where Ra and Rb, taken together with the nitrogen atom to which they are attached, form a 5-to 7-membered heterocyclic ring; or a group -NHCO-NR'R", where R' and R" are as defined above for R'; $R^3$ is hydrogen or as defined for $R^1$ and $R^2$ is a hydrogen atom; $C_{1-5}$ alkyl; halogen; a hydroxymethyl group; or a formyl group; $R^5$ is $C_1$-$C_5$ alkyl; a $C_3$-$C_7$ cycloalkyl group; a cycloalkylalkyl group in which the cycloalkyl portion is $C_3$-$C_7$ and the alkyl portion is $C_1$-$C_5$; or $C_5$-$C_6$ alkenyl; n is 0 or 1; $R^6$ is $C_{1-5}$ alkyl; alkoxyalkyl in which the alkyl portions are $C_1$-$C_5$; $C_3$-$C_7$ cycloalkyl; a cycloalkylalkyl group in which the cycloalkyl portion is $C_3$-$C_7$ and the alkyl portion is $C_1$-$C_5$; a cycloalkyloxyalkyl radical in which the cycloalkyl is $C_3$-$C_7$ and the alkyl is $C_1$-$C_4$; a hydroxyalkyloxyalkyl radical in which the alkyls are $C_2$-$C_{10}$; or an alkoxyalkyloxyalkyl radical in which the alkyls are $C_3$-$C_{12}$; and Z is an optionally substituted bi- or tricyclic aromatic or heteroaromatic group; or a stereoisomer or addition salt thereof.

Another group of preferred CRF antagonists for use in the compositions, methods, and kits of the present invention are those wherein the CRF antagonist is a compound of formula:

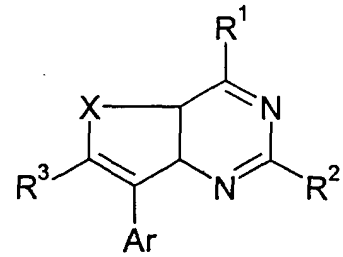

or a stereoisomer or pharmaceutically acceptable acid addition salt form thereof, wherein

X is S, SO or $SO_2$;
$R^1$ is $NR^4R^5$ or $OR^5$;
$R^2$ is $C_1$-$C_6$alkyl, $C_1$-$C_6$alkyloxy or $C_1$-$C_6$alkylthio;
$R^3$ is hydrogen, $C_1$-$C_6$alkyl, $C_1$-$C_6$alkylsulfonyl, $C_1$-$C_6$alkylsulfoxy or $C_1$-$C_6$alkylthio;
$R^4$ is hydrogen, $C_{1-6}$alkyl, mono- or di($C_3$-$C_6$cycloalkyl)methyl, $C_3$-$C_6$cycloalkyl, $C_3$-$C_6$alkenyl, hydroxy$C_1$-$C_6$alkyl, $C_1$-$C_6$alkylcarbonyloxy$C_1$-$C_6$alkyl or $C_1$-$C_6$alkyloxy$C_1$-$C_6$alkyl;
$R^5$ is $C_1$-$C_8$alkyl, mono- or di($C_3$-$C_6$cycloalkyl)methyl, $Ar^1CH_2$, $C_3$-$C_6$alkenyl, $C_1$-$C_6$alkyloxy$C_1$-$C_6$alkyl, hydroxy$C_1$-$C_6$alkyl, thienylmethyl, furanylmethyl, $C_1$-$C_6$alkylthio$C_1$-$C_6$alkyl, morpholinyl, mono- or di($C_1$-$C_6$alkyl)amino$C_1$-$C_6$alkyl, di($C_1$-$C_6$alkyl)amino, $C_1$-$C_6$alkylcarbonyl$C_1$-$C_6$alkyl, $C_1$-$C_6$alkyl substituted with imidazolyl; or a radical of formula -Alk-O-CO-Ar I;
or $R^4$ and $R^5$ taken together with the nitrogen atom to which they are attached may form a pyrrolidinyl, piperidinyl, homopiperidinyl or morpholinyl group, optionally substituted with $C_1$-$C_6$alkyl or $C_1$-$C_6$alkyloxy$C_1$-$C_6$alkyl;
Ar is phenyl; phenyl substituted with 1, 2 or 3 substituents independently selected from halo, $C_1$-$C_6$alkyl, trifluoromethyl, hydroxy, cyano, $C_1$-$C_6$alkyloxy, benzyloxy, $C_1$-$C_6$alkylthio, nitro, amino and mono- or di($C_1$-$C_6$alkyl)amino; pyridinyl; pyridinyl substituted with 1, 2 or 3 substituents independently selected from halo, $C_1$-$C_6$alkyl, trifluoromethyl, hydroxy, cyano, $C_1$-$C_6$alkyloxy, benzyloxy, $C_1$-$C_6$alkylthio, nitro, amino, mono- or di($C_1$-$C_6$alkyl)amino and piperidinyl; and wherein said substituted phenyl may optionally be further substituted with one or more halogens;
$Ar^1$ is phenyl; phenyl substituted with 1, 2 or 3 substituents each independently selected from halo, $C_1$-$C_6$alkyl, $C_1$-$C_6$alkyloxy, di($C_1$-$C_6$alkyl)amino$C_1$-$C_6$alkyl trifluoromethyl, and $C_1$-$C_6$alkyl substituted with morpholinyl; or pyridinyl; and Alk is $C_1$-$C_6$alkanediyl.

Another group of preferred CRF antagonists for use in the compositions, methods, and kits of the present invention are those wherein the CRF antagonist is a compound selected from the group consisting of:

4-(1-ethyl-propoxy)-3,6-dimethyl-2-(2,4,6-trimethylphenoxy)-pyridine;
butyl-[2,5-dimethyl-7-(2,4,6-trimethylphenyl)-6,7-dihydro-5H-pyrrolo[2,3-d]pyrimidin-4-yl]-ethyl-amine;
4-(butyl-ethylamino)-2,5-dimethyl-7-(2,4,6-trimethylphenyl)-5,7-dihydropyrrolo[2,3-d]pyrimidin-6-one;
4-(1-ethylpropoxy)-2,5-dimethyl-6-(2,4,6-trimethylphenoxy)-pyrimidine;
N-butyl-N-ethyl-2,5-dimethyl-NN-(2,4,6-trimethylphenyl)-pyrimidine-4,6-diamine;
[4-(1-ethyl-propoxy)-3,6-dimethyl-pyridin-2-yl]-(2,4,6-trimethylphenyl)-amine;
6-(ethyl-propyl-amino)-2,7-dimethyl-9-(2,4,6-trimethylphenyl)-7,9-dihydropurin-8-one;
3-{(4-methyl-benzyl)-[3,6-dimethyl-1-(2,4,6-trimethylphenyl)-1H-pyrazolo[3,4-d]pyrimidin-4-yl]-amino}-propan-1-ol;
diethyl-[6-methyl-3-methylsulfanyl-1-(2,4,6-trichlorophenyl)-1H-pyrazolo[3,4-d]pyrimidin-4-yl]-amine;
2-{butyl-[6-methyl-3-methylsulfanyl-1-(2,4,6-trichlorophenyl)-1H-pyrazolo[3,4-d]pyrimidin-4-yl]-amino}-ethanol;
dibutyl-[6-methyl-3-methylsulfanyl-1-(2,4,6-trichlorophenyl)-1H-pyrazolo[3,4-d]pyrimidin-4-yl}-amine;
butyl-ethyl-[6-methyl-3-methylsulfanyl-1-(2,4,6-trichlorophenyl)-1H-pyrazolo[3,4-d]pyrimidin-4-yl]-amine;
butyl-ethyl-[6-methyl-3-methylsulfonyl-1-(2,4,6-trichlorophenyl)-1H-pyrazolo[3,4-d]pyrimidin-4-yl]-amine;
butyl-cyclopropylmethyl-[6-methyl-3-methylsulfanyl-1-(2,4,6-trichlorophenyl)-1H-pyrazolo[3,4-d]pyrimidin-4-yl]-amine;
di-1-propyl-[6-methyl-3-methylsulfanyl-1-(2,4,6-trichlorophenyl)-1H-pyrazolo[3,4-d]pyrimidin-4-yl]-amine;
diallyl-[6-methyl-3-methylsulfanyl-1-(2,4,6-trichlorophenyl)-1H-pyrazolo[3,4-d]pyrimidin-4-yl]-amine;
butyl-ethyl-[6-chloro-3-methylsulfanyl-1-(2,4,6-trichlorophenyl)-1H-pyrazolo[3,4-d]pyrimidin-4-yl]-amine;
butyl-ethyl-[6-methoxy-3-methylsulfanyl-1-(2,4,6-trichlorophenyl)-1H-pyrazolo[3,4-d]pyrimidin-4-yl]-amine;
propyl-ethyl-[3,6-dimethyl-1-(2,4,6-trimethylphenyl)-1H-pyrazolo[3,4-d]pyrimidin-4-yl]-amine;

4-(1-ethyl-propyl )-6-methyl-3-methylsulfanyl-1-(2,4,6-trimethylphenyl)-1H-pyrazolo[3,4-d]pyrimidine;
n-butyl-ethyl-[2,5-dimethyl-7-(2,4,6-trimethylphenyl)-7H-pyrrolo[2,3-d]pyrimidin-4-yl]amine;
di-n-propyl-[2,5-dimethyl-7-(2,4,6-trimethylphenyl)-7H-pyrrolo[2,3-d]pyrimidin-4-yl]amine;
ethyl-n-propyl-[2,5-dimethyl-7-(2,4,6-trimethylphenyl)-7H-pyrrolo[2,3-d]pyrimidin-4-yl]amine;
diethyl-2,5-dimethyl-7-(2,4,6-trimethylphenyl)-7H-pyrrolo[2,3-d]pyrimidin-4-yl]amine;
n-butyl-ethyl-[2,5,6-trimethyl-7-(2,4,6-trimethylphenyl)-7H-pyrrolo[2,3-d]pyrimidin-4-yl]amine;
2-{N-n-butyl-N-[2,5-dimethyl-7-(2,4,6-trimethylphenyl)-7H-pyrrolo[2,3-d]pyrimidin-4-yl]amino}-ethanol;
4-(1-ethyl-propyl)-2,5,6-trimethyl-7-(2,4,6-trimethylphenyl)-7H-pyrrolo[2,3-d]pyrimidine;
n-butyl-ethyl-[2,5-dimethyl-7-(2,4-dimethylphenyl)-7H-pyrrolo[2,3-d]pyrimidin-4-yl]amine;
2,5-dimethyl-7-(2,4,6-trimethylphenyl)-7H-pyrrolo[2,3-d]pyrimidyl-4-yl]-(1-ethylpropyl)amine;
butyl-[3,6-dimethyl-1-(2,4,6-trimethylphenyl)-1H-pyrazolo[3,4-b]pyridin-4-yl]-ethylamine;
[3,6-dimethyl-1-(2,4,6-trimethylphenyl)-1H-pyrazolo[3,4,b]pyridin-4-yl]-(1-methoxymethylpropyl)-amine;
4-(1-methoxymethylpropoxy)-3,6-dimethyl-1-(2,4,6-trimethylphenyl)-1H-pyrazolo[3,4-b]pyridine;
(1-ethylpropyl)-[3,5,6-trimethyl-1-(2,4,6-trimethylphenyl)-1H-pyrazolo[3,4-b]pyridin-4-yl]-amine;
4-(1-ethylpropoxy)-2,5-dimethyl-7-(2,4,6-trimethylphenyl)-7H-pyrrolo[2,3-b]pyridine;
4-(1-ethylpropoxy)-2,5,6-trimethyl-7-(2,4,6-trimethylphenyl)-7H-pyrrolo[2,3-b]pyridine;
4-(1-ethylpropoxy)-2,5-dimethyl-7-(2,6-dimethyl-4-bromophenyl)-7H-pyrrolo[2,3-b]pyridine;
2,5,6-trimethyl-7-(1-propylbutyl)-4-(2,4,6-trimethylphenoxy)-7H-pyrrolo[2,3-d]pyrimidine;
1-(1-ethylpropyl)-6-methyl-4-(2,4,6-trimethylphenylamino)-1,3-dihydro-imidazo[4,5-c]pyridin-2-one;
9-(1-ethylpropyl)-2-methyl-6-(2,4,6-trimethylphenylamino)-7,9-dihydro-purin-8-one;
1-(1-ethylpropyl)-6-methyl-4-(2,4,6-trimethylphenoxy)-1,3-dihydro-imidazo[4,5-c]pyridin-2-one;
1-(1-ethylpropyl)-6-methyl-4-(2,4,6-trimethylphenoxy)-1H-imidazo[4,5-c]pyridine;
1-(1-ethylpropyl)-3,6-dimethyl-4-(2,4,6-trimethylphenoxy)-1,3-dihydro-imidazo[4,5-c]pyridin-2-one;
1-(1-ethylpropyl)-3,6-dimethyl-4-(2,4,6-trimethylphenylamino)-1,3-dihydro-imidazo[4,5-c]pyridin-2-one;
1-(1-ethyl-propyl)-4,7-dimethyl-5-(2,4,6-trimethyl-phenoxy)-1,4-dihydro-2H-pyrido[3,4-b]pyrazin-3-one;
1-(1-ethyl-propyl)-4,7-dimethyl-5-(2,4,6-trimethyl-phenoxy)-1,2,3,4-tetrahydro-pyrido[3,4-b]pyrazine;
1-(1-ethyl-propyl)-7-methyl-5-(2,4,6-trimethyl-phenoxy)-1,2,3,4-tetrahydro-pyrido[3,4-b]pyrazine;
1-(1-ethyl-propyl)-7-methyl-2-oxo-5-(2,4,6-trimethyl-phenoxy)-1,2,3,4-tetrahydro-[1,6]naphthyridine-3-carboxylic acid methyl ester;
1-(1-ethyl-propyl)-7-methyl-2-oxo-5-(2,4,6-trimethyl-phenoxy)-1,2,3,4-tetrahydro-[1,6]naphthyridine-3-carboxylic acid isopropyl ester;
1-(1-ethyl-propyl)-7-methyl-5-(2,4,6-trimethyl-phenoxy)-3,4-dihydro-1H-[1,6]naphthyridin-2-one;
1-(1-ethyl-propyl)-7-methyl-5-(2,4,6-trimethyl-phenoxy)-1,2,3,4-tetrahydro-[1,6]naphthyridine;
1-(1-ethyl-propyl)-7-methyl-5-(2,4,6-trimethyl-phenoxy)-1,4-dihydro-2H-3-oxa-1,6-diaza-naphthalene;
1-(1-ethyl-propyl)-4,7-dimethyl-5-(2,4,6-trimethyl-phenoxy)-1,4-dihydro-2H-3-oxa-1,6-diaza-naphthalene;
1-(1-ethyl-propyl)-3,7-dimethyl-5-(2,4,6-trimethyl-phenoxy)-3,4-dihydro-1H-3-oxa-[1,6]-naphthyridin-2-one;
1-(1-ethyl-propyl)-3,3,6-trimethyl-4-(2,4,6-trimethyl-phenoxy)-2,3-dihydro-1H-pyrrolo[3,2-c]pyridine;
7-(1-ethyl-propoxy)-5-methyl-3-(2,4,6-trimethyl-phenyl)-pyrazolo[1,5-a]pyrimidine;
[2,5-dimethyl-3-(2,4,6-trimethyl-phenyl)-pyrazolo[1,5-a]pyrimidin-7-yl]-(1-ethylpropyl)-amine;
(1-ethyl-propyl)-[5-methyl-3-(2,4,6-trimethyl-phenyl)-pyrazolo[1,5-a]pyrimidin-7-yl]-amine;
7-(1-ethyl-propoxy)-2,5-dimethyl-3-(2,4,6-trimethyl-phenyl)-pyrazolo[1,5-a]pyrimidine;
[2,5-dimethyl-3-(2,4,6-trimethyl-phenyl)-pyrazolo[1,5-a]pyrimidin-7-yl]-ethyl-propyl-amine;
[6-bromo-5-bromomethyl-3-(2,4,6-trimethyl-phenyl)-3H-[1,2,3]triazolo[4,5-b]pyridin-7-yl]-(1-ethyl-propyl)-amine;
(1-ethyl-propyl)-[5-methyl-3-(2,4,6-trimethyl-phenyl)-3H-[1,2,3]triazolo[4,5-b]pyridin-7-yl]-amine;
[6-bromo-5-methyl-3-(2,4,6-trimethyl-phenyl)-3H-[1,2,3]triazolo[4,5-b]pyridin-7-yl]-( 1-ethyl-propyl)-methyl-amine;
7-(1-ethyl-propoxy)-5-methyl-3-(2,4,6-trimethyl-phenyl)-3H-[1,2,3]triazolo[4,5-b]pyridine;
4-(1-ethyl-propoxy)-2,5-dimethyl-7-(2,4,6-trimethyl-phenyl)-5H-pyrrolo[3,2-d]pyrimidine;
(±)-2,5-dimethyl-4-(tetrahydro-furan-3-yloxy)-7-(2,4,6-trimethyl-phenyl)-5H-pyrrolo-[3,2-d]pyrimidine;
2,5-dimethyl-4-(S)-(tetrahydro-furan-3-yloxy)-7-(2,4,6-trimethyl-phenyl)-5H-pyrrolo-[3,2-d]pyrimidine;
2,5-dimethyl-4-(1-propyl-butoxy)-7-(2,4,6-trimethyl-phenyl )-5H-pyrrolo[3,2-d]pyrimidine;
4-sec-butylsulfanyl-2,5-dimethyl-7-(2,4,6-trimethyl-phenyl)-5H-pyrrolo[3,2-d]pyrimidine;
4-(butyl-ethyl-amino)-2,6-dimethyl-8-(2,4,6-trimethyl-phenyl)-5,8-dihydro-6H-pyrido[2,3-d]pyrimidin-7-one;
8-(1-ethyl-propoxy)-6-methyl-4-(2,4,6-trimethyl-phenyl)-3,4-dihydro-1H-pyrido[2,3-b] pyrazin-2-one;
8-(1-ethyl-propoxy)-6-methyl-4-(2,4,6-trimethyl-phenyl)-1,2,3,4-tetrahydro-pyrido [2,3-b]pyrazine;
4-(1-ethyl-propoxy)-2-methyl-8-(2,4,6-trimethyl-phenyl)-quinoline;
5-(1-ethyl-propoxy)-7-methyl-1-(2,4,6-trimethyl-phenyl)-1,4-dihydro-2H-3-oxa-1,8-diaza-naphthalene;
5-(1-ethyl-propoxy)-7-methyl-1-(2,4,6-trimethyl-phenyl)-1,2-dihydro-3-oxa-1,8-diaza-naphthalen-4-one;

8-(1-ethyl-propoxy)-1,6-dimethyl-4-(2,4,6-trimethyl-phenyl)-1,2,3,4-tetrahydro-pyrido[2,3-b]pyrazine;
(1-ethyl-propyl)-[2-methyl-8-(2,4,6-trimethyl-phenyl)-quinolin-4-yl]-amine;
4-(butyl-ethyl-amino)-2,6-dimethyl-8-(2,6-dimethyl-4-bromo-phenyl)-5,8-dihydro-6H-pyrido[2,3-d]pyrimidin-7-one;
4-(butyl-ethyl-amino)-2-methyl-8-(2,6-dimethyl-4-bromo-phenyl)-5,8-dihydro-6 H- pyrido[2,3-d]pydmidin-7-one;
4-(1-ethyl-propoxy)-2-methyl-8-(2,6-dimethyl-4-bromo-phenyl)-5,8-dihydro-6H-pyrido[2,3-d]pyrimidin-7-one;
(butyl-ethyl)-[2-methyl-8-(2,6-dimethyl-4-bromo-phenyl)-5,6,7,8-tetrahydro-pyrido[2,3-d]pyrimidin-4-yl]-amine;
(propyl-ethyl)-[2-methyl-8-(2,6-dimethyl-4-bromo-phenyl)-5,6,7,8-tetrahydro-pyrido[2,3-d]pyrimidin-4-yl]-amine;
(diethyl)-[2-methyl-8-(2,6-dimethyl-4-bromo-phenyl )-5,6,7,8-tetrahydro-pyrido [2,3-d]pyrimidin-4-yl]-amine;
(1-ethyl-propyl)-[2-methyl-8-(2,6-dimethyl-4-bromo-phenyl)-5,6,7,8-tetrahydro-pyrido[2,3-d]pyrimidin-4-yl]-amine;
(1-ethyl-propoxy)-2-methyl-8-(2,6-dimethyl-4-bromo-phenyl)-5, 6,7,8-tetrahydro- pyrido[2,3-d]pyrimidine;
4-(butyl-ethyl-amino)-2-methyl-8-(2,4,6-trimethyl-phenyl)-5,8-dihydro-6H-pyrido[2,3-d]pyrimidin-7-one;
4-(1-ethyl-propoxy)-2-methyl-8-(2,4,6-trimethyl-phenyl)-5,8-dihydro-6H-pyrido [2,3-d]pyrimidin-7-one;
(butyl-ethyl)-[2-methyl-8-(2,4,6-trimethyl-phenyl)-5,6,7,8-tetrahydro-pyrido[2,3-d] pyrimidin-4-yl]-amine;
(propyl-ethyl)-[2-methyl-8-(2,4,6-trimethyl-phenyl)-5,6,7,8-tetrahydro-pyrido-[2,3-d] pyrimidin-4-yl]-amine;
(diethyl)-[2-methyl-8-(2,4,6-trimethyl-phenyl)-5,6,7,8-tetrahydro-pyrido[2,3-d] pyrimidin-4-yl]-amine;
(1-ethyl-propyl)-[2-methyl-8-(2,4,6-trimethyl-phenyl)-5,6,7,8-tetrahydro-pyrido[2,3-d] pyrimidin-4-yl]-amine;
(1-ethyl-propoxy)-2-methyl-8-(2,4,6-trimethyl-phenyl)-5,6,7,8-tetrahydro-pyrido[2,3-d] pyrimidine;
8-(1-ethyl-propoxy)-6-methyl-4-(2,6-dimethyl-4-bromo-phenyl)-3,4-dihydro-1H-pyrido [2,3-b]pyrazin-2-one;
8-(1-ethyl-propoxy)-6-methyl-4-(2,6-dimethyl-4-bromo-phenyl)-1,2,3,4-tetrahydro- pyrido[2,3-b]pyrazine;
4-(1-ethyl-propoxy)-2-methyl-8-(2,6-dimethyl-4-bromo-phenyl)-quinoline;
5-(1-ethyl-propoxy)-7-methyl-1-(2,6-dimethyl-4-bromo-phenyl)-1,4-dihydro-2H-3-oxa-1,8-diaza-naphthalene;
5-(1-ethyl-propoxy)-7-methyl-1-(2,6-dimethyl-4-bromo-phenyl)-1,2-dihydro-3-oxa-1,8-diaza-naphthalen-4-one;
8-(1-ethyl-propoxy)-1,6-dimethyl-4-(2,6-dimethyl-4-bromo-phenyl)-1,2,3,4-tetrahydro-pyrido[2,3-b]pyrazine;
(1-ethyl-propyl)-[2-methyl-8-(2,6-dimethyl-4-bromo-phenyl)-quinolin-4-yl]-amine;
4-(butyl-ethyl-amino)-2,6-dimethyl-8-(2,6-dimethy(-4-chloro-phenyl)-5,8-dihydro-6H-pyrido[2,3-d]pyrimidin-7-one;
8-(1-ethyl-propoxy)-6-methyl-4-(2,6-dimethyl-4-chloro-phenyl)-3,4-dihydro-1H-pyrido[2,3-b]pyrazin-2-one;
8-(1-ethyl-propoxy)-6-methyl-4-(2,6-dimethyl-4-chloro-phenyl)-1,2,3,4-tetrahydro- pyrido[2,3-b]pyrazine;
4-(1-ethyl-propoxy)-2-methyl-8-(2,6-dimethyl-4-chloro-phenyl)-quinoline;
5-(1-ethyl-propoxy)-7-methyl-1-(2,6-dimethyl-4-chloro-phenyl)-1,4-dihydro-2H-3-oxa-1,8-diaza-naphthalene;
5-(1-ethyl-propoxy)-7-methyl-1-(2,6-d imethyl-4-chloro-phenyl)-1,2-dihydro-3-oxa-1,8-diaza-naphthalen-4-one;
8-(1-ethyl-propoxy)-1,6-dimethyl-4-(2,6-dimethyl-4-chloro-phenyl)-1,2,3,4-tetrahydro-pyrido[2,3-b]pyrazine;
(1-ethyl-propyl)-[2-methyl-8-(2,6-dimethyl-4-chloro-phenyl)-quinolin-4-yl]-amine;
8-(1-hydroxymethyl-propoxy)-6-methyl-4-(2,4,6-trimethyl-phenyl)-3,4-dihydro-1H-pyrido[2,3-b]pyrazin-2-one;
8-(1-hydroxymethyl-propylamino)-6-methyl-4-(2,4,6-trimethyl-phenyl)-3,4-dihydro-1H-pyrido[2,3-b]pyrazin-2-one;
8-(1-ethyl-propylamino)-6-methyl-4-(2,4,6-trimethyl-phenyl)-3,4-dihydro-1H-pyrido[2,3-b]pyrazin-2-one;
8-diethylamino-6-methyl-4-(2,4,6-trimethyl-phenyl)-3,4-dihydro-1H-pyrido-[2,3-b] pyrazin-2-one;
8-(ethyl-propyl-amino)-6-methyl-4-(2,4,6-trimethyl-phenyl)-3,4-dihydro-1H-pyrido[2,3-b]pyrazin-2-one;
8-(butyl-ethyl-amino)-6-methyl-4-(2,4,6-trimethyl-phenyl)-3,4-dihydro-1H-pyrido [2,3-b]pyrazin-2-one;
8-(1-hydroxymethyl-propoxy)-6-methyl-4-(2,4,6-trimethyl-phenyl)-1,2,3,4-tetrahydro-pyrido[2,3-b]pyrazine;
8-(1-hydroxymethyl-propylamino)-6-methyl-4-(2,4,6-trimethyl-phenyl)-1,2,3,4-tetrahydro-pyrido[2,3-b]pyrazine;
8-(1-ethyl-propylamino)-6-methyl-4-(2,4,6-trimethyl-phenyl)-1,2,3,4-tetrahydro-pyrido[2,3-b]pyrazine;
8-diethylamino-6-methyl-4-(2,4,6-trimethyl-phenyl)-1,2,3,4-tetrahydro-pyrido[2,3-b]pyrazine;
8-(ethyl-propyl-amino)-6-methyl-4-(2,4,6-trimethyl-phenyl)-1,2,3,4-tetrahydro-pyrido[2,3-b]pyrazine;
8-(butyl-ethyl-amino)-6-methyl-4-(2,4,6-trimethyl-phenyl )-1,2,3,4-tetrahydro-pyrido[2,3-b]pyrazine;
4-(1-hydroxymethyl-propoxy)-2-methyl-8-(2,4,6-trimethyl-phenyl)-quinoline;
4-(1-hyd roxymethyl-propylamino)-2-methyl-8-(2,4,6-trimethyl-phenyl )-quinoline;
4-(1-ethyl-propylamino)-2-methyl-8-(2,4,6-trimethyl-phenyl)-quinoline;
4-diethylamino-2-methyl-8-(2,4,6-trimethyl-phenyl)-quinoline;
4-(ethyl-propyl-amino)-2-methyl-8-(2,4,6-trimethyl-phenyl)-quinoline;
4-(butyl-ethyl-amino)-2-methyl-8-(2,4,6-trimethyl-phenyl)-quinoline;
5-(1-hydroxymethyl-propoxy)-7-methyl-1-(2,4,6-trimethyl-phenyl)-1,4-dihydro-2H-3-oxa-1,8-diaza-naphthalene;
5-(1-hydroxymethyl-propylamino)-7-methyl-1-(2,4,6-trimethyl-phenyl)-1,4-dihydro-2H-3-oxa-1,8-diaza-naphthalene;
5-(1-ethyl-propylamino)-7-methyl-1-(2,4,6-trimethyl-phenyl)-1,4-dihydro-2H-3-oxa-1,8-diaza-naphthalene;

5-diethylamino-5-methyl-1-(2,4,6-trimethyl-phenyl)-1,4-dihydro-2H-3-oxa-1,8-diaza-naphthalene;
5-(ethyl-propyl-amino)-7-methyl-1-(2,4,6-trimethyl-phenyl)-1,4-dihydro-2H-3-oxa-1,8-diaza-naphthalene;
8-(butyl-ethyl-amino)-6-methyl-4-(2,4,6-trimethyl-phenyl)-1,4-dihydro-2H-3-oxa-1,8-diaza-naphthalene;
4-(2,4-dichlorophenyl)-5-methyl-2-[N-(1-(methoxymethyl)-1-(naphth-2-yl) methyl)-N-propylamino]thiazole;
oxalate of 4-(2,4-dichlorophenyl)-5-methyl-2-[N-(6-methoxyisoquinol-5-yl)-N-propylamino]thiazole;
oxalate of 4-(2-chloro-4-methoxyphenyl)-5-methyl-2-[N-(6-methylisoquinol-5 -yl)-N-propylamino]thiazole;
4-(2-chloro-4-methoxyphenyl)-5-methyl-2-[N-(1-methoxycarbonylmethylindol-5-yl)-N-propylamino]thiazole;
oxalate of 4-(2-chloro-4-methoxyphenyl)-5-methyl-2-[N-(6-methoxyisoquinol-5-yl)-N-propylamino]thiazole;
oxalate of 4-(2-chloro-4-methoxyphenyl)-5-methyl-2-[N-(6-chloroisoquinol-5 -yl)-N- propylamino]thiazole;
oxalate of 4-(2-chloro-4-methoxyphenyl)-5-methyl-2-[N-(6-methoxyisoquinol-5 -yl)-N- propylamino]thiazole;
4-(2-chloro-4-methoxyphenyl)-5-methyl-2-[N-1-methoxynaphth-2-yl)-N-propylamino]thiazole;
oxalate of 4-(2-chloro-4-trifluoromethylphenyl)-5-methyl-2-[N-6-methoxyisoquinol-5-yl)-N-propylamino]thiazole;
chlorhydrate of 4-(2-chloro-4-methoxyphenyl)-5-methyl-2-[N-(2-ethoxynaphth-1-yl)-N- propylamino]thiazole;
chlorhydrate of 4-(2-chloro-4-methoxyphenyl)-5-methyl-2[N-(2,3-dimethylnaphth-1-yl)-N-propylamino]thiazole;
chlorhydrate of 4-(2-chloro-4-methoxyphenyl)-5-methyl-2-[N-(6-bromo-2-methoxynaphth-1-yl)-N-propylamino]thiazole;
chlorhydrate of 4-(2-chloro-4-methoxyphenyl)-5-methyl-2-[N-(2,6-dimethylnaphth-1-yl)-N-propylamino]thiazole;
chlorhydrate of 4-(2-chloro-4-methoxyphenyl)-5-methyl-2-[N-(1-(methoxymethyl)-1-(naphth-2-yl)methyl)-N-propylamino]thiazole;
chlorhydrate of 4-(2-chloro-4-methoxyphenyl)-5-methyl-2-[N-(1-(cyclopropyl)-1-(naphth-2-yl)methyl)-N-propylamino]thiazole;
3-(2,4-dichlorophenyl)-5-methyl-7(N-propyl-N-cyclopropanemethylamino)-pyrazolo[2,3-a]pyrimidine;
3-(2,4-dichlorophenyl)-5-methyl-7-(N-allyl-N-cyclopropanemethylamino)-pyrazolo[2,3-a]pyrimidine;
2-methylthio-3-(2,4-dichlorophenyl)-5-methyl-7-(N,N-diallylamino)-pyrazolo[2,3-a]pyrimidine;
2-methylthio-3-(2,4-dichlorophenyl)-5-methyl-7-(N-butyl-N-cyclopropane-methyl-amino)pyrazolo[2,3-a]pyrimidine;
2-methylthio-3-(2,4-dichlorophenyl)-5-methyl-7-(N-propyl-N-cyclopropane-methyl-amino)pyrazolo[2,3-a]pyrimidine;
2-methyl-3-(4-chlorophenyl)-5-methyl-7-(N,N-dipropylamino)-pyrazolo[2,3-a] pyrimidine;
3-[6-(dimethylamino)-3-pyridinyl-2,5-dimethyl-N,N-dipropylpyrazolo[2,3-a] pyrimidin-7-amine;
3-[6-(dimethylamino)-4-methyl-3-pyridinyl]-2,5-dimethyl-N,N-dipropyl-pyrazolo[2,3-a]pyrimidine-7-amine;
3-(2,4-dimethoxyphenyl)-2,5-dimethyl-7-(N-propyl-N-methyloxyethylamino)-pyrazolo(2,3-a)pyrimidine;
7-(N-diethylamino)-2,5-dimethyl-3-(2-methyl-4-methoxyphenyl-[1,5-a]-pyrazolopyrimidine;
7-(N-(3-cyanopropyl)-N-propylamino-2,5,dimethyl-3-(2,4-dimethylphenyl)-[1,5-a]-pyrazolopyrimidine;
[3,6-dimethyl-2-(2,4,6-trimethyl-phenoxy)-pyridin-4-yl]-(1-ethyl-propyl)-amine;
[2-(4-chloro-2,6-dimethyl-phenoxy)-3,6-dimethyl-pyridin-4-yl]-(1-ethyl-propyl)-amine;
cyclopropylmethyl-[3-(2,4-dimethyl-phenyl)-2,5-dimethyl-pyrazolo[1,5-a]pyrimidin-7-yl]-propyl-amine;
cyclopropylmethyl-[3-(2-methyl-4-chloro-phenyl)-2,5-dimethyl-pyrazolo[1,5-a]pyrimidin-7-yl]-propyl-amine;
cyclopropylmethyl-[3-(2,4-di-chloro-phenyl)-2,5-dimethyl-pyrazolo[1,5-a]pyrimidin-7-yl]-propyl-amine;
[3-(2-methyl-4-chloro-phenyl)-2,5-dimethyl-pyrazolo[1,5-a]pyrimidin-7-yl]-di-propyl-amine;
[2,5-dimethyl-3-(2,4-dimethyl-phenyl)-pyrazolo[1,5-a]pyrimidin-7-yl]-( 1-ethylpropyl)-amine;
[2,5-dimethyl-3-(2,4-dichloro-phenyl)-pyrazolo[1,5-a]pyrimidin-7-yl]-(1-ethyl-propyl)-amine;
4-(1-ethyl-propylamino)-6-methyl-2-(2,4,6-trimethyl-phenoxy)-nicotinic acid methyl ester;
3-[6-(dimethylamino)-4-methyl-3-pyridinyl]-2,5-dimethyl-N-propyl-N-cyclopropylmethyl-pyrazolo[2,3-a]pyrimidin-7-amine; and
3-[6-(dimethylamino)-4-methyl-3-pyridinyl]-2,5-dimethyl-N-ethyl-N-cyclopropylmethyl-pyrazolo[2,3-a]pyrimidin-7-amine.

Another group of useful CRF antagonists for use in the compositions, methods, and kits of the present invention are those wherein the CRF antagonist is a compound of the following formula, disclosed in WO 95/10506:

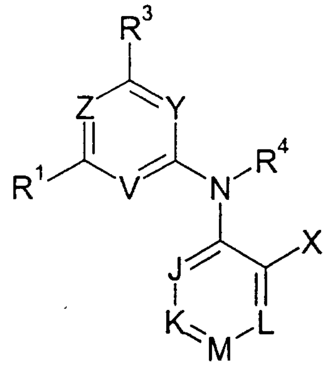

or a pharmaceutically acceptable salt or prodrug thereof, wherein Y is $CR^{3a}$, N, or $CR^{29}$; when

Y is $CR^{3a}$ or N:

$R^1$ is independently selected at each occurrence from the group consisting of $C_1$-$C_4$ alkyl, $C_2$-$C_4$ alkenyl, $C_2$-$C_4$ alkynyl, halogen, $C_1$-$C_2$ haloalkyl, $NR^6R^7$, $OR^8$, and $S(O)_nR^8$;

$R^3$ is $C_1$-$C_4$ alkyl, aryl, $C_3$-$C_6$ cycloalkyl, $C_1$-$C_2$ haloalkyl, halogen, nitro, $NR^6R^7$, $OR^8$, $S(O)_nR^8$ $C(=O)R^9$, $C(=O)NR^6R^7$, $C(=S)NR^6R^7$, $-(CHR^{16})_kNR^6R^7$, $(CH_2)_kOR^8$, $C(=O)NR^{10}CH(R^{11})CO_2R^{12}$, $-C(OH)(R^{25})(R^{25a})$, $-(CH_2)_pS(O)_n$-alkyl, $-(CHR^{16})R^{25}$, $-C(CN)(R^{25})(R^{16})$ provided that $R^{25}$ is not -NH- containing rings, $-C(=O)R^{25}$, $-CH(CO_2R^{16})_2$, $NR^{10}C(=O)CH(R^{11})NR^{10}R^{12}$, $NR^{10}CH(R^{11})CO_2R^{12}$; substituted $C_1$-$C_4$ alkyl, substituted $C_2$-$C_4$ alkenyl, substituted $C_2$-$C_4$ alkynyl, substituted $C_1$-$C_4$ alkoxy, aryl-(substituted $C_1$-$C_4$) alkyl, aryl-(substituted $C_1$-$C_4$) alkoxy, substituted $C_3$-$C_6$ cycloalkyl, amino-(substituted $C_1$-$C_4$)alkyl, substituted $C_1$-$C_4$ alkylamino, where substitution by $R^{27}$ can occur on any carbon containing substituent; 2-pyridinyl, imidazolyl, 3-pyridinyl, 4-pyridinyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, phenyl, 1*H*-indazolyl, 2-pyrrolidonyl, *2H,6H*-1,5,2-dithiazinyl, 2*H*-pyrrolyl, 3*H*-indolyl, 4-piperidonyl, 4a*H*-carbazolyl, 4*H*-quinolizinyl, 6*H*-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, imidazolidinyl, indolinyl, indolizinyl, indolyl, isobenzofuranyl, isochromanyl, isoindolinyl, isoindolyl, isoquinolinyl, benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenoxathiinyl, phenoxazinyl, phthalazinyl, piperazinyl, piperidinyl, pteridinyl, purinyl, pyranyl, pyrazolidinyl, pyrazolinyl, pyrazolyl, pyridazinyl, pyrimidinyl, pyrrolidinyl, pyrrolinyl, pyrrolyl, quinazolinyl, quinolinyl, quinoxalinyl, quinuclidinyl, β-carbolinyl, tetrahydrofuranyl, tetrahydroisoquinolinyl, tetrahydroquinolinyl, tetrazolyl, thianthrenyl, thiazolyl, thiophenyl, triazinyl, xanthenyl; or 1-tetrahydroquinolinyl or 2-tetrahydroisoquinolinyl either of which can be substituted with 0-3 groups chosen from keto and $C_1$-$C_4$ alkyl;

J, K, and L are independently selected at each occurrence from the group of N, CH, and CX';

M is $CR^5$ or N;

V is $CR^{1a}$ or N;

Z is $CR^2$ or N;

$R^{1a}$, $R^2$, and $R^{3a}$ are independently selected at each occurrence from the group consisting of hydrogen, halo, halomethyl, $C_1$-$C_3$ alkyl, and cyano;

$R^4$ is $(CH_2)_mOR^{16}$, $C_1$-$C_4$ alkyl, allyl, propargyl, $(CH_2)_mR^{13}$, or $-(CH_2)_mOC(O)R^{16}$;

X is halogen, aryl, heteroaryl, $S(O)_2R^8$, $SR^8$, halomethyl, $-(CH_2)_pOR^8$, cyano, $-(CHR^{16})_pNR^{14}R^{15}$, $-C(=O)R^8$, $C_1$-$C_6$ alkyl, $C_4$-$C_{10}$ cycloalkylalkyl, $C_1$-$C_{10}$alkenyl, $C_2$-$C_{10}$alkynyl, $C_2$-$C_{10}$alkoxy, aryl-($C_2$-$C_{10}$)-alkyl, $C_3$-$C_6$cycloalkyl, aryl-($C_1$-$C_{10}$)-alkoxy, nitro, thio-($C_1$-$C_{10}$)-alkyl, $-C(=NOR^{16})$-$C_1$-$C_4$-alkyl, $-C(=NOR^{16})H$, or $-C(=O)NR^{14}R^{15}$, where substitution by $R^{18}$ can occur on any carbon containing substituents;

X' is independently selected at each occurrence from the group consisting of hydrogen, halogen, aryl, heteroaryl, $S(O)_nR^8$, halomethyl, $-(CHR^{16})_pOR^8$, cyano, $-(CHR^{16})_pNR^{14}R^{15}$, $C(=O)R^8$, $C_1$-$C_6$ alkyl, $C_2$-$C_{10}$alkenyl, $C_2$-$C_{10}$alkynyl, $C_1$-$C_{10}$alkoxy, aryl-($C_1$-$C_{10}$)-alkyl, $C_3$-$C_6$cycloalkyl, aryl-($C_1$-$C_{10}$)-alkoxy, nitro, thio-($C_1$-$C_{10}$)-alkyl, $-C(=NOR^{16})$-$C_1$-$C_4$-alkyl, $-C(= NOR^{16})H$, and $-C(=O)NR^{14}R^{15}$, where substitution by $R^{16}$ can occur on any carbon containing substituents;

$R^5$ is halo, $-C(=NOR^{16})$-$C_1$-$C_4$-alkyl, $C_1$-$C_4$alkyl, $C_1$-$C_3$ haloalkyl, $-(CHR^{16})_pOR^8$, $-(CHR^{16})_pS(O)_nR^8$, $-(CHR^6)_pNR^{14}R^{15}$, $C_3$-$C_6$ cycloalkyl, $C_2$-$C_{10}$alkenyl, $C_2$-$C_{10}$alkynyl, aryl-($C_2$-$C_{10}$)-akyl, aryl-($C_1$-$C_{10}$)-alkoxy, cyano, $C_3$-$C_6$ cycloalkoxy, nitro, amino-($C_2$-$C_{10}$)-alkyl, thio-($C_2$-$C_{10}$)-alkyl, $SO_n(R^8)$, $C(=O)R^8$ $-C(=NOR^{16})H$, or $-C(=O)NR^{14}R^{15}$, where substitution by $R^{18}$ can occur on any carbon containing substituents;

$R^6$ and $R^7$ are independently selected at each occurrence from the group consisting of hydrogen, $C_1$-$C_6$ alkyl, $C_3$-$C_{10}$ cycloalkyl, $C_1$-$C_6$ alkoxy, ($C_4$-$C_{12}$)-cycloalkylalkyl, -$(CH_2)_kR^{13}$, $(CHR^{16})_pOR^8$, -($C_1$-$C_6$alkyl)-aryl, heteroaryl, -$S(O)_z$-aryl or -($C_1$-$C_6$alkyl)-heteroaryl or aryl, wherein the aryl or heteroaryl groups are optionally substituted with 1-3 groups selected from the group consisting of hydrogen, halogen, $C_1$-$C_6$alkyl, $C_1$-$C_6$ alkoxy, amino, NHC(=O)($C_1$-$C_6$ alkyl), NH($C_1$-$C_6$ alkyl), N($C_1$-$C_6$ alkyl)$_2$, nitro, carboxy, $CO_2$($C_1$-$C_6$ alkyl), cyano, and $S(O)_2$-($C_1$-$C_6$-alkyl); or can be taken together to form -$(CH_2)_pA(CH_2)_r$-, optionally substituted with 0-3 $R^{17}$; or, when considered with the commonly attached nitrogen, can be taken together to form a heterocycle, said heterocycle being substituted on carbon with 1-3 groups consisting of hydrogen, $C_1$-$C_6$ alkyl, hydroxy, or $C_1$-$C_6$ alkoxy;

A is $CH_2$, O, $NR^{25}$, C(=O), $S(O)_n$, N(C(=O)$R^{17}$), N($R^{19}$), C(H)($NR^{14}R^{15}$), C(H)($OR^{20}$), C(H)(C(=O)$R^{21}$), or N(S(O)$_nR^{21}$);

$R^8$ is independently selected at each occurrence from the group consisting of hydrogen; $C_1$-$C_6$ alkyl; -($C_4$-$C_{12}$) cycloalkylalkyl; $(CH_2)_tR^{22}$; $C_3$-$C_{10}$ cycloalkyl; -$NR^6R^7$; aryl; heteroaryl; -$NR^{16}(CH_2)_nR^6R^7$; -$(CH_2)_kR^{25}$; and $(CH_2)_t$heteroaryl or $(CH_2)_t$aryl, either of which can optionally be substituted with 1-3 groups selected from the group consisting of hydrogen, halogen, $C_1$-$C_6$ alkyl, $C_1$-$C_6$ alkoxy, amino, NHC(=O)($C_1$-$C_6$ alkyl), NH($C_1$-$C_6$ alkyl), N($C_1$-$C_6$ alkyl)$_2$, nitro, carboxy, $CO_2$($C_1$-$C_6$ alkyl), cyano, and $S(O)_2$($C_1$-$C_6$-alkyl);

$R^9$ is independently selected at each occurrence from $R^{10}$, hydroxy, $C_1$-$C_4$ alkoxy, $C_3$-$C_6$ cycloalkyl, $C_2$-$C_4$ alkenyl, aryl substituted with 0-3 $R^{18}$, and -($C_1$-$C_6$ alkyl)-aryl substituted with 0-3 $R^{18}$;

$R^{10}$, $R^{16}$, $R^{23}$, and $R^{24}$ are independently selected at each occurrence from hydrogen or $C_1$-$C_4$ alkyl;

$R^{11}$ is $C_1$-$C_4$ alkyl substituted with 0-3 groups chosen from the following: keto, amino, sulfhydryl, hydroxyl, guanidinyl, p-hydroxyphenyl, imidazolyl, phenyl, indolyl, and indolinyl, or, when taken together with an adjacent $R^{10}$, are $(CH_2)_t$;

$R^{12}$ is hydrogen or an appropriate amine protecting group for nitrogen or an appropriate carboxylic add protecting group for carboxyl;

$R^{13}$ is independently selected at each occurrence from the group consisting of CN, $OR^{19}$, $SR^{19}$, and $C_3$-$C_6$ cycloalkyl;

$R^{14}$ and $R^{15}$ are independently selected at each occurrence from the group consisting of hydrogen, $C_4$-$C_{10}$, cycloalkyl-alkyl, and $R_{19}$;

$R^{17}$ is independently selected at each occurrence from the group consisting of $R^{10}$, $C_1$-$C_4$ alkoxy, halo, $OR^{23}$, $SR^{23}$, $NR^{23}R^{24}$, and ($C_1$-$C_6$) alkyl ($C_1$-$C_4$) alkoxy;

$R^{18}$ is independently selected at each occurrence from the group consisting of $R^{10}$, hydroxy, halogen, $C_1$-$C_2$ haloalkyl, $C_1$-$C_4$ alkoxy, C(=O)$R^{24}$, and cyano;

$R^{19}$ is independently selected at each occurrence from the group consisting of $C_1$-$C_6$ alkyl, $C_3$-$C_6$ cycloalkyl, $(CH_2)_wR^{22}$, and aryl substituted with 0-3 $R^{18}$;

$R^{20}$ is independently selected at each occurrence from the group consisting of $R^{10}$, C(=O)$R^{31}$, and $C_2$-$C_4$ alkenyl;

$R^{21}$ is independently selected at each occurrence from the group consisting of $R^{10}$, $C_1$-$C_4$ alkoxy, $NR^{23}R^{24}$, and hydroxyl;

$R^{22}$ is independently selected at each occurrence from the group consisting of cyano, $OR^{24}$, $SR^{24}$, $NR^{23}R^{24}$,$C_1$-$C_6$ alkyl, $C_3$-$C_6$ cycloalkyl, -$S(O)_nR^{31}$, and -C(=O)$R^{25}$;

$R^{25}$, which can be optionally substituted with 0-3 R17, is independently selected at each occurrence from the group consisting of phenyl, pyrazolyl, imidazolyl, 2-methyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-pheno-thiazinyl, 4-pyrazinyl, azetidinyl, 1*H*-indazolyl, 2-pyrrolidonyl, 2*H*,6*H*-1,5,2-dithiazinyl, 2*H*-pyrrolyl, 3*H*-indolyl, 4-piperidonyl, 4a*H*-carbazolyl, 4*H*-quinolizinyl, 6*H*-1,2,5-thiadiazinyl, acridinyl, azocinyl, azepinyl, benzofuranyl, benzothiophenyl, carbazolyl, chromanyl, chromenyl, cinnolinyl, decahydroquinolinyl, furazanyl, indolinyl, indolizinyl, indolyl, isobenzofuranyl, isochromanyl, isoindolinyl, isoindolyl, isoquinolinyl benzimidazolyl, isothiazolyl, isoxazolyl, morpholinyl, naphthyridinyl, octahydroisoquinolinyl, oxazolidinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazolidinyl, pyridazinyl, pyridyl, pyrimidinyl, pyrrolidinyl, pyrrolinyl, pyrrolyl, quinazolinyl, quinolinyl, quinoxalinyl, quinuclidinyl, B-carbolinyl, tetrahydrofuranyl, tetrazolyl, thianthrenyl, thiazolyl, thiophenyl, triazinyl, xanthenyl; and 1-tetrahydroquinolinyl or 2-tetrahydroisoquinolinyl either of which can be substituted with 0-3 groups chosen from keto and $C_1$-$C_4$ alkyl;

$R^{25a}$, which can be optionally substituted with 0-3 $R^{17}$, is independently selected at each occurrence from the group consisting of H and $R^{25}$;

$R^{27}$ is independently selected at each occurrence from the group consisting of $C_1$-$C_3$ alkyl, $C_2$-$C_4$ alkenyl, $C_2$-$C_4$ alkynyl, $C_2$-$C_4$ alkoxy, aryl, nitro, cyano, halogen, aryloxy, and heterocycle optionally linked through 0;

$R^{31}$ is independently selected at each occurrence from the group consisting of $C_1$-$C_4$ alkyl, $C_3$-$C_7$ cycloalkyl, $C_4$-$C_{10}$ cycloalkyl-alkyl, and aryl-($C_1$-$C_4$) alkyl;

k, m, and r are independently selected at each occurrence from 1-4;

n is independently, selected at each occurrence from 0-2,

p, q, and z are independently selected at each occurrence from 0-3;
t and w are independently selected at each occurrence from 1-6,

provided that when J is CX' and K and L are both CH, and M is $CR^5$, then

(A) when V and Y are N and Z is CH and $R^1$ and $R^3$ are methyl,

(1) and $R^4$ is methyl, then

(a) $R^5$ can not be methyl when X is OH and X' is H;
(b) $R^5$ can not be $-NHCH_3$, or $-N(CH_3)_2$ when X and X' are $-OCH_3$; and
(c) $R^5$ can not be $-N(CH_3)_2$ when X and X' are $-OCH_2CH_3$;

(2) and $R^4$ is ethyl, then

(a) $R^5$ can not be methylamine when X and X' are $-OCH_3$;
(b) $R^5$ can not be OH when X is Br and X' is OH; and
(c) $R^5$ can not be $-CH_2OH$ or $-CH_2N(CH_3)_2$ when X is $-SCH_3$ and X' is H;

(B) when V and Y are N, Z is CH, $R^4$ is ethyl, $R^5$ is iso-propyl, X is Br, X' is H, and

(1) $R^1$ is $CH_3$, then
(a) $R^3$ can not be OH, piperazin-1-yl, $-CH_2$,-piperidin-1-yl, $-CH_2$-(N-4-methylpiperazi n-1-yl), -C(O)N H-phenyl, $-CO_2H$, $-CH_2O$-(4-pyridyl), $-C(O)NH_2$, 2-indolyl, $-CH_2O$-(4-carboxyphenyl), $-N(CH_2CH_3)$(2-bromo-4-isopropylphenyl);
(2) $R^2$ is $-CH_2CH_2CH_3$ then $R^3$ can not be $-CH_2CH_2CH_3$

(C) when V, Y and Z are N, $R^4$ is ethyl, and

(1) $R^5$ is iso-propyl, X is bromo, and X' is H, then

(a) $R^3$ can not be OH or $-OCH_2CN$ when $R^1$ is $CH_3$ and
(b) $R^3$ can not be $-N(CH_3)_2$ when $R^1$ is $-N(CH_3)_2$;

(2) $R^5$ is $-OCH_3$, X is $-OCH_3$, and X' is H, then $R^3$ and $R^1$ can not both be chloro;

further provided that when J, K, and L are all CH and M is $CR^5$, then
(D) at least one of V, Y, and Z must be N;
(E) when V is $CR^{1a}$, Z and Y can not both be N;
(F) when Y is $CR^{3a}$, Z and V can not both be N;
(G) when Z is $CR^2$, V and Y must both be N;
(H) Z can be N only when both V and Y are N or when V is $CR^{1a}$ and Y is $CR^{3a}$;
(I) when V and Y are N, Z is $CR^2$, and $R^2$ is H or $C_1$-$C_3$ alkyl, and $R^4$ is $C_1$-$C_3$ alkyl, $R^3$ can not be 2-pyridinyl, indolyl, indolinyl, imidazolyl, 3-pyridinyl, 4-pyridinyl, 2-rnethyl-3-pyridinyl, 4-methyl-3-pyridinyl, furanyl, 5-methyl-2-furanyl, 2,5-dimethyl-3-furanyl, 2-thienyl, 3-thienyl, 5-methyl-2-thienyl, 2-phenothiazinyl, or 4-pyrazinyl;
(J) when V and Y are N; Z is $CR^2$; $R^2$ is H or $C_1$-$C_3$ alkyl; $R^4$ is $C_1$-$C_4$ alkyl, $R^5$, X, and/or X' are OH, halo, $CF_3$, $C_1$-$C_4$ alkyl, $C_1$-$C_4$ alkoxy, $C_1$-$C_4$ alkylthio, cyano, amino, carbamoyl, or $C_1$-$C_4$ alkanoyl; and $R^1$ is $C_1$-$C_4$ alkyl, then $R^4$ can not be -NH(substituted phenyl) or -N($C_1$-$C_4$ alkyl) (substituted phenyl);

and wherein, when Y is $CR^{29}$:

J, K, L, M, Z, A, k, m, n, p, q, r, t, w, $R^3$, $R^{10}$, $R^{11}$, $R^{12}$, $R^{13}$, $R^{16}$, $R^{18}$, $R^{19}$, $R^{21}$, $R^{23}$, $R^{24}$, $R^{25}$, and $R^{27}$ are as defined above and $R^{25a}$, in addition to being as defined above, can also be $C_1$-$C_4$ alkyl, but
V is N;
$R^1$ is $C_1$-$C_2$ alkyl, $C_2$-$C_4$ alkenyl, $C_2$-$C_4$ alkynyl, $C_2$-$C_4$ alkoxy, halogen, amino, methylamino, dimethylamino, aminomethyl, or N-methylaminomethyl;
$R^2$ is independently selected at each occurrence from the group consisting of hydrogen, halo, $C_1$-$C_3$, alkyl, nitro, amino, and $-CO_2R^{10}$;

$R_4$ is taken together with $R^{29}$ to form a 5-membered ring and is -C($R^{26}$) = or -N= when $R^{29}$ is -C($R^{30}$)= or -N=, or -CH($R^{26}$)- when $R^{29}$ is -CH($R^{30}$)-;

X is Cl, Br, I, S(O)n$R^8$, O$R^8$, halomethyl, -$(CHR^{16})_p$O$R^8$, cyano, -$(CHR^{16})_p$N$R^{14}R^{15}$, C(=O)$R^8$, $C_1$-$C_6$ alkyl, $C_2$-$C_{10}$ alkenyl, $C_2$-$C_{10}$ alkynyl, $C_1$-$C_{10}$, alkoxy, aryl-($C_1$-$C_{10}$)-alkyl, $C_3$-$C_6$ cycloalkyl, aryl-($C_1$-$C_{10}$)-alkoxy, nitro, thio-($C_1$-$C_{10}$)-alkyl, -C(=NO$R^{16}$)-$C_1$-$C_4$-alkyl, -C(=NO$R^{16}$)H, or C(=O)N$R^{14}R^{15}$ where substitution by $R^{18}$ can occur on any carbon containing substituents;

X' is hydrogen, Cl, Br, I, $S(O)_n$$R^8$, -$(CHR^{16})_p$O$R^8$, halomethyl, cyano, -$(CHR^{15})_p$N$R^{14}R^{15}$, C(=O)$R^8$, $C_1$-$C_6$ alkyl, $C_2$-$C_{10}$alkenyl, $C_2$-$C_{10}$, alkynyl, $C_1$-$C_{10}$ alkoxy, aryl-($C_1$-$C_{10}$)-alkyl, $C_3$-$C_6$ cycloalkyl, aryl-($C_2$-$C_{10}$)-alkoxy, nitro, thio-($C_2$-$C_{10}$)-alkyl, -C(=NO$R^{16}$)-$C_1$-$C_4$-alkyl, -C(=NO$R^{16}$)H, or C(=O)N$R^8R^{15}$ where substitution by $R^{18}$ can occur on any carbon containing substituents;

$R^5$ is halo, -C(=NO$R^{16}$)-$C_1$-$C_4$-alkyl, $C_1$-$C_6$ alkyl, $C_1$-$C_3$ haloalkyl, $C_1$-$C_6$ alkoxy, $(CHR^{16})_p$O$R^5$, $(CHR^{16})_p$$S(O)_n$$R^8$, $(CHR^{16})_p$N$R^{14}R^{15}$, $C_3$-$C_6$ cycloalkyl, $C_2$-$C_{10}$ alkenyl, $C_2$-$C_{10}$ alkynyl, aryl-($C_2$-$C_{10}$)-alkyl, aryl-($C_1$-$C_{10}$)-alkoxy, cyano, $C_3$-$C_6$ cycloalkoxy, nitro, amino-($C_1$-$C_{10}$)-alkyl, thio-($C_1$-$C_{10}$)-alkyl, $SO_n$($R^8$), C(=O)$R^8$, -C(=NO$R^{16}$)H, or C (=O)N$R^8R^{15}$ where substitution by $R^{18}$ can occur on any carbon containing substituents;

$R^6$ and $R^7$ are independently selected at each occurrence from the group consisting of hydrogen, $C_1$-$C_6$ alkyl, $C_3$-$C_{10}$ cycloalkyl, -$(CH_2)_k$$R^{13}$, ($C_4$-$C_{12}$)-cycloalkylalkyl, $C_1$-$C_6$ alkoxy, -($C_1$-$C_6$ alkyl)-aryl, heteroaryl, aryl, -$S(O)_z$-aryl or -($C_1$-$C_6$ alkyl)-heteroaryl or aryl wherein the aryl or heteroaryl groups are optionally substituted with 1-3 groups selected from hydrogen, halogen, $C_1$-$C_6$ alkyl, $C_1$-$C_6$ alkoxy, amino, NHC(=O)($C_1$-$C_6$ alkyl), NH($C_1$-$C_6$ alkyl), N $(C_1$-$C_6$ alkyl$)_2$, nitro, carboxy, $CO_2$($C_1$-$C_6$ alkyl), and cyano; or can be taken together to form -$(CH_2)$qA$(CH_2)_r$-, optionally substituted with 0-3 $R^{17}$; or, when considered with the commonly attached nitrogen, can be taken together to form a heterocycle, said heterocycle being substituted on carbon with 1-3 groups consisting of hydrogen, $C_1$-$C_6$ alkyl, hydroxy, or $C_1$-$C_6$ alkoxy;

$R^8$ is independently selected at each occurrence from the group consisting of hydrogen, $C_1$-$C_6$ alkyl, -($C_4$-$C_{12}$) cycloalkylalkyl, $(CH_2)_t$$R^{22}$, $C_3$-$C_{10}$ cycloalkyl, -($C_1$-$C_6$ alkyl)-aryl, heteroaryl, -N$R^{16}$, -N$(CH_2)_n$N$R^6R^7$; -$(CH_2)_k$$R^{25}$, -($C_1$-$C_6$ alkyl)-heteroaryl or aryl optionally substituted with 1-3 groups selected from hydrogen, halogen, $C_1$-$C_6$ alkyl, $C_1$-$C_6$ alkoxy, amino, NHC(=O)($C_1$-$C_6$ alkyl), NH($C_1$-$C_6$ alkyl), N$(C_1$-$C_6$alkyl$)_2$, nitro, carboxy, $CO_2$($C_1$-$C_6$ alkyl), and cyano;

$R^9$ is independently selected at each occurrence from $R^{10}$, hydroxy, $C_1$-$C_4$ alkoxy, $C_3$-$C_6$ cycloalkyl, $C_2$-$C_4$ alkenyl, and aryl substituted with 0-3 $R^{18}$;

$R^{14}$ and $R^{15}$ are independently selected at each occurrence from the group consisting of hydrogen, $C_1$-$C_6$ alkyl, $C_3$-$C_6$ cycloalkyl, $(CH_2)_t$$R^{22}$, and aryl substituted with 0-3 $R^{18}$;

$R^{17}$ is independently selected at each occurrence from the group consisting of $R^{10}$, $C_1$-$C_4$ alkoxy, halo, O$R^{23}$, S$R^{23}$, and N$R^{23}R^{24}$;

$R^{20}$ is independently selected at each occurrence from the group consisting of $R^{10}$ and C(=O)$R^{31}$;

$R^{22}$ is independently selected at each occurrence from the group consisting of cyano, O$R^{24}$, S$R^{24}$, N$R^{23}R^{24}$, $C_3$-$C_6$ cycloalkyl, -$S(O)_n$$R^{31}$, and -C(=O)$R^{25}$;

$R^{26}$ is hydrogen or halogen;

$R^{28}$ is $C_1$-$C_2$, alkyl, $C_2$-$C_4$ alkenyl, $C_2$-$C_4$ alkynyl, hydrogen, $C_1$-$C_2$ alkoxy, halogen, or $C_2$-$C_4$ alkylamino;

$R^{29}$ is taken together with $R^4$ to form a five membered ring and is: -CH($R^{30}$)- when $R^4$ is -CH($R^{28}$)-, -C($R^{30}$) = or -N = when $R^4$ is -C($R^{28}$) = or -N=;

$R^{30}$ is hydrogen, cyano, $C_1$-$C_2$ alkyl, $C_1$-$C_2$ alkoxy, halogen, $C_1$-$C_2$ alkenyl, nitro, amido, carboxy, or amino;

$R^{31}$ is $C_1$-$C_4$ alkyl, $C_3$-$C_7$ cycloalkyl, or aryl-($C_1$-$C_4$) alkyl; provided that when J, K, and L are all CH, M is C$R^5$, Z is CH, $R^3$ is $CH_3$, $R^{28}$ is H, $R^5$ is isopropyl, X is Br, X' is H, and $R^1$ is $CH_3$, then $R^{30}$ can not be H, -$CO_2$H, or -$CH_2NH_2$; and further provided that when J, K and L are all CH; M is C$R^5$; Z is N; and

(A) $R^{29}$ is -C($R^{30}$)=; then one of $R^{28}$ or $R^{30}$ is hydrogen;
(B) $R^{29}$ is N; then $R^3$ is not halo, $NH_2$, $NO_2$, $CF_3$, $CO_2$H, $CO_2$-alkyl, alkyl, acyl, alkoxy, OH, or -$(CH_2)_m$Oalkyl;
(C) $R^{29}$ is N; then $R^{28}$ is not methyl if X or X' are bromo or methyl and $R^5$ is nitro; or
(D) $R^{29}$ is N; and $R^1$ is $CH_3$; and $R^3$ is amino; then $R^5$ is not halogen or methyl.

Preferred compounds of this group include those wherein:

i) V is N, $R^1$ is methyl; and $R^3$ is aryl, N$R^6R^7$, or O$R^8$;
ii) V is N, $R^1$ is methyl; $R^3$ is aryl, N$R^6R^7$, or O$R^8$; and $R^4$ is methyl or ethyl;
iii) V is N, $R^1$ is methyl; $R^3$ is aryl, N$R^6R^7$, or O$R^8$; $R^4$ is methyl or ethyl; and X is O($C_1$-$C_4$ alkyl), Br, or $C_1$-$C_4$ alkyl;
iv) V is N, $R^1$ is methyl; $R^3$ is aryl, N$R^6R^7$, or O$R^8$; $R^4$ is methyl, ethyl; X is OMe, Br, or ($C_1$-$C_4$ alkyl), M is $C_1$-$C_4$ alkyl, Br, Cl, or O($C_1$-$C_4$ alkyl); and
v) V is N, $R^1$ is methyl; $R^3$ is aryl, N$R^6R^7$, O$R^8$; or $R^4$ is methyl, ethyl; X is OMe, Br, or $C_1$-$C_4$ alkyl, M is $C_1$-$C_4$

alkyl, Br, Cl, or O($C_1$-$C_4$ alkyl); and L is CH, or N.

Another group of useful CRF antagonists for use in the compositions, methods, and kits of the present invention are those wherein the CRF antagonist is a compound of the following formula, disclosed in EP 0773023:

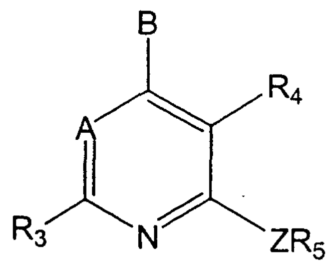

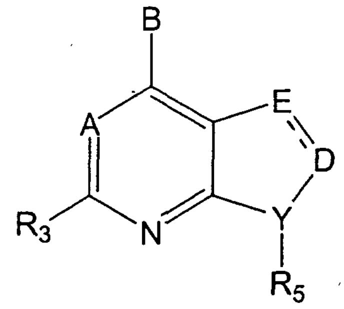

or a pharmaceutically acceptable salt thereof, wherein

the dashed line represents an optional double bond;
A is $-CR_7$ or N;
B is $-NR_1R_2$, $-CR_1R_2R_{11}$, $-C(=CR_1R_{12})R_2$, $-NHCR_{11}R_1R_2$, $-OCR_{11}R_1R_2$, $-SCR_{11}R_1R_2$, $-CR_{11}R_2OR_1$, $-CR_{11}R_2SR_1$, $-C(S)R_2$, $-NHNR_1R_2$, $-CR_2R_{11}NHR_1$ or $-C(O)R_2$;
D is N or $-CR_{10}$ when a double bond connects E and D and E is $-CR_4$; $-CR_{10}$ when a double bond connects E and D and E is N; or $-CR_8R_9$, $-CHR_{10}$, -C=O,-C=S, -C=NH, or $-C=NCH_3$ when a single bond connects E and D;
E is $-CR_4$ or N when a double bond connects E and D, and E is $-CR_4R_6$ or $-NR_6$ when a single bond connects E and D;
Y is N or -CH;
Z is NH, O, S, -N($C_1$-$C_2$ alkyl), or $-CR_{12}R_{13}$, wherein $R_{12}$ and $R_{13}$ are each, independently, hydrogen, trifluoromethyl, or methyl, or one of $R_{12}$ and $R_{13}$ is cyano and the other is hydrogen or methyl;
$R_1$ is hydrogen or $C_1$-$C_6$ alkyl which is optionally substituted with up to two substituents independently selected from hydroxy, cyano, nitro, fluoro, chloro, bromo, iodo, $CF_3$, $C_1$-$C_4$ alkoxy, -O-CO-($C_1$-$C_4$ alkyl), -O-CO-NH($C_1$-$C_4$ alkyl), -O-CO-N($C_1$-$C_4$ alkyl)($C_1$-$C_2$ alkyl), -NH($C_1$-$C_4$ alkyl), -N($C_1$-$C_2$ alkyl)($C_1$-$C_4$ alkyl), -S($C_1$-$C_4$ alkyl), -N($C_1$-$C_4$alkyl)CO($C_1$-$C_4$ alkyl), -NHCO($C_1$-$C_4$ alkyl), $-CO_2$($C_1$-$C_4$ alkyl), -CONH($C_1$-$C_4$ alkyl), -CON($C_1$-$C_4$ alkyl)($C_1$-$C_2$ alkyl), ($C_1$-$C_4$ alkyl)sulfinyl, ($C_1$-$C_4$ alkyl)sulfonyl, and ($C_1$-$C_4$ alkyl)sulfanyl, and wherein said $C_1$-$C_6$ alkyl, $C_1$-$C_4$ alkoxy, and $C_1$-$C_4$ alkyl moieties in the foregoing $R_1$ groups optionally contain one double or triple bond;
$R_2$ is $C_1$-$C_6$ alkyl, heteroaryl, aryl, heteroaryl ($C_1$-$C_4$ alkyl), or aryl ($C_1$-$C_4$ alkyl), wherein said aryl and the aryl moiety of said (aryl)$C_1$-$C_4$ alkyl are selected from the group consistiig of phenyl and naphthyl, and said heteroaryl and the heteroaryl moiety of said (heteroaryl)$C_1$-$C_4$ alkyl is selected from the group consisting of thienyl, benzothienyl, pyridyl, thiazolyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, and benzoxazolyl; or $R^2$ is $C_3$-$C_8$ cycloalkyl or ($C_3$-$C_8$ cycloalkyl)$C_1$-$C_6$ alkyl, wherein one or two of the ring carbons of said cycloalkyl having at least 4 ring members and the cycloalkyl moiety of said ($C_3$-$C_8$ cycloalkyl)$C_1$-$C_6$ alkyl having at least 4 ring members is optionally replaced by an oxygen or sulfur atom or by $-NR_{14}$ wherein $R_{14}$ is hydrogen or $C_1$-$C_4$ alkyl; and wherein each of the foregoing $R_2$ groups is optionally substituted by up to three substituents independently selected from chloro, fluoro, and $C_1$-$C_4$ alkyl, or by one substituent selected from bromo, iodo, cyano, nitro, $C_1$-$C_6$ alkoxy, -O-CO-($C_1$-$C_4$ alkyl), -O-CO-N($C_1$-$C_4$ alkyl)($C_1$-$C_2$ alkyl),$-CO_2$($C_1$-$C_4$ alkyl), ($C_1$-$C_4$ alkyl)sulfanyl, ($C_1$-$C_4$ alkyl)sulfinyl, and ($C_1$-$C_4$ alkyl)sulfonyl, and wherein said $C_1$-$C_4$ alkyl and $C_1$-$C_6$ alkyl moieties of the foregoing $R_2$ groups optionally contain one carbon-carbon double or triple bond;
or $R^1$ and $R^2$ of said $-NR_1R_2$ and said $-CR_1R_2R_{11}$ are taken together to form a saturated or partially saturated 5- to 8-membered ring, wherein said ring optionally contains one or two carbon-carbon double bonds, and wherein one or two of the ring carbons is optionally replaced by a heteroatom selected from O, S, and N;
$R_3$ is hydrogen, $C_1$-$C_6$ alkyl, fluoro, chloro, bromo, iodo, hydroxy, amino, SH, -NH($C_1$-$C_4$ alkyl), -N($C_1$-$C_4$ alkyl)($C_1$-$C_2$ alkyl), $-CH_2OH$, $-CH_2OCH_3$, -O($C_1$-$C_4$ alkyl), ($C_1$-$C_4$ alkyl)sulfanyl, ($C_1$-$C_4$ alkyl)sulfonyl, or ($C_1$-$C_4$ alkyl)sulfinyl, wherein said $C_1$-$C_6$ alkyl and $C_1$-$C_4$ alkyl moieties of the foregoing $R_3$ groups optionally contain one double or triple bond and are optionally substituted by from one to three substituents independently selected from hydroxy, amino, $C_1$-$C_3$ alkoxy, -NH($C_1$-$C_2$ alkyl), $-N(C_1$-$C_2$ alkyl$)_2$, $-NHCOCH_3$, fluoro, chloro, and $C_1$-$C_3$ thioalkyl;

$R_4$ is hydrogen, $C_1$-$C_6$ alkyl, fluoro, chloro, bromo, iodo, $C_1$-$C_6$ alkoxy, formyl, trifluoromethoxy, $-CH_2OCH_3$, $-CH_2OCH_2CH_3$, $-CH_2CH_2OCH_3$, $-CH_2CF_3$, $CF_3$, amino, nitro, -NH($C_1$-$C_4$ alkyl), $-N(CH_3)_2$, $-NHCOCH_3$, $-NHCONHCH_3$, ($C_1$-$C_4$ alkyl)sulfanyl, ($C_1$-$C_4$ alkyl)sulfinyl, ($C_1$-$C_4$ alkyl)sulfonyl, cyano, hydroxy, -CO($C_1$-$C_4$ alkyl), -CHO, or $-CO_2$($C_1$-$C_4$ alkyl), wherein said $C_1$-$C_6$ alkyl, $C_1$-$C_6$ alkoxy, and $C_1$-$C_4$ alkyl moieties of the foregoing $R_4$ groups optionally contain one double or triple bond and are optionally substituted with one substituent selected from hydroxy, amino, $-NHCOCH_3$, -NH($C_1$-$C_2$ alkyl), -N($C_1$-$C_2$ alkyl)$_2$, $-CO_2$($C_1$-$C_4$ alkyl), -CO($C_1$-$C_4$ alkyl), $C_1$-$C_3$ alkoxy, ($C_1$-$C_3$ alkyl)sulfanyl, fluoro, chloro, cyano, and nitro;

$R_5$ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, pyridinyl, tetrazolyl, or a 3- to 8-membered cycloalkyl ring or a 9- to 12-membered bicycloalkyl ring system, wherein said cycloalkyl ring and said bicycloalkyl ring system optionally contain one or two of O, S, or -N-G wherein G is hydrogen, $C_1$-$C_4$ alkyl, $C_1$-$C_4$ alkanoyl, phenyl, or benzyl, wherein each of the above $R_5$ groups is optionally substituted by up to three substituents independently selected from fluoro, chloro, $C_1$-$C_6$ alkyl, $C_1$-$C_6$ alkoxy, and trifluoromethyl, or one substituent selected from bromo, iodo, cyano, nitro, amino, -NH($C_1$-$C_4$ alkyl), -N($C_1$-$C_4$ alkyl)($C_1$-$C_2$ alkyl),-$CO_2$($C_1$-$C_4$ alkyl), -CO($C_1$-$C_4$ alkyl), $-SO_2NH$($C_1$-$C_4$ alkyl), $-SO_2N$($C_1$-$C_4$ alkyl)($C_1$-$C_2$ alkyl), $-SO_2NH_2$, $-NHSO_2$ ($C_1$-$C_4$ alkyl), -S($C_1$-$C_4$ alkyl), and $-SO_2$($C_1$-$C_4$ alkyl), wherein said $C_1$-$C_4$ alkyl and $C_1$-$C_6$ alkyl moieties of the foregoing $R_5$ groups optionally contain one double or triple bond and are optionally substituted by one or two substituents independently selected from fluoro, chloro, hydroxy, amino, methylamino, dimethylamino, and acetyl;

$R_6$ is hydrogen or $C_1$-$C_6$ alkyl, wherein said $C_1$-$C_6$ alkyl is optionally substituted by a single hydroxy, methoxy, ethoxy, or fluoro group;

$R_7$ is hydrogen, $C_1$-$C_4$ alkyl, fluoro, chloro, bromo, iodo, cyano, hydroxy, $C_1$-$C_4$ alkoxy, -CO($C_1$-$C_4$ alkyl), $-CO_2$($C_1$-$C_4$ alkyl), $-OCF_3$, $CF_3$, $-CH_2OH$, $-CH_2OCH_3$, or $-CH_2OCH_2CH_3$;

$R_8$ and $R_9$ are each, independently, hydrogen, hydroxy, methyl, ethyl, methoxy, or ethoxy;

or $R_8$ and $R_9$ together form an oxo (=O) group;

$R_{10}$ is hydrogen, $C_1$-$C_6$ alkyl, fluoro, chloro, bromo, iodo, $C_1$-$C_6$ alkoxy, formyl, amino, -NH($C_1$-$C_4$ alkyl), -N($C_1$-$C_4$ alkyl)($C_1$-$C_2$ alkyl), cyano, carboxy, amido, or-$SO_n$($C_1$-$C_4$ alkyl) wherein n is 0, 1, or 2, wherein said $C_1$-$C_6$ alkyl and $C_1$-$C_4$ alkyl moieties of the foregoing $R_{10}$ groups are optionally substituted by one of hydroxy, trifluoromethyl, amino, carboxy, amido, -NHCO($C_1$-$C_4$ alkyl), -NH($C_1$-$C_4$ alkyl), -N($C_1$-$C_4$ alkyl)($C_1$-$C_2$ alkyl), $-CO_2$($C_1$-$C_4$ alkyl), $C_1$-$C_3$ alkoxy, $C_1$-$C_3$ thioalkyl, fluoro, bromo, chloro, iodo, cyano, or nitro; and

$R_{11}$ is hydrogen, hydroxy, fluoro, or methoxy.

Another group of preferred CRF antagonists for use in the compositions, methods, and kits of the present invention are those wherein the CRF antagonist is a compound selected from the group consisting of:

4-(1-ethyl-propoxy)-3,6-dimethyl-2-(2,4,6-trimethylphenoxy)-pyridine;
4-(1-ethylpropoxy)-2,5-dimethyl-6-(2,4,6-trimethylphenoxy)-pyrimidine;
[4-(1-ethyl-propoxy)-3,6-dimethyl-pyridin-2-yl]-(2,4,6-trimethylphenyl)-amine;
3-{(4-methyl-benzyl)-[3,6-dimethyl-1-(2,4,6-trimethylphenyl)-1H-pyrazolo[3,4-d]pyrimidin-4-yl]-amino}-propan-1-ol;
propyl-ethyl-[3,6-dimethyl-1-(2,4,6-trimethylphenyl)-1H-pyrazolo[3,4-d]pyrimidin-4-yl]-amine;
ethyl-n-propyl-[2,5-dimethyl-7-(2,4,6-trimethylphenyl)-7H-pyrrolo[2,3-d]pyrimidin-4-yl]amine;
2-{N-n-butyl-N-[2,5-dimethyl-7-(2,4,6-trimethylphenyl)-7H-pyrrolo[2,3-d]pyrimidin-4-yl]amino}-ethanol;
[3,6-dimethyl-1-(2,4,6-trimethylphenyl)-1H-pyrazolo[3,4, b]pyridin-4-yl]-(1-methoxymethylpropyl)-amine;
4-(1-ethylpropoxy)-2,5-dimethyl-7-(2,4,6-trimethylphenyl)-7H-pyrrolo[2,3-b]pyridine;
2,5,6-trimethyl-7-(1-propylbutyl)-4-(2,4,6-trimethylphenoxy)-7H-pyrrolo[2,3-d]pyrimidine;
1-(1-ethylpropyl)-6-methyl-4-(2,4,6-trimethylphenoxy)-1,3-dihydro-imidazo[4, 5-c]pyridin-2-one;
1-(1-ethyl-propyl)-4,7-dimethyl-5-(2,4,6-trimethyl-phenoxy)-1,4-dihydro-2H-pyrido[3,4-b]pyrazin-3-one;
1-(1-ethyl-propyl)-4,7-dimethyl-5-(2,4,6-trimethyl-phenoxy)-1,2,3,4-tetrahydro-pyrido[3,4-b]pyrazine;
1-(1-ethyl-propyl)-7-methyl-2-oxo-5-(2,4,6-trimethyl-phenoxy)-1,2,3,4-tetra-hydro-[1,6]naphthyridine-3-carboxylic acid isopropyl ester;
1-(1-ethyl-propyl )-7-methyl-5-(2,4,6-trimethyl-phenoxy)-1,4-dihydro-2H-3-oxa-1,6-diaza-naphthalene;
(1-ethyl-propyl)-[5-methyl-3-(2,4,6-trimethyl-phenyl)-pyrazolo[1,5-a]pyrimidin-7-yl]-amine;
7-(1-ethyl-propoxy)-2,5-dimethyl-3-(2,4,6-trimethyl-phenyl)-pyrazolo[1,5-a]pyrimidine;
4-(1-ethyl-propoxy)-2,5-dimethyl-7-(2,4,6-trimethyl-phenyl)-5H-pyrrolo[3,2-d]pyrimidine;
4-(butyl-ethyl-amino)-2,6-dimethyl-8-(2,4,6-trimethyl-phenyl)-5,8-dihydro-6H-pyrido[2,3-d]pyrimidin-7-one;
8-(1-ethyl-propoxy)-6-methyl-4-(2,4,6-trimethyl-phenyl)-1,2,3,4-tetrahydro-pyrido [2,3-b]pyrazine;
4-(1-ethyl-propoxy)-2-methyl-8-(2,4,6-trimethyl-phenyl)-quinoline;

(1-ethyl-propyl)-[2-methyl-8-(2,4,6-tiime.thyl-phenyl)-quinolin-4-yl]-amine;
(propyl-ethyl)-[2-methyl-8-(2,4,6-trimethyl-phenyl)-5,6,7,8-tetrahydro-pyrido-[2,3-d] pyrimidin-4-yl]-amine;
(1-ethyl-propoxy)-2-methyl-8-(2,4,6-trimethyl-phenyl)-5,6,7,8-tetrahydro-pyrido[2,3-d] pyrimidine;
8-(1-hydroxymethyl-propylamino)-6-methyl-4-(2,4,6-trimethyl-phenyl)-3,4-dihydro-1H-pyrido[2,3-b]pyrazin-2-one;
4-(1-hydroxymethyl-propylamino)-2-methyl-8-(2,4,6-trimethyl-phenyl)-quinoline;
5-(1-hydroxymethyl-propylamino)-7-methyl-1-(2,4,6-trimethyl-phenyl)-1,4-dihydro-2H-3-oxa-1,8-diaza-naphthalene;
[3,6-dimethyl-2-(2,4,6-trimethyl-phenoxy)-pyridin-4-yl]-(1-ethyl-propyl)-amine;
cyclopropylmethyl-[3-(2,4-dimethyl-phenyl)-2,5-dimethyl-pyrazolo[1,5-a]pyrimidin-7-yl]-propyl-amine;
[2,5-dimethyl-3-(2,4-dimethyl-phenyl)-pyrazolo[1,5-a]pyrimidin-7-yl]-( 1-ethylpropyl)-amine;
3-[6-(dimethylamino)-3-pyridinyl-2,5-dimethyl-N,N-dipropylpyrazolo[2,3-a] pyrimidin-7-amine;
3-[6-(dimethylamino)-4-methyl-3-pyridinyl]-2,5-dimethyl-N,N-dipropyl-pyrazolo[2,3-a]pyrimidine-7-amine;
3-(2,4-dimethoxyphenyl)-2,5-dimethyl-7-(N-propyl-N-methyloxyethylamino)-pyrazolo(2,3-a)pyrimidine;
7-(N-diethylamino)-2,5-dimethyl-3-(2-methyl-4-methoxyphenyl-[1,5-a]-pyrazolopyrimidine; and
7-(N-(3-cyanopropyl)-N-propylamino-2,5,dimethyl-3-(2,4-dimethylphenyl)-[1,5-a]-pyrazolopyrimidine.

A group of preferred growth hormones or growth hormone secretagogues for use in the compositions, methods, and kits of the present invention are those wherein the growth hormone or growth hormone secretagogue is a growth hormone.

A group of preferred growth hormone secretagogues for use in the compositions, methods, and kits of the present invention are those wherein the growth hormone secretagogue is a compound of formula IV:

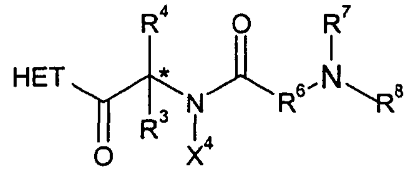

IV

or a stereoisomeric mixture thereof, a diastereomerically enriched, diastereomerically pure, enantiomerically enriched, or enantiomerically pure isomer thereof, or a prodrug of such compound, mixture, or isomer thereof, or a pharmaceutically acceptable salt of the compound, mixture, isomer, or prodrug, wherein:

HET is a heterocyclic moiety selected from the group consisting of

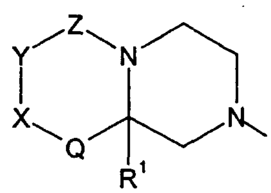

,

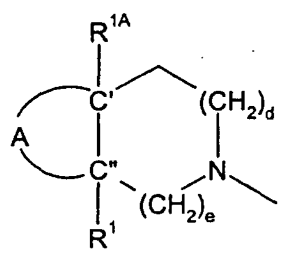

,

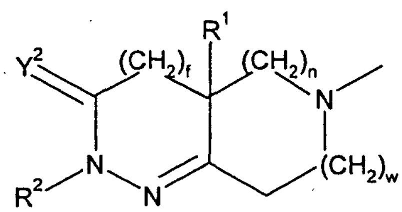

,

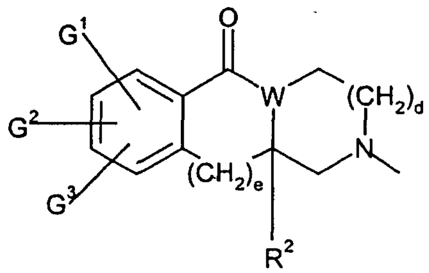

and

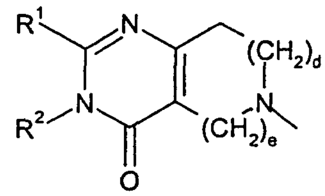

d is 0, 1, or 2;
e is 1 or 2;
f is 0 or 1;
n and w are 0, 1, or 2, provided that n and w cannot both be 0 at the same time;
$Y^2$ is oxygen or sulfur;
A is a divalent radical, wherein the left hand side of the radical as shown below is connected to C" and the right hand side of the radical as shown below is connected to C', selected from the group consisting of $-NR^2-CO-NR^2-$, $-NR^2-SO_2-NR^2-$, $-O-CO-NR^2-$, $-NR^2-CO_2-$, $-CO-NR^2-CO-$, $-CO-NR^2-C(R^9R^{10})-$, $-C(R^9R^{10})-NR^2-CO-$, $-C(R^9R^{10})-C(R^9R^{10})-C(R^9R^{10})-$, $-SO_2-C(R^9R^{10})-C(R^9R^{10})-$, $-C(R^9R^{10})-O-CO-$, $-C(R^9R^{10})-O-C(R^9R^{10})-$, $-NR^2-CO-C(R^9R^{10})-$, $-O-CO-C(R^9R^{10})-$, $-C(R^9R^{10})-CO-NR^2-$, $-CO-NR^2-CO-$, $-C(R^9R^{10})-CO_2-$, $-CO-NR^2-C(R^9R^{10})-C(R^9R^{10})-$, $-CO_2-C(R^9R^{10})-$, $-C(R^9R^{10})-C(R^9R^{10})-C(R^9R^{10})-C(R^9R^{10})-$, $-SO_2-NR^2-C(R^9R^{10})-C(R^9R^{10})-$, $-C(R^9R^{10})-C(R^9R^{10})-NR^2-CO-$, $-C(R^9R^{10})-C(R^9R^{10})-O-CO-$, $-NR^2-CO-C(R^9R^{10})-C(R^9R^{10})-$, $-NR^2-SO_2-C(R^9R^{10})-C(R^9R^{10})-$, $-O-CO-C(R^9R^{10})-C(R^9R^{10})-$, $-C(R^9R^{10})-C(R^9R^{10})-CO-NR^2-$, $-C(R^9R^{10})-C(R^9R^{10})-CO-$, $-C(R^9R^{10})-NR^2-CO_2-$, $-C(R^9R^{10})-O-CO-NR^2$, $-C(R^9R^{10})-NR^2-CO-NR^2-$, $-NR^2-CO_2-C(R^9R^{10})-$, $-NR^2-CO-NR^2-C(R^9R^{10})-$, $-NR^2-SO_2-NR^2-C(R^9R^{10})-$, $-O-CO-NR^2-C(R^9R^{10})-$, $-CO-N=C(R^{11})-NR^2-$, $-CO-NR^2-C(R^{11})=N-$, $-C(R^9R^{10})-NR^{12}-C(R^9R^{10})-$, $-NR^{12}-C(R^9R^{10})-$, $-NR^{12}-C(R^9R^{10})-C(R^9R^{10})-$, $-CO_2-C(R^9R^{10})-C(R^9R^{10})-$, $-NR^2-C(R^{11})=N-CO-$, $-C(R^9R^{10})-C(R^9R^{10})-N(R^{12})-$, $-C(R^9R^{10})-NR^{12}-$, $-N=C(R^{11})-NR^2-CO-$, $-C(R^9R^{10})-C(R^9R^{10})-NR^2-SO_2-$, $-C(R^9R^{10})-C(R^9R^{10})-SO_2-NR^2-$, $-C(R^9R^{10})-C(R^9R^{10})-CO_2-$, $-C(R^9R^{10})-SO_2-C(R^9R^{10})-$, $-C(R^9R^{10})-C(R^9R^{10})-SO_2-$, $-O-C(R^9R^{10})-C(R^9R^{10})-$, $-C(R^9R^{10})-C(R^9R^{10})-O-$, $-C(R^9R^{10})-CO-C(R^9R^{10})-$, $-CO-C(R^9R^{10})-C(R^9R^{10})-$, and $-C(R^9R^{10})-NR^2-SO_2-NR^2-$;
Q is a covalent bond or $CH_2$;
W is CH or N;
X is $CR^9R^{10}$, $C=CH_2$, or C=O;
Y is $CR^9R^{10}$, O, or $NR^2$;
Z is C=O, C=S, or $SO_2$;
$G^1$ is hydrogen, halo, hydroxy, nitro, amino, cyano, phenyl, carboxyl, -CONH2, $-C_1-C_4$ alkyl optionally independently substituted with one or more phenyl, one or more halogen, or one or more hydroxy groups, $-C_1-C_4$ alkoxy optionally independently substituted with one or more phenyl, one or more halogen, or one or more hydroxy groups, $-C_1-C_4$ alkylthio, phenoxy, $-CO_2-(C_1-C_4$ alkyl), N,N-di-($C_1-C_4$ alkylamino), $-C_2-C_6$ alkenyl optionally independently substituted with one or more phenyl, one or more halogen, or one or more hydroxy groups, $-C_2-C_6$ alkynyl optionally independently substituted with one or more phenyl, one or more halogen, or one or more hydroxy groups, $-C_3-C_6$ cycloalkyl optionally independently substituted with one or more $C_1-C_4$ alkyl groups, one or more halogen, or one or more hydroxy groups, $-C_1-C_4$ alkylamino carbonyl, or di-$C_1-C_4$ alkylamino) carbonyl;
$G^2$ and $G^3$ are each independently selected from the group consisting of hydrogen, halo, hydroxy, $-C_1-C_4$ alkyl optionally independently substituted with one to three halo groups, and $-C_1-C_4$ alkoxy optionally independently substituted with one to three halo groups;
$R^1$ is hydrogen, -CN, $-(CH_2)_qNX^6COX^6$, $-(CH_2)_qNX^6CO(CH_2)_t-A^1$, $-(CH2)_qNX^6SO_2(CH_2)_t-A^1$, $(CH_2)_qNX^6SO_2X^6$, $-(CH_2)_qNX^6CONX^6(CH_2)_t-A^1$, $-(CH_2)_qNX^6CONX^6X^6$, $-(CH_2)_qCONX^6X^6$, $(CH_2)_qCONX^6(CH_2)_t-A^1$, $-(CH_2)_qCO_2X^6$, $-(CH_2)_qCO_2(CH_2)_t-A^1$, $-(CH_2)_qOX^6$, $-(CH_2)_qOCOX^6$, $-(CH_2)_qOCO(CH_2)_t-A^1$, $-(CH_2)_qOCONX^6(CH_2)_t-A^1$, $-(CH_2)_qOCONX^6X^6$, $-(CH_2)_qCOX^6$, $-(CH_2)_qCO(CH_2)_t-A^1$, $-(CH_2)_qNX^6CO_2X^6$, $-(CH_2)_qNX^6SO_2NX^6X^6$, $-(CH_2)_qSO_mX^6$, $-(CH_2)_qSO_m(CH_2)_t-A^1$, $-C_1-C_{10}$ alkyl, $-(CH_2)_t-A^1$, $-(CH_2)_q-(C_3-C_7$ cycloalkyl), $-(CH_2)_q-Y^1-(C_1-C_6$ alkyl), $-(CH_2)_q-Y^1-(CH_2)_t-A^1$, or $-(CH_2)_q-Y^1-(CH_2)_t-(C_3-C_7$ cycloalkyl);

wherein the alkyl and cycloalkyl groups in the definition of $R^1$ are optionally substituted with $C_1-C_4$ alkyl, hydroxy, $C_1-C_4$ alkoxy, carboxyl,$-CONH_2$, $-SO_m-(C_1-C_6$ alkyl), $-CO_2-(C_1-C_4$ alkyl) ester, 1H-tetrazol-5-yl, or 1, 2, or 3 fluoro groups;
$Y^1$ is O, $SO_m$, $-CONX^6-$, -CH=CH-, -C≡C-, $-NX^6CO-$, $-CONX^6-$, $-CO_2-$, $-OCONX^6-$ or -OCO-;

q is 0, 1, 2, 3, or 4;
t is 0, 1, 2, or 3;
said $(CH_2)_q$ group and $(CH_2)_t$ group in the definition of $R^1$ are optionally independently substituted with hydroxy, $C_1$-$C_4$ alkoxy, carboxyl, -$CONH_2$,-$SO_m$-($C_1$-$C_6$ alkyl), -$CO_2$-($C_1$-$C_4$ alkyl) ester, 1H-tetrazol-5-yl, 1, 2, or 3 fluoro groups, or 1 or 2 $C_1$-$C_4$ alkyl groups;

$R^{1A}$ is selected from the group consisting of hydrogen, F, Cl, Br, I, $C_1$-$C_6$ alkyl, phenyl-($C_1$-$C_3$ alkyl), pyridyl-($C_1$-$C_3$ alkyl), thiazolyl-($C_1$-$C_3$ alkyl), and thienyl-($C_1$-$C_3$ alkyl), provided that $R^{1A}$ is not F, Cl, Br, or I when a heteroatom is vicinal to C";
$R^2$ is hydrogen, $C_1$-$C_8$ alkyl, -($C_0$-$C_3$ alkyl)-($C_3$-$C_8$ cycloalkyl), -($C_1$-$C_4$ alkyl)-$A^1$, or $A^1$, wherein the alkyl groups and the cycloalkyl groups in the definition of $R^2$ are optionally substituted with hydroxy, -$CO_2X^6$, -$CONX^6X^6$, -$NX^6X^6$, -$SO_m$($C_1$-$C_6$ alkyl),-$COA^1$, -$COX^6$, $CF_3$, CN, or 1, 2, or 3 independently selected halo groups;
$R^3$ is selected from the group consisting of $A^1$, $C_1$-$C_{10}$ alkyl, -($C_1$-$C_6$ alkyl)-$A^1$,-($C_1$-$C_6$ alkyl)-($C_3$-$C_7$ cycloalkyl), -($C_1$-$C_5$ alkyl)-$X^1$-($C_1$-$C_5$ alkyl), -($C_1$-$C_5$ alkyl)-$X^1$-($C_0$-$C_5$ alkyl)-$A^1$, and -($C_1$-$C_5$ alkyl)-$X^1$-($C_1$-$C_5$ alkyl)-($C_3$-$C_7$ cycloalkyl);

wherein the alkyl groups in the definition of $R^3$ are optionally substituted with -$SO_m$($C_1$-$C_6$ alkyl), -$CO_2X^3$, 1, 2, 3, 4, or 5 independently selected halo groups, or 1, 2, or 3 independently selected -$OX^3$ groups;
$X^1$ is O, $SO_m$, -$NX^2CO$-, -$CONX^2$-, -OCO-, -$CO_2$-, -$CX^2$=$CX^2$-, -$NX^2CO_2$-, -$OCONX^2$-, or -C≡C-;

$R^4$ is hydrogen, $C_1$-$C_6$ alkyl, or $C_3$-$C_7$ cycloalkyl, or $R^4$ taken together with $R^3$ and the carbon atom to which they are attached form $C_5$-$C_7$ cycloalkyl, $C_5$-$C_7$ cycloalkenyl, a partially saturated or fully saturated 4- to 8-membered ring having 1 to 4 heteroatoms independently selected from the group consisting of oxygen, sulfur, and nitrogen, or a bicyclic ring system consisting of a partially saturated or fully saturated 5- or 6-membered ring, fused to a partially saturated, fully unsaturated, or fully saturated 5- or 6-membered ring, optionally having 1 to 4 heteroatoms independently selected from the group consisting of nitrogen, sulfur, and oxygen;
$X^4$ is hydrogen or $C_1$-$C_6$ alkyl, or $X^4$ is taken together with $R^4$ and the nitrogen atom to which $X^4$ is attached and the carbon atom to which $R^4$ is attached and form a five to seven membered ring;
$R^6$ is a bond or is

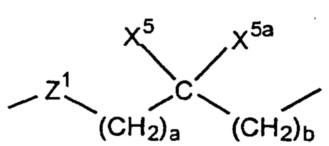
;

wherein a and b are each independently 0, 1, 2, or 3;
$X^5$ and $X^{5a}$ are each independently selected from the group consisting of hydrogen, $CF_3$, $A^1$, and $C_1$-$C_6$ alkyl optionally substituted with $A^1$, $OX^2$,-$SO_m$-($C_1$-$C_6$ alkyl), -$CO_2X^2$, $C_3$-$C_7$ cycloalkyl, -$NX^2X^2$, or -$CONX^2X^2$;
or the carbon bearing $X^5$ or $X^{5a}$ forms one or two alkylene bridges with the nitrogen atom bearing $R^7$ and $R^8$ wherein each alkylene bridge contains 1 to 5 carbon atoms, provided that when one alkylene bridge is formed then only one of $X^5$ or $X^{5a}$ is on the carbon atom and only one of $R^7$ or $R^8$ is on the nitrogen atom, and further provided that when two alkylene bridges are formed then $X^5$ and $X^{5a}$ cannot be on the carbon atom and $R^7$ and $R^8$ cannot be on the nitrogen atom;
or $X^5$ taken together with $X^{5a}$ and the carbon atom to which they are attached form a partially saturated or fully saturated 3- to 7-membered ring, or a partially saturated or fully saturated 4- to 8-membered ring having 1 to 4 heteroatoms independently selected from the group consisting of oxygen, sulfur, and nitrogen;
or $X^5$ taken together with $X^{5a}$ and the carbon atom to which they are attached form a bicyclic ring system consisting of a partially saturated or fully saturated 5- or 6-membered ring, optionally having 1 or 2 heteroatoms independently selected from the group consisting of nitrogen, sulfur, and oxygen, fused to a partially saturated, fully saturated, or fully unsaturated 5- or 6-membered ring, optionally having 1 to 4 heteroatoms independently selected from the group consisting of nitrogen, sulfur, and oxygen;
$Z^1$ is a bond, O, or N-$X^2$, provided that when a and b are both 0 then $Z^1$ is not N-$X^2$ or O;

$R^7$ and $R^8$ are each independently hydrogen or $C_1$-$C_6$ alkyl optionally independently substituted with $A^1$, -$CO_2$-($C_1$-$C_6$ alkyl), -$SO_m$($C_1$-$C_6$ alkyl), 1 to 5 halo groups, 1 to 3 hydroxy groups, 1 to 3 -O-CO($C_1$-$C_{10}$ alkyl) groups, or

1 to 3 $C_1$-$C_6$ alkoxy groups; or
$R^7$ and $R^8$ can be taken together to form $(CH_2)_r$-L-$(CH_2)_r$-, wherein L is $CX^2X^2$, $SO_m$, or $NX^2$;
$R^9$ and $R^{10}$ are each independently selected from the group consisting of hydrogen, fluoro, hydroxy, and $C_1$-$C_5$ alkyl optionally independently substituted with 1-5 halo groups;
$R^{11}$ is selected from the group consisting of $C_1$-$C_5$ alkyl and phenyl optionally substituted with 1-3 substituents each independently selected from the group consisting of $C_1$-$C_5$ alkyl, halo, and $C_1$-$C_5$ alkoxy;
$R^{12}$ is selected from the group consisting of $C_1$-$C_5$ alkylsulfonyl, $C_1$-$C_5$ alkanoyl, and $C_1$-$C_5$ alkyl wherein the alkyl portion is optionally independently substituted by 1-5 halo groups;
$A^1$ for each occurrence is independently selected from the group consisting of $C_5$-$C_7$ cycloalkenyl, phenyl, a partially saturated, fully saturated, or fully unsaturated 4-to 8-membered ring optionally having 1 to 4 heteroatoms independently selected from the group consisting of oxygen, sulfur, and nitrogen, and a bicyclic ring system consisting of a partially saturated, fully unsaturated, or fully saturated 5- or 6-membered ring, optionally having 1 to 4 heteroatoms independently selected from the group consisting of nitrogen, sulfur, and oxygen, fused to a partially saturated, fully saturated, or fully unsaturated 5- or 6-membered ring, optionally having 1 to 4 heteroatoms independently selected from the group consisting of nitrogen, sulfur, and oxygen;

$A^1$ for each occurrence is independently optionally substituted, on one or optionally both rings if $A^1$ is a bicyclic ring system, with up to three substituents, each substituent independently selected from the group consisting of F, Cl, Br, I, $OCF_3$, $OCF_2H$, $CF_3$, $CH_3$, $OCH_3$, $-OX^6$, $-CONX^6X^6$,$-CO_2X^6$, oxo, $C_1$-$C_6$ alkyl, nitro, cyano, benzyl, $-SO_m(C_1$-$C_6$ alkyl), 1H-tetrazol-5-yl, phenyl, phenoxy, phenylalkyloxy, halophenyl, methylenedioxy, $-NX^6X^6$,$-NX^6COX^6$, $-SO_2NX^6X^6$, $-NX^6SO_2$-phenyl, $NX^6SO_2X^6$, $-CONX^{11}X^{12}$, $-SO_2NX^{11}X^{12}$, $-NX^6SO_2X^{12}$, $-NX^6CONX^{11}X^{12}$, $-NX^6SO_2NX^{11}X^{12}$, $-NX^6COX^{12}$, imidazolyl, thiazolyl, and tetrazolyl, provided that if $A^1$ is optionally substituted with methylenedioxy then it can only be substituted with one methylenedioxy;

wherein $X^{11}$ is hydrogen or $C_1$-$C_6$ alkyl optionally independently substituted with phenyl, phenoxy, $C_1$-$C_6$ alkoxycarbonyl, $-SO_m(C_1$-$C_6$ alkyl), 1 to 5 halo groups, 1 to 3 hydroxy groups, 1 to 3 $C_1$-$C_{10}$ alkanoyloxy groups, or 1 to 3 $C_1$-$C_6$ alkoxy groups;
$X^{12}$ is hydrogen, $C_1$-$C_6$ alkyl, phenyl, thiazolyl, imidazolyl, furyl, or thienyl, provided that when $X^{12}$ is not hydrogen, the $X^{12}$ group is optionally substituted with one to three substituents independently selected from the group consisting of Cl, F, $CH_3$, $OCH_3$, $OCF_3$, and $CF_3$;
or $X^{11}$ and $X^{12}$ are taken together to form $(CH_2)_r$-$L^1$-$(CH_2)_r$-, wherein $L^1$ is $CX^2X^2$, O, $SO_m$, or $NX^2$;

r for each occurrence is independently 1, 2, or 3;
$X^2$ for each occurrence is independently hydrogen, optionally substituted $C_1$-$C_6$ alkyl, or optionally substituted $C_3$-$C_7$ cycloalkyl, wherein the optionally substituted $C_1$-$C_6$ alkyl and optionally substituted $C_3$-$C_7$ cycloalkyl in the definition of $X^2$ are optionally independently substituted with $-SO_m(C_1$-$C_6$ alkyl), $-CO_2X^3$, 1 to 5 halo groups, or 1-3 $OX^3$ groups;
$X^3$ for each occurrence is independently hydrogen or $C_1$-$C_6$ alkyl;
$X^6$ for each occurrence is independently hydrogen, optionally substituted $C_1$-$C_6$ alkyl, halogenated $C_2$-$C_6$ alkyl, optionally substituted $C_3$-$C_7$ cycloalkyl, halogenated $C_3$-$C_7$ cycloalkyl, wherein the optionally substituted $C_1$-$C_6$ alkyl and optionally substituted $C_3$-$C_7$ cycloalkyl in the definition of $X^6$ are optionally independently mono- or di-substituted with $C_1$-$C_4$ alkyl, hydroxy, $C_1$-$C_4$ alkoxy, carboxyl, $CONH_2$, $-SO_m(C_1$-$C_6$ alkyl), carboxylate ($C_1$-$C_4$ alkyl) ester, or 1H-tetrazol-5-yl; or
when there are two $X^6$ groups on one atom and both $X^6$ are independently $C_1$-$C_6$ alkyl, the two $C_1$-$C_6$ alkyl groups may be optionally joined, and together with the atom to which the two $X^6$ groups are attached, form a 4- to 9-membered ring optionally having oxygen, sulfur, or $NX^7$ as a ring member, wherein $X^7$ is hydrogen or $C_1$-$C_6$ alkyl optionally substituted with hydroxy;
m for each occurrence is independently 0, 1, or 2; with the provisos that:

$X^6$ and $X^{12}$ cannot be hydrogen when attached to CO or $SO_2$ in the form $COX^6$, $COX^{12}$, $SO_2X^6$ or $SO_2X^{12}$; and when $R^6$ is a bond then L is $NX^2$ and each r in the definition $-(CH_2)_r$-L-$(CH_2)_r$- is independently 2 or 3.

Another group of preferred growth hormone secretagogues for use in the compositions, methods, and kits of the present invention are those wherein the growth hormone secretagogue is 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide; 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide; 2-amino-N-{1(R)-benzyloxymethyl-2-[1,3-dioxo-8a(S)-pyridin-2-ylmethyl-2-(2,2,2-trifluoro-ethyl)-hexahydro-imidazo[1,5-a]pyrazin-7-yl]-2-oxo-ethyl}-2-methyl-propionamide; N-(1(R)-((1,2-dihydro-1-methanesulfonyl-spiro(3H-indole-3,4'-piperidin)-1'-yl)carbonyl)-2-(phenylmethyloxy)ethyl)-2-amino-2-methyl-propanamide; or a prodrug of any of these compounds, or a pharmaceutically acceptable salt of any of said compounds or said prodrugs.

DETAILED DESCRIPTION OF THE INVENTION

The present invention is directed to pharmaceutical compositions, methods, and kits comprising a CRF antagonist and a growth hormone secretagogue or growth hormone useful for treating a wide variety of diseases and conditions as fully described herein. While many specific compounds that serve as CRF antagonists, growth hormones, or growth hormone secretagogues are described and discussed herein, all such compounds, either cited herein or not, presently known or yet to be discovered, are considered to be useful in the practice of the present invention.

The second component of the compositions, methods, and kits of the present invention is a growth hormone secretagogue or growth hormone *per se.*

A representative first class of growth hormone secretagogues is set forth in PCT publication WO 97/24369, which is incorporated herein by reference, as compounds having the formula III:

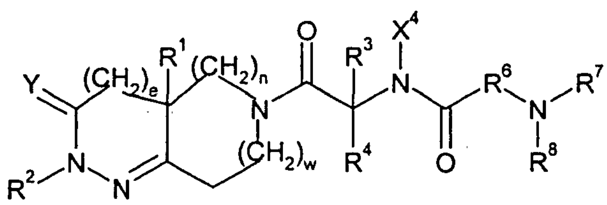

III

wherein the various substituents are as defined in WO 97/24369. Said compounds are prepared as disclosed therein.

Preferred members of this first class of growth hormone secretagogues are 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, having the following structure:

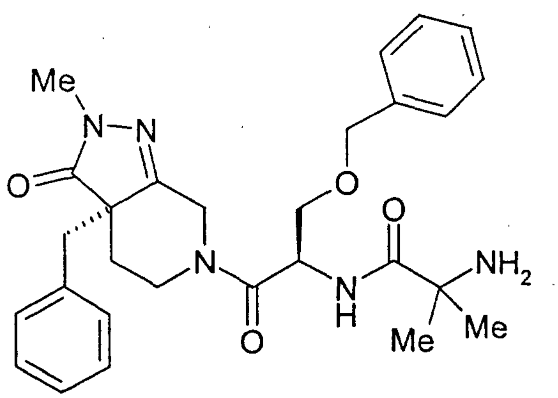

and 2-amino-N-(1-(R)-(2,4-difluoro-benzyloxymethyl)-2-oxo-2-(3-oxo-3a-(R)-pyridin-2-ylmethyl)-2-(2,2,2-trifluoroethyl)-2,3,3a,4,6,7-hexahydro-pyrazolo-[4,3-c]pyridin-5-yl)-ethyl)-2-methyl-propionamide, having the following structure:

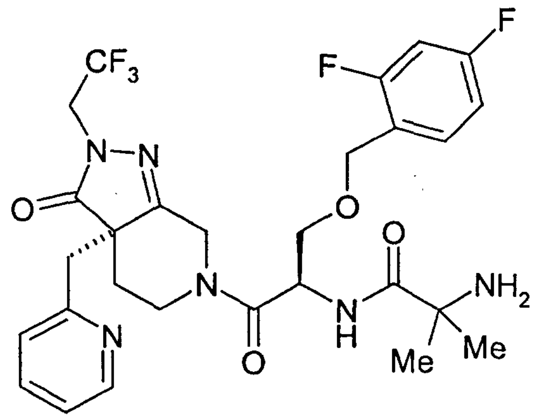

both of which are within the scope of the disclosure of international patent application publication number WO 97/24369. With respect to the latter compound, see also WO 98/58948.

A representative second class of growth hormone secretagogues is set forth in U.S. patent No., 5,206,235, which is incorporated herein by reference, as having the following structure:

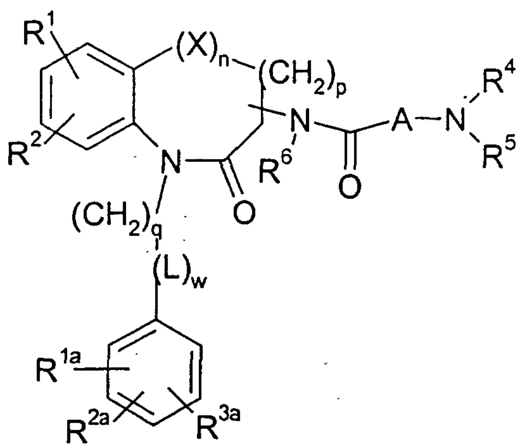

wherein the various substituents are as defined in U.S. patent 5,206,235. Said compounds are prepared as disclosed therein.

Preferred compounds within this second class include 3-(2(R)-hydroxypropyl)amino-3-methyl-N-[2,3,4,5-tetrahydro-2-oxo-1-[[2'-(1H-tetrazol-5-yl)[1,1'-biphenyl]-4-yl]methyl-1H-1-benzazepin-3(R)-yl]butanamide, having the following structure:

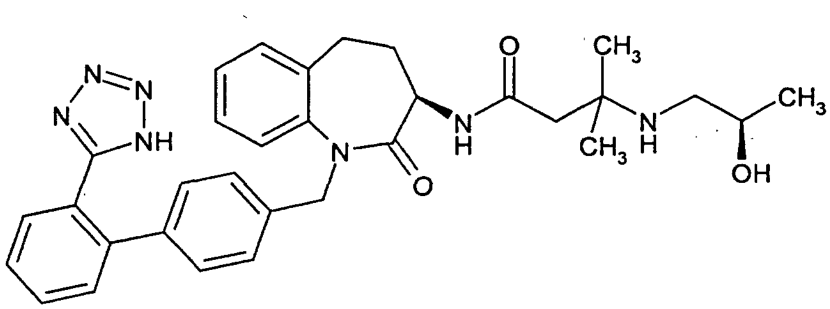

and 3-amino-3-methyl-N-[2,3,4,5-tetrahydro-2-oxo-1-[[2'-(1H-tetrazol-5-yl)[1,1'-biphenyl]-4-yl]methyl-1H-1-benzazepin-3(R)-yl]butanamide, having the following structure:

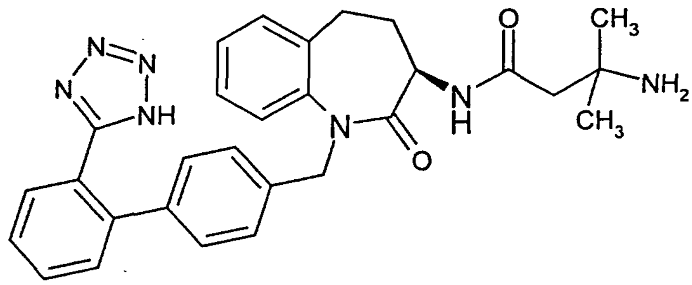

both of which are disclosed in U.S. patent 5,206,235.

A representative third class of growth hormone secretagogues is set forth in U.S. patent 5,283,241, which is incorporated herein by reference, as having the following formula:

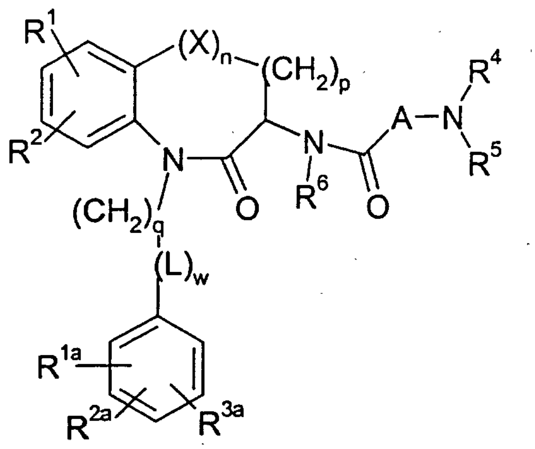

wherein the various substituents are as defined in U.S. patent 5,283,241. Said compounds are prepared as disclosed therein.

A representative fourth class of growth hormone secretagogues is disclosed in PCT publication WO 97/41879, which is incorporated herein by reference, as compounds having the following formulas:

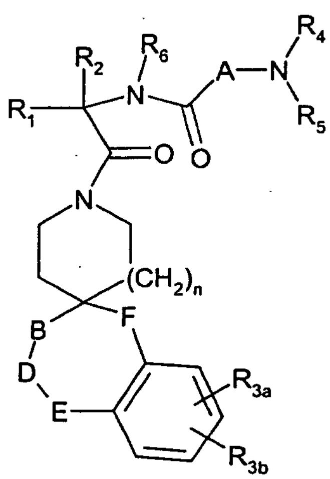

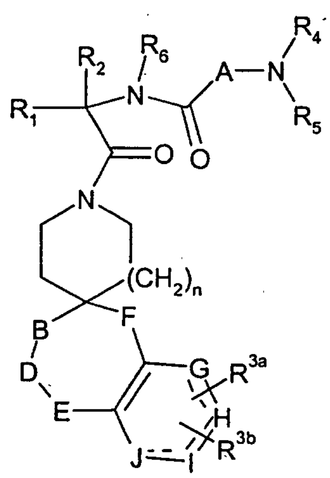

wherein the various substituents are as defined in WO 97/41879. Said compounds are prepared as disclosed therein.

The most preferred compound within this fourth class which may be employed in the present invention is identified as N-[1(R)-[(1,2-dihydro-1-methanesulfonylspiro[3H-indole-3,4'-piperidin]-1'-yl)carbonyl]-2-(phenyl-methyloxy)ethyl]-2-amino-2-methylpropanamide, having the following structure:

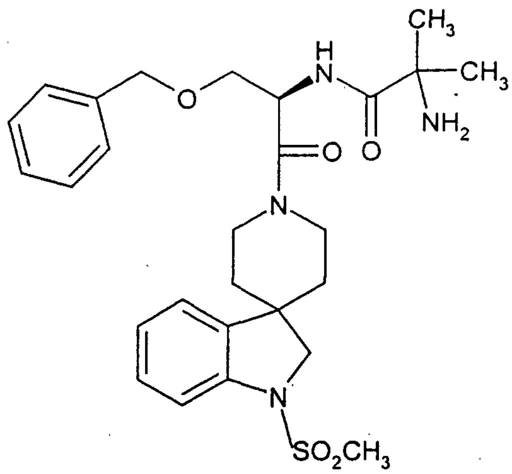

or a pharmaceutically acceptable salt thereof, in particular, the methanesulfonate salt, all of which are disclosed in WO 97/41879.

A representative fifth class of growth hormone secretagogues is disclosed in U.S. patent 5,492,916, which is incorporated herein by reference, as being compounds of the formula:

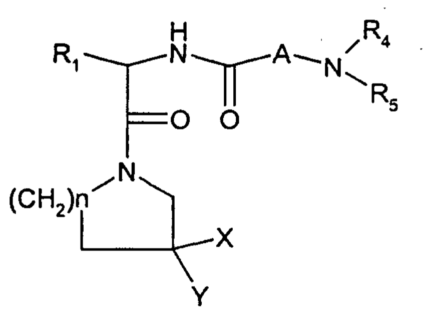

wherein the various substituents are as defined in U.S. patent 5,492,916. Said compounds are prepared as disclosed therein.

A representative sixth class of growth hormone secretagogues is set forth in WO 98/58947, as compounds having the formula:

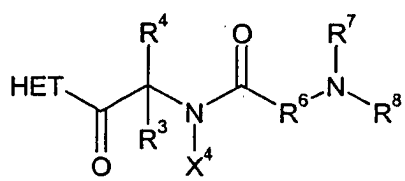

IV

wherein the various substituents are as defined in WO 98/58947. The preparation of the compounds of formula IV of the present invention can be carried out in sequential or convergent synthetic routes. Syntheses detailing the preparation of the compounds of formula IV in a sequential manner are presented in WO 98/58947.

The expression "prodrug" refers to compounds that are drug precursors which, following administration, release the drug *in vivo* via some chemical or physiological process (e.g., a prodrug on being brought to the physiological pH is converted to the desired drug form). A prodrug of any or all of the compounds (i.e., a CRF antagonist, a growth hormone secretagogue, or a growth hormone) may be used in the methods, kits, and compositions of the instant invention. Upon cleavage, exemplary prodrugs release the corresponding free acid (where applicable), and such hydrolyzable ester-forming residues of the prodrugs of this invention include but are not limited to carboxylic acid substituents wherein the free hydrogen is replaced by $(C_1\text{-}C_4)$alkyl, $(C_2\text{-}C_{12})$alkanoyloxymethyl, $(C_4\text{-}C_9)$1-(alkanoyloxy)

ethyl, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl) amino)ethyl having from 4 to 10 carbon atoms, 3-phthalidyl, 4-crotonolactonyl, gamma-butyrolacton-4-yl, di-N,N-($C_1$-$C_2$)alkylamino($C_2$-$C_3$)alkyl (such as β-dimethylaminoethyl), carbamoyl-($C_1$-$C_2$)alkyl, N,N-di($C_1$-$C_2$)-alkylcarbamoyl-($C_1$-$C_2$)alkyl, piperidino-, pyrrolidino-, or morpholino($C_2$-$C_3$)alkyl, and the like.

Other exemplary prodrugs (where applicable) are derivatives of an alcohol of the compounds used in this invention wherein the free hydrogen of a hydroxyl substituent is replaced by ($C_1$-$C_6$)alkanoyloxymethyl, 1-(($C_1$-$C_6$) alkanoyloxy)ethyl, 1-methyl-1-(($C_1$-$C_6$)alkanoyloxy)ethyl, ($C_1$-$C_6$)alkoxycarbonyloxymethyl, N-($C_1$-$C_6$)alkoxy-carbonylamino-methyl, succinoyl, ($C_1$-$C_6$)alkanoyl, α-amino($C_1$-$C_4$)alkanoyl, arylacetyl, α-aminoacyl, α-aminoacyl-α-aminoacyl wherein said α-aminoacyl moieties are independently any of the naturally occurring L-amino acids found in proteins,-$P(O)(OH)_2$, -P(O)(O($C_1$-$C_6$)alkyl)$_2$, glycosyl (the radical resulting from detachment of the hydroxyl of the hemiacetal of a carbohydrate), or the like.

Of the compositions, methods, and kits of the present invention as defined and claimed herein, particularly preferred are those compositions, methods, and kits that contain one of the following two CRF antagonists: 4-(1-ethyl-propoxy)-3,6-dimethyl-2-(2,4,6-trimethylphenoxy)-pyridine, having the formula

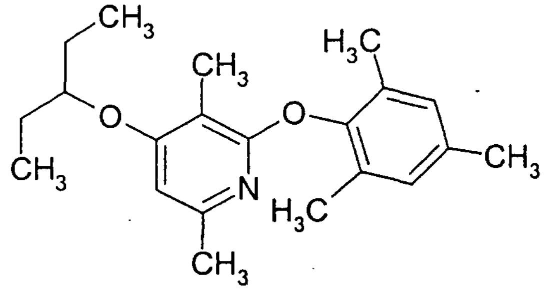

or (3,6-dimethyl-2-(2,4,6-trimethyl-phenoxy)-pyridin-4-yl)-(1-ethyl-propyl)-amine, having the formula

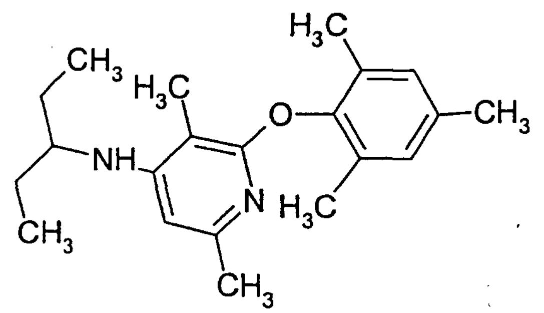

and alternatively one of the following two growth hormone secretagogues: 2-amino-N-[2-(3a(R)-benzyl-2-methyl-3-oxo-2,3,3a,4,6,7-hexahydro-pyrazolo-[4,3-c]pyridin-5-yl)-1(R)-benzyloxymethyl-2-oxo-ethyl]-isobutyramide, having the formula:

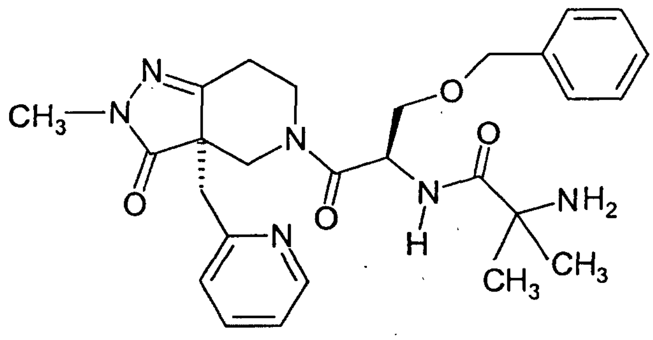

or 2-amino-N-(1(R)-(2,4-difluoro-benzyloxymethyl)-2-oxo-2-(3-oxo-3a(R)-(pyridin-2-ylmethyl)-2-(2,2,2-trifluoro-ethyl)-2,3,3a,4,6,7-hexahydro-pyrazolo-[4,3-c]pyridin-5-yl)- )L ethyl)-2-methyl-propionamide, having the formula:

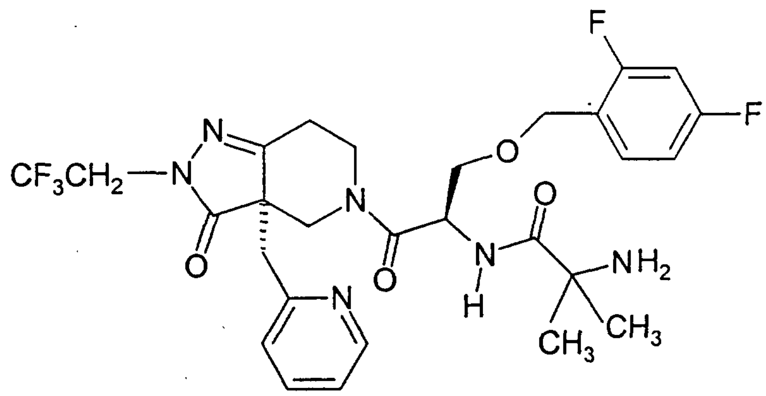

In the preferred kits of the present invention, the pharmaceutical composition comprising a CRF antagonist is a pharmaceutical composition comprising one of the preferred CRF antagonists as defined above (i.e., 4-(1-ethyl-propoxy)-3,6-dimethyl-2-(2,4,6-trimethylphenoxy)-pyridine or (3,6-dimethyl-2-(2,4,6-trimethyl-phenoxy)-pyridin-4-yl)-(1-ethyl-propyl)-amine).

In the preferred kits of the present invention, the pharmaceutical composition comprising a growth hormone secretagogue is a pharmaceutical composition comprising one of the preferred growth hormone secretagogues as defined above (i.e., 2-amino-N-[2-(3a(R)-benzyl-2-methyl-3-oxo-2,3,3a,4,6,7-hexahydro-pyrazolo-[4,3-c]pyridin-5-yl)-1(R)-benzyloxymethyl-2-oxo-ethyl]-isobutyramide or 2-amino-N-(1(R)-(2,4-difluoro-benzyloxymethyl)-2-oxo-2-(3-oxo-3a(R)-(pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide).

In the preferred kits of the present invention comprising both a pharmaceutical composition comprising a CRF antagonist and a pharmaceutical composition comprising a growth hormone secretagogue, the pharmaceutical composition comprising a CRF antagonist comprises a preferred CRF antagonist as defined above and the pharmaceutical composition comprising a growth hormone secretagogue comprises a preferred growth hormone secretagogue as defined herein.

The preferred methods of treatment of the present invention are those methods that employ a preferred CRF antagonist, growth hormone secretagogue, or a pharmaceutical composition(s) of the present invention, as defined herein.

Also preferred are those methods that employ a preferred CRF antagonist, growth hormone secretagogue, or a pharmaceutical composition(s) of the present invention, as defined herein, for treating or preventing osteoporosis or frailty associated with aging or obesity, cardiovascular or heart related disease, in particular hypertension, tachycardia, and congestive heart failure, accelerating bone fracture repair, attenuating protein catabolic response after a major. operation, reducing cachexia and protein loss due to chronic illness, accelerating wound healing, or accelerating the recovery of burn patients or of patients having undergone major surgery.

Presently most preferred, the pharmaceutical compositions, methods, and kits of the present invention can be used for treating and preventing congestive heart failure.

Preferably, the combinations of pharmaceutically active compounds of the present invention show a synergistic effect and/or show less side effects, as compared to the individual compounds, when treating a mammal, preferably a human. Thus, in treating or preventing a particular disease, at a specific dosage level, the combinations of the present invention show a better activity than the activity which could be expected when administering the individual compounds and/or show less (or less severe) side effects than could be expected when administering the individual compounds.

The compositions and combinations of this invention can be administered by oral, parenteral (e.g., intramuscular, intraperitoneal, intravenous, or subcutaneous injection, or through an implant), nasal, vaginal, rectal, sublingual, or topical routes of administration and can be formulated with pharmaceutically acceptable carriers, vehicles, or diluents to provide dosage forms appropriate for each route of administration.

Solid dosage forms for oral administration include capsules, tablets, pills, powders, granules, and the like, and for non-human mammals (cats and dogs are the presently preferred non-human mammals) the solid dosage forms can include admixtures with food and chewable forms. In such solid dosage forms, the compounds and combinations of this invention can be admixed with at least one inert pharmaceutically acceptable carrier such as sucrose, lactose, starch, or the like. Such dosage forms can also comprise, as is normal practice, additional substances other than such inert diluents, e.g., lubricating agents such as magnesium stearate. In the case of capsules, tablets, and pills, the dosage forms may also comprise buffering agents. Tablets and pills can additionally be prepared with enteric coatings. In the case of chewable forms, the dosage form may comprise flavoring agents and perfuming agents.

Liquid dosage forms for oral administration include pharmaceutically acceptable emulsions, solutions, suspensions, syrups, and elixirs containing inert diluents commonly used in the art, such as water. Besides such inert diluents, compositions can also include adjuvants (such as wetting agents), emulsifying and suspending agents, sweetening agents, flavorings, perfuming agents, and the like.

Preparations according to this invention for parenteral administration include sterile aqueous or non-aqueous solutions, suspensions, emulsions, and the like. Examples of non-aqueous solvents or vehicles are propylene glycol, polyethylene glycol, vegetable oils such as olive oil and corn oil, gelatin, and injectable organic esters such as ethyl oleate. Such dosage forms may also contain adjuvants such as preserving, wetting, emulsifying, and dispersing agents. They may be sterilized, for example, by filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories that may contain, in addition to the active substance, excipients such as cocoa butter or a suppository wax.

Compositions for nasal or sublingual administration are also prepared with standard excipients well known in the art.

The dosage of active ingredients in the compositions and methods of this invention may be varied; however, it is necessary that the amount of the active ingredients in such compositions be such that a suitable dosage form is obtained. The selected dosage depends upon the desired therapeutic effect, on the route of administration, the particular compounds administered, the duration of the treatment, and other factors. All dosage ranges and dosage levels mentioned herein refer to each pharmaceutically active compound present in the pharmaceutical compositions and kits of the present invention, as well as those used in the methods of the present invention. Generally, dosage levels of between 0.0001 to 100 mg/kg of body weight daily are administered to humans and other animals, e.g., mammals.

A preferred dosage range in humans is 0.01 to 5.0 mg/kg of body weight daily which can be administered as a single dose or divided into multiple doses.

A preferred dosage range in mammals other than humans is 0.01 to 10.0 mg/kg of body weight daily which can be administered as a single dose or divided into multiple doses. A more preferred dosage range in mammals other than humans is 0.1 to 5.0 mg/kg of body weight daily which can be administered as a single dose or divided into multiple doses.

The present invention includes within its scope the use of a combination of this invention, e.g., a corticotropin releasing factor antagonist and a growth hormone secretagogue or growth hormone, for the prevention or treatment of congestive heart failure in mammals. The preferred mammal for purposes of this invention is a human.

Since the present invention has an aspect that relates to treatment with a combination of active ingredients which may be administered separately, the invention also relates to combining separate pharmaceutical compositions in kit form. Thus, in one embodiment, the kit comprises two separate pharmaceutical compositions: a corticotropin releasing factor antagonist, a prodrug thereof, or a pharmaceutically acceptable salt of said corticotropin releasing factor antagonist or said prodrug; and a growth hormone secretagogue, a prodrug thereof, or a pharmaceutically acceptable salt of said growth hormone secretagogue or said prodrug. The kit also comprises a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably

administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil that is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of a corticotropin releasing factor antagonist, a prodrug thereof, or a pharmaceutically acceptable salt of said corticotropin releasing factor antagonist or said prodrug can consist of one tablet or capsule, while a daily dose of the growth hormone secretagogue, prodrug thereof, or pharmaceutically acceptable salt of said growth hormone secretagogue or said prodrug can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter that indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

In another embodiment, the present invention comprises kits comprising a pharmaceutical composition, a package, and a package insert. The pharmaceutical composition of these kits contains either a corticotropin releasing factor antagonist or a growth hormone/growth hormone secretagogue. The kits of the present invention containing a pharmaceutical composition containing a corticotropin releasing factor antagonist differ from known kits containing a pharmaceutical composition containing a corticotropin releasing factor antagonist in that on the package and/or on the package insert of the kits it is stated that the pharmaceutical composition is to be administered together with a pharmaceutical composition containing a growth hormone or growth hormone secretagogue. The kits of the present invention containing a pharmaceutical composition containing a growth hormone or growth hormone secretagogue differ from known kits containing a pharmaceutical composition containing a growth hormone or growth hormone secretagogue in that on the package and/or on the package insert of the kits it is stated that the pharmaceutical composition is to be administered together with a pharmaceutical composition containing a corticotropin releasing factor antagonist.

The term "together with" as used in the immediately preceding paragraph is intended to encompass the simultaneous administration of the two pharmaceutical compositions (e.g., a tablet containing one pharmaceutical composition is to be administered orally while the other pharmaceutical composition is administered by way of infusion, two tablets or capsules are to be swallowed together, etc.). The term "together with" is also intended to include the administration of the two pharmaceutical compositions in a specifically timed manner, i.e., one pharmaceutical composition is to be administered a certain time period after administration of the other pharmaceutical composition. The time period in which the two pharmaceutical compositions are to be administered must be sufficiently short for the corticotropin releasing factor antagonist and the growth hormone secretagogue to exhibit their activity contemporaneously, preferably in a synergistic manner. The exact time period depends on the specific compounds of the pharmaceutical compositions, the application route, the kind and severeness of the disease to be treated, the kind, age, and condition of the patient to be treated, etc., and can be determined by a physician using known methods in combination with the disclosure of the present invention. Generally, the two compositions are to be administered within one day, preferably within 5 hours, more preferably within 2 hours, and even more preferably within one hour. Most preferably, the two compositions are to be administered at the same time or one immediately after the other.

Methods that may be used to determine CRF antagonist activity of the compounds employed to practice the present invention are as described in, e.g., Wynn et al., *Endocrinology,* 116:1653-59 (1985), and Grigoriadis et al., *Peptides,* 10:179-88 (1989). Methods that can be used to determine the CRF binding protein inhibiting activity of compounds employed to practice the present invention are described in Smith et al., *Brain Research,* 745(1,2):248-56 (1997). These methods determine the binding affinity of a test compound for a CRF receptor, which is highly related

to its expected activity as a CRF antagonist.

The combinations of this invention, i.e., a corticotropin releasing factor antagonist and growth hormone or a growth hormone secretagogue, may be tested for hypoglycemic activity according to the following procedure.

Five to eight week old C57 BL/6J-ob/ob mice (obtained from Jackson Laboratory, Bar Harbor, Maine) are housed five per cage under standard animal care practices. After a one week acclimation period, the animals are weighed and 25 microliters of blood are collected via an ocular bleed prior to any treatment. The blood sample is immediately diluted 1:5 with saline containing 2% sodium heparin, and held on ice for glucose analysis. Animals are then regrouped, in groups of five per cage, such that the mean glucose values of the groups are similar, dosed daily for five days with test compounds (0.01-100 mg/kg), a positive control such as englitazone or ciglitazone (50 mg/kg p.o.), (U.S. Patent 4,467,902; Sohda et al., *Chem. Pharm. Bull.*, 32:4460-65 (1984)), or vehicle. All compounds are administered by oral gavage in a vehicle consisting of 0.25% w/v methyl cellulose. On day 5, the animals are weighed again and bled (via the ocular route) for blood glucose level determinations. The freshly collected samples are centrifuged for two minutes at 10,000 x g at room temperature. The supernatant is analyzed for glucose, for example, by the ABA 200 Bichromatic Analyzer™[1], using the A-gent™ glucose UV reagent system[2] (hexokinase method) using 20, 60 and 100 mg/dl standards. Plasma glucose is then calculated by the equation,

$$\text{Plasma glucose (mg/dl)} = \text{Sample value} \times 5 \times 1.67 = \text{Sample value} \times 8.35,$$

where 5 is the dilution factor and 1.67 is the plasma hematocrit adjustment (assuming the hematocrit is 40%).

The animals dosed with vehicle maintain substantially unchanged hyperglycemic glucose levels (e.g., 250 mg/dl), while positive control animals have depressed glucose levels (e.g., 130 mg/dl). The glucose lowering activity of test compounds is expressed in terms of % glucose normalization. For example, a glucose level that is the same as the positive control is expressed as 100%.

## Claims

1. A pharmaceutical composition comprising a corticotropin-releasing factor antagonist and a growth hormone secretagogue or growth hormone.

2. A pharmaceutical composition according to Claim 1 wherein said corticotropin-releasing factor antagonist is a compound of formula

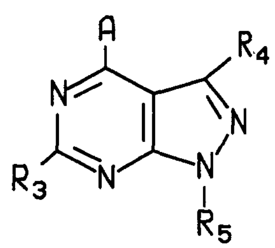

or a pharmaceutically acceptable acid addition salt thereof, wherein

A is $-NR_1R_2$, $-CR_1R_2R_{11}$, $-C(=CR_1R_{12})R_2$, $-NHCR_1R_2R_{11}$, $-OCR_1R_2R_{11}$, $-SCR_1R_2R_{11}$, $-NHNR_1R_2$, $-CR_2R_{11}NHR_1$, $-CR_2R_{11}OR_1$, $-CR_2R_{11}SR_1$, or $-COR_2$;

$R_1$ is hydrogen or $C_1$-$C_6$ alkyl which may be substituted by one or two substituents $R_6$ independently selected from the group consisting of hydroxy, fluoro, chloro, bromo, iodo, $C_1$-$C_6$ alkoxy, -OCO($C_1$-$C_6$ alkyl), -OCON($C_1$-$C_4$ alkyl)($C_1$-$C_2$ alkyl), amino, -NH($C_1$-$C_4$ alkyl), -S($C_1$-$C_6$ alkyl), -OCONH($C_1$-$C_4$ alkyl), -N($C_1$-$C_2$ alkyl)CO($C_1$-$C_4$ alkyl), -NHCO($C_1$-$C_4$ alkyl), -COOH, -CO($C_1$-$C_4$ alkyl), -CONH($C_1$-$C_4$ alkyl), -CON($C_1$-$C_4$ alkyl)($C_1$-$C_2$ alkyl), -SH, -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl), wherein said $C_1$-$C_6$ alkyl group may contain one or two double or triple bonds;

[1] ™A registered trademark of Abbott Laboratories, Diagnostics Division, 820 Mission Street, So. Pasadena, CA 91030.
[2] A modification of the method of Richterrich et al., *Schweizerische Medizinische Wochenschrift,* 101:860 (1971).

$R_2$ is $C_1$-$C_{12}$ alkyl, aryl, or -($C_1$-$C_{10}$ alkylene)aryl, wherein said aryl group is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, oxazolyl, or benzoxazolyl; or 3- to 8-membered cycloalkyl or -($C_1$-$C_6$ alkylene)cycloalkyl, wherein said cycloalkyl group or the cycloalkyl moiety of said -($C_1$-$C_6$ alkylene)cycloalkyl group may have one or two of O, S, or NZ, wherein Z is hydrogen, substituted independently for one or two carbons thereof, $C_1$-$C_4$ alkyl, benzyl, or $C_1$-$C_4$ alkanoyl; and wherein $R^2$ may be substituted independently by from one to three of chloro, fluoro, or $C_1$-$C_4$ alkyl or one of hydroxy, bromo, iodo, $C_1$-$C_6$ alkoxy, -OCO($C_1$-$C_6$ alkyl), -OCN($C_1$-$C_4$ alkyl)($C_1$-$C_2$ alkyl), -S($C_1$-$C_6$ alkyl), -$NH_2$, -NH($C_1$-$C_2$ alkyl), -N($C_1$-$C_4$ alkyl)CO($C_1$-$C_4$ alkyl), -NHCO($C_1$-$C_4$ alkyl), -COOH, -$CO_2$($C_1$-$C_4$ alkyl), -CONH($C_1$-$C_4$ alkyl), -CON($C_1$-$C_4$ alkyl)($C_1$-$C_2$ alkyl), -SH, -CN, -$NO_2$, -SO($C_1$-$C_4$ alkyl), -$SO_2$($C_1$-$C_4$ alkyl), $SO_2$NH($C_1$-$C_4$ alkyl), -$SO_2$N($C_1$-$C_4$ alkyl)($C_1$-$C_2$ alkyl), wherein said $C_1$-$C_{12}$ alkyl and $C_1$-$C_{10}$ alkylene groups may contain from one to three double or triple bonds; or

-$NR_1R_2$ or -$CR_1R_2R_{11}$ may form a 4- to 8-membered ring optionally containing one or two double bonds or one or two of O, S, or NZ, wherein Z is hydrogen, $C_1$-$C_4$ alkyl, benzyl, or $C_1$-$C_4$ alkanoyl;

$R_3$ is hydrogen, $C_1$-$C_6$ alkyl, fluoro, chloro, bromo, iodo, hydroxy, amino, -O($C_1$-$C_6$ alkyl), -NH($C_1$-$C_6$ alkyl), -N($C_1$-$C_4$ alkyl)($C_1$-$C_2$ alkyl), -SH, -S($C_1$-$C_4$ alkyl), -SO($C_1$-$C_4$ alkyl), or -$SO_2$($C_1$-$C_4$ alkyl), wherein said $C_1$-$C_4$ alkyl and $C_1$-$C_6$ alkyl groups may contain one or two double or triple bonds and may be substituted by from one to three $R_7$ substituents independently selected from the group consisting of hydroxy, amino, $C_1$-$C_3$ alkoxy, dimethylamino, diethylamino, methylamino, ethylamino, -$NHCOCH_3$, fluoro, chloro and $C_1$-$C_3$ thioalkyl;

$R_4$ is hydrogen, $C_1$-$C_6$ alkyl, fluoro, chloro, bromo, iodo, $C_1$-$C_6$ alkoxy, amino, -NH($C_1$-$C_6$ alkyl), -N($C_1$-$C_6$ alkyl)($C_1$-$C_2$ alkyl), -$SO_n$($C_1$-$C_6$ alkyl), wherein n is 0,1, or 2, cyano, hydroxy, carboxy, or amido, wherein said $C_1$-$C_6$ alkyl group may be substituted by from one to three of hydroxy, amino, carboxy, amido, -NHCO($C_1$-$C_4$ alkyl), -NH($C_1$-$C_4$ alkyl), -N($C_1$-$C_4$ alkyl)($C_1$-$C_2$ alkyl), -$CO_2$($C_1$-$C_4$ alkyl), $C_1$-$C_3$ alkoxy, $C_1$-$C_3$ thioalkyl, fluoro, bromo, chloro, iodo, cyano, or nitro;

$R_5$ is phenyl, naphthyl, thienyl, benzothienyl, pyridyl, quinolyl, pyrazinolyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl benzoxazolyl, oxazolyl, pyrrolidinyl, thiazolidinyl, piperazinyl, piperidinyl, or tetrazolyl; and wherein $R_5$ may be substituted independently by from one to three of fluoro, chloro, bromo, formyl, $C_1$-$C_6$ alkyl, $C_1$-$C_6$ alkoxy, or trifluoromethyl or one of hydroxy, iodo, cyano, nitro, amino, cyclopropyl, -NH($C_1$-$C_4$ alkyl), -N($C_1$-$C_4$ alkyl)($C_1$-$C_2$ alkyl), -$CO_2$($C_1$-$C_4$ alkyl), -CO($C_1$-$C_4$ alkyl), -$SO_2$NH($C_1$-$C_4$ alkyl), -$SO_2$N($C_1$-$C_4$ alkyl)($C_1$-$C_2$ alkyl), -$SO_2NH_2$, -$NHSO_2$($C_1$-$C_4$ alkyl), -S($C_1$-$C_6$ alkyl), or -$SO_2$($C_1$-$C_6$ alkyl), wherein said $C_1$-$C_4$ alkyl and $C_1$-$C_6$ alkyl groups may contain one double or triple bond and may be substituted by one or two of fluoro, chloro, hydroxy, amino, methylamino, dimethylamino, or acetyl, with the proviso that $R_5$ is not unsubstituted phenyl;

$R_{11}$ is hydrogen, hydroxy, fluoro, chloro, -$CO_2$($C_1$-$C_2$ alkyl), cyano, or -CO($C_1$-$C_2$ alkyl); and

$R_{12}$ is hydrogen or $C_1$-$C_4$ alkyl;

with the provisos that:

(a) A is not straight chain $C_1$-$C_{12}$ alkyl;

(b) when $R_3$ is hydrogen, A is benzyl or phenethyl, and $R_4$ is fluoro, chloro, bromo, or iodo, then $R_5$ is not 5'-deoxy-ribofuranosyl or 5'-amino-5'-deoxy-ribofuranosyl; and

(c) when $R^5$ is phenyl, said phenyl is substituted by two or three substituents.

**3.** A pharmaceutical composition according to Claim 1 wherein said corticotropin-releasing factor antagonist is a compound of formula

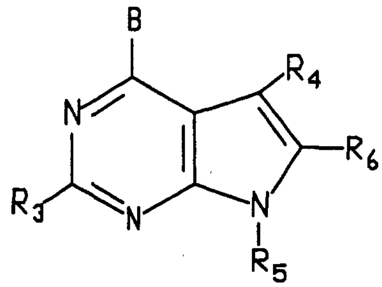

or a pharmaceutically acceptable acid addition salt thereof, wherein

B is $-NR_1R_2$, $-CR_1R_2R_{11}$, $-C(=CR_2R_{12})R_1$, $-NHR_1R_2R_{11}$, $-OCR_1R_2R_{11}$, $-SCR_1R_2R_{11}$, $-NHNR_1R_2$, $-CR_2R_{11}NHR_1$, $-CR_2R_{11}OR_1$, $-CR_2R_{11}SR_1$, or $-C(O)R_2$;

$R_1$ is hydrogen or $C_1$-$C_6$ alkyl which may be substituted by one or two substituents $R_7$ independently selected from the group consisting of hydroxy, fluoro, chloro, bromo, iodo, $C_1$-$C_8$ alkoxy, -OCO($C_1$-$C_6$ alkyl), -OCONH ($C_1$-$C_4$ alkyl), -OCON($C_1$-$C_4$ alkyl)($C_1$-$C_2$ alkyl), amino, -NH($C_1$-$C_4$ alkyl), -N($C_1$-$C_2$ alkyl)($C_1$-$C_4$ alkyl), -S($C_1$-$C_6$ alkyl), -N($C_1$-$C_4$alkyl)CO($C_1$-$C_4$ alkyl), -NH($C_1$-$C_4$ alkyl), -COOH, $-CO_2$($C_1$-$C_4$ alkyl), -CONH($C_1$-$C_4$ alkyl), -CON($C_1$-$C_4$ alkyl) -($C_1$-$C_2$ alkyl), -SH, -CN, $-NO_2$, -SO($C_1$-$C_4$ alkyl), $-SO_2$($C_1$-$C_4$ alkyl), $-SO_2NH$-($C_1$-$C_4$ alkyl) and $-SO_2N$($C_1$-$C_4$ alkyl)($C_1$-$C_2$ alkyl), wherein said $C_1$-$C_6$ alkyl group may contain one or two double or triple bonds;

$R_2$ is $C_1$-$C_{12}$ alkyl, aryl, or -($C_1$-$C_{10}$ alkylene)aryl, wherein said aryl group is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl, or benzoxazolyl; or 3- to 8-membered cycloalkyl or -($C_1$-$C_6$ alkylene)cycloalkyl, wherein said cycloalkyl group or the cycloalkyl moiety of said -($C_1$-$C_6$ alkylene)cycloalkyl group may contain one or two of O, S, or NZ, wherein Z is hydrogen, $C_1$-$C_4$ alkyl, benzyl, or $C_1$-$C_4$ alkanoyl; and wherein $R_2$ may be substituted independently by from one to three of chloro, fluoro, or $C_1$-$C_4$ alkyl or one of hydroxy, bromo, iodo, $C_1$-$C_6$ alkoxy, -OCO($C_1$-$C_6$ alkyl), -OCN($C_1$-$C_4$ alkyl)($C_1$-$C_2$ alkyl), -S($C_1$-$C_6$ alkyl), $-NH_2$, -NH($C_1$-$C_2$ alkyl), -N ($C_1$-$C_2$ alkyl)($C_1$-$C_4$ alkyl), -N($C_1$-$C_4$alkyl)CO($C_1$-$C_4$ alkyl), -NHCO($C_1$-$C_4$alkyl), -COOH, $-CO_2$($C_1$-$C_4$ alkyl), -CONH($C_1$-$C_4$ alkyl), -CON($C_1$-$C_4$ alkyl)($C_1$-$C_2$ alkyl), -SH, -CN, $-NO_2$, -SO($C_1$-$C_4$ alkyl), $-SO_2$($C_1$-$C_4$ alkyl), $-SO_2NH$($C_1$-$C_4$ alkyl), or $-SO_2N$($C_1$-$C_4$ alkyl)($C_1$-$C_2$ alkyl), wherein said $C_1$-$C_{12}$ alkyl and $C_1$-$C_{10}$ alkylene groups may contain from one to three double or triple bonds; or

$-NR_1R_2$ or $-CR_1R_2R_{11}$ may form a saturated 3- to 8-membered carbocyclic ring of which the 5- to 8-membered rings may contain one or two double bonds or one or two of O, S, or NZ, wherein Z is hydrogen, $C_1$-$C_4$ alkyl, benzyl, or $C_1$-$C_4$ alkanoyl;

$R_3$ is hydrogen, $C_1$-$C_6$ alkyl, fluoro, chloro, bromo, iodo, hydroxy, amino, -O($C_1$-$C_6$ alkyl), -NH($C_1$-$C_6$ alkyl), -N($C_1$-$C_4$ alkyl)($C_1$-$C_2$ alkyl), -SH, -S($C_1$-$C_4$ alkyl), -SO($C_1$-$C_4$ alkyl), or $-SO_2$($C_1$-$C_4$ alkyl), wherein said $C_1$-$C_4$ alkyl and $C_1$-$C_6$ alkyl groups may contain one or two double or triple bonds and may be substituted by from one to three $R_8$ substituents independently selected from the group consisting of hydroxy, amino, $C_1$-$C_3$ alkoxy, dimethylamino, diethylamino, methylamino, ethylamino, $-NHCH_3$, fluoro, chloro and $C_1$-$C_3$ thioalkyl;

$R_4$ and $R_6$ are each independently hydrogen, $C_1$-$C_6$ alkyl, fluoro, chloro, bromo, iodo, $C_1$-$C_6$ alkoxy, amino, -NH($C_1$-$C_6$ alkyl), -N($C_1$-$C_6$ alkyl)($C_1$-$C_2$ alkyl), $-SO_n$($C_1$-$C_6$ alkyl), wherein n is 0, 1, or 2, cyano, hydroxy, carboxy, or amido, wherein said $C_1$-$C_6$ alkyl group may be substituted by from one to three of hydroxy, amino, carboxy, amido, -NHCO($C_1$-$C_4$ alkyl), -NH($C_1$-$C_4$ alkyl), -N($C_1$-$C_4$ alkyl)($C_1$-$C_2$ alkyl), $-CO_2$($C_1$-$C_4$ alkyl), $C_1$-$C_3$ alkoxy, $C_1$-$C_3$ thioalkyl, fluoro, bromo, chloro, iodo, cyano, or nitro;

$R_5$ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, azaindolyl, benzoxazolyl, oxazolyl, pyrrolidinyl, thiazolidinyl, morpholinyl, piperidinyl, piperazinyl, or tetrazolyl; or 3- to 8-membered cycloalkyl or 9- to 12-membered bicycloalkyl, optionally containing from one to three of O, S, or NZ, wherein Z is hydrogen, $C_1$-$C_4$ alkyl, $C_1$-$C_4$ alkanoyl, phenyl, or phenylmethyl; and wherein $R_5$ may be substituted independently by from one to four of fluoro, chloro, $C_1$-$C_6$ alkyl, $C_1$-$C_6$ alkoxy, or trifluoromethyl or one of bromo, iodo, cyano, nitro, amino, -NH($C_1$-$C_4$ alkyl), -N($C_1$-$C_4$)($C_1$-$C_2$ alkyl), -$CO_2$($C_1$-$C_4$ alkyl), -CO($C_1$-$C_4$ alkyl), -$SO_2$NH($C_1$-$C_4$ alkyl), -$SO_2$N($C_1$-$C_4$ alkyl)($C_1$-$C_2$ alkyl), -$SO_2NH_2$, -$NHSO_2$($C_1$-$C_4$ alkyl), -S($C_1$-$C_6$ alkyl), or -$SO_2$($C_1$-$C_6$ alkyl), wherein said $C_1$-$C_4$ alkyl and $C_1$-$C_6$ alkyl groups may be substituted by one or two of fluoro, chloro, hydroxy, amino, methylamino, dimethylamino, or acetyl, with the proviso that $R_5$ is not unsubstituted phenyl;

$R_{11}$ is hydrogen, hydroxy, fluoro, chloro, -$CO_2$($C_1$-$C_2$ alkyl), cyano, or -CO($C_1$-$C_2$ alkyl); and

$R_{12}$ is hydrogen or $C_1$-$C_4$ alkyl;

with the provisos that

(a) when $R_5$ is 4-bromophenyl, $R_3$ is hydrogen, and $R_4$ and $R_6$ are methyl, then B is not methylamino or ethyl; and

(b) when $R_5$ is 4-bromophenyl and $R_3$, $R_4$ and $R_6$ are methyl, then B is not 2-hydroxyethylamino.

**4.** A pharmaceutical composition according to Claim 1 wherein said corticotropin-releasing factor antagonist is a compound of formula

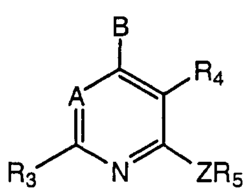
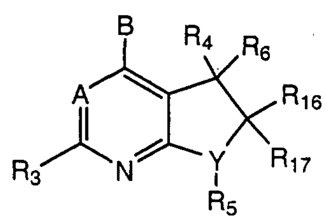
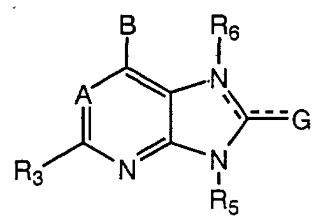

wherein

A is $CR_7$ or N;

B is -$NR_1R_2$, -$CR_1R_2R_{11}$, -C(=$CR_2R_{12}$)$R_1$, -$NHCHR_1R_2$, -$OCHR_1R_2$, -$SCHR_1R_2$, -$CHR_2OR_{12}$, -$CHR_2SR_{12}$, -$CSR_2$, or -$COR_2$;

G is oxygen, sulfur, -NH-, -$NH_3$, hydrogen, methoxy, ethoxy, trifluoromethoxy, methyl, ethyl, thiomethoxy, -NH2, -$NHCH_3$, -$N(CH_3)_2$, or trifluromethyl;

Y is CH or N;

Z is -NH-, -O-, -S-, -N($C_1$-$C_2$ alkyl)-, or -$CR_{13}R_{14}$-, wherein $R_{13}$ and $R_{14}$ are each independently hydrogen, trifluoromethyl, or $C_1$-$C_4$ alkyl; or one of $R_{13}$ and $R_{14}$ may be cyano, chloro, bromo, iodo, fluoro, hydroxy, -O ($C_1$-$C_2$ alkyl), amino, or -NH($C_1$-$C_2$ alkyl); or -$CR_{13}R_{14}$ may be C=O or cyclopropyl;

$R_1$ is $C_1$-$C_6$ alkyl which may be substituted by one or two substituents $R_8$ independently selected from the group consisting of hydroxy, fluoro, chloro, bromo, iodo, $C_1$-$C_4$ alkoxy, -OCO($C_1$-$C_4$ alkyl), -OCONH($C_1$-$C_4$ alkyl), -OCON($C_1$-$C_4$ alkyl)($C_1$-$C_2$ alkyl), -NH($C_1$-$C_4$ alkyl), -N($C_1$-$C_2$ alkyl)($C_1$-$C_4$ alkyl), -S($C_1$-$C_4$ alkyl), -N ($C_1$-$C_4$alkyl)CO($C_1$-$C_4$ alkyl), -NHCO($C_1$-$C_4$ alkyl), -$CO_2$($C_1$-$C_4$ alkyl), -CONH($C_1$-$C_4$ alkyl), -CON($C_1$-$C_4$ alkyl) ($C_1$-$C_2$ alkyl), -S($C_1$-$C_4$ alkyl), -CN, -N02, -SO($C_1$-$C_4$ alkyl) and -$SO_2$($C_1$-$C_4$ alkyl), wherein said $C_1$-$C_6$ alkyl and $C_1$-$C_4$ alkyl groups may contain one double or triple bond;

$R_2$ is $C_1$-$C_{12}$ alkyl, aryl, or -($C_1$-$C_4$ alkylene)aryl, wherein said aryl group is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, or benzoxazolyl; or 3- to 8-membered cycloalkyl or

-($C_1$-$C_6$ alkylene)cycloalkyl, wherein said cycloalkyl group and the cycloalkyl moiety of said -($C_1$-$C_6$ alkylene) cycloalkyl group may contain one or two of O, S, or $NR_9$, wherein $R_9$ is hydrogen or $C_1$-$C_4$ alkyl; and wherein $R_2$ may be substituted independently by from one to three of chloro, fluoro, or $C_1$-$C_4$ alkyl or one of bromo, iodo, $C_1$-$C_6$ alkoxy, -OCO($C_1$-$C_6$ alkyl), -OCON-($C_1$-$C_4$alkyl)($C_1$-$C_2$ alkyl), -S($C_1$-$C_6$ alkyl), -CN, -$NO_2$, -SO($C_1$-$C_4$ alkyl), or

-$SO_2$($C_1$-$C_4$ alkyl), wherein said $C_1$-$C_{12}$ alkyl and $C_1$-$C_4$ alkylene groups may contain one double or triple bond; or

-$NR_1R_2$ or -$CR_1R_2R_{11}$ may form a saturated 5- to 8-membered carbocyclic ring which may contain one or two double bonds or one or two of O or S;

$R_3$ is methyl, ethyl, fluoro, chloro, bromo, iodo, cyano, methoxy, -$OCF_3$, methylthio, methylsulfonyl, -$CH_2OH$, or -$CH_2OCH_3$;

$R_4$ is hydrogen, $C_1$-$C_4$ alkyl, fluoro, chloro, bromo, iodo, $C_1$-$C_4$ alkoxy, amino, nitro, -NH($C_1$-$C_4$ alkyl), -N($C_1$-$C_4$ alkyl)($C_1$-$C_2$ alkyl), -$SO_n$($C_1$-$C_4$ alkyl), wherein n is 0, 1, or 2, cyano, hydroxy, -CO($C_1$-$C_4$ alkyl), -CHO, or -$CO_2$($C_1$-$C_4$ alkyl), wherein said $C_1$-$C_4$ alkyl may contain one or two double or triple bonds and may be substituted by one or two of hydroxy, amino, carboxy, -$NHCOCH_3$, -NH($C_1$-$C_2$ alkyl), -N($C_1$-$C_2$ alkyl)$_2$, -$CO_2$($C_1$-$C_4$ alkyl), -CO($C_1$-$C_4$ alkyl), $C_1$-$C_3$ alkoxy, $C_1$-$C_3$ thioalkyl, fluoro, chloro, cyano, or nitro;

$R_5$ is phenyl, naphthyl, thienyl, benzothienyl, pyridyl, quinolyl, pyrazinyl, pyrimidyl, furanyl, benzofuranyl, benzothiazolyl, or indolyl; and wherein $R_5$ is substituted independently by from one to three of fluoro, chloro, $C_1$-$C_6$ alkyl, or $C_1$-$C_6$ alkoxy or one of hydroxy, iodo, bromo, formyl, cyano, nitro, trifluoromethyl, amino, -NH($C_1$-$C_4$ alkyl), -N($C_1$-$C_6$)($C_1$-$C_2$ alkyl), -COOH, -$CO_2$($C_1$-$C_4$ alkyl), -CO($C_1$-$C_4$ alkyl), -$SO_2NH$($C_1$-$C_4$ alkyl), -$SO_2N$($C_1$-$C_4$ alkyl)($C_1$-$C_2$ alkyl), -$SO_2NH_2$, -$NHSO_2$($C_1$-$C_4$ alkyl), -S($C_1$-$C_6$ alkyl), or -$SO_2$($C_1$-$C_6$ alkyl), wherein said $C_1$-$C_4$ alkyl and $C_1$-$C_6$ alkyl groups may be substituted by one or two of fluoro, hydroxy, amino, methylamino, dimethylamino, or acetyl;

$R_6$ is hydrogen or $C_1$-$C_6$ alkyl, wherein said $C_1$-$C_6$ alkyl group may be substituted by one of hydroxy, methoxy, ethoxy, or fluoro;

$R_7$ is hydrogen, $C_1$-$C_4$ alkyl, fluoro, chloro, bromo, iodo, cyano, hydroxy, -O($C_1$-$C_4$ alkyl), -CO($C_1$-$C_4$ alkyl), or $CO_2$($C_1$-$C_4$ alkyl), wherein said $C_1$-$C_4$ alkyl group may be substituted by one of hydroxy, chloro, or bromo or from one to three fluorine atoms;

$R_{11}$ is hydrogen, hydroxy, fluoro, or methoxy;

$R_{12}$ is hydrogen or $C_1$-$C_4$ alkyl; and

$R_{16}$ and $R_{17}$ are each independently hydrogen, hydroxy, methyl, ethyl, methoxy, or ethoxy, except that they are not both methoxy or ethoxy, and -$CR_4R_6$- and -$CR_{16}R_{17}$- may each independently be C=O.

**5.** A pharmaceutical composition according to Claim 1 wherein said corticotropin-releasing factor antagonist is a compound of formula

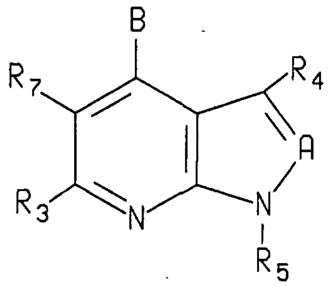

or a pharmaceutically acceptable acid addition salt thereof, wherein

A is N or $CR_6$;

B is $-NR_1R_2$, $-CR_1R_2R_{11}$, $-C(=CR_2R_{12})R_1$, $-NHCHR_1R_2$, $-OCHR_1R_2$, $-SCHR_1R_2$, $-CHR_2OR_{12}$, $-CHR_2SR_{12}$, $-CSR_1$, or $-COR_1$;

$R_1$ is $C_1$-$C_6$ alkyl which may optionally be substituted by one or two substituents independently selected from the group consisting of hydroxy, fluoro, chloro, bromo, iodo, $C_1$-$C_4$ alkoxy, -OCO($C_1$-$C_4$ alkyl), -OCONH($C_1$-$C_4$ alkyl), -OCON($C_1$-$C_4$ alkyl)($C_1$-$C_2$ alkyl), -NH($C_1$-$C_4$ alkyl), -N($C_1$-$C_2$ alkyl)-($C_1$-$C_4$ alkyl), -S($C_1$-$C_4$ alkyl), -N($C_1$-$C_4$alkyl)CO($C_1$-$C_4$ alkyl), -NHCO($C_1$-$C_4$ alkyl), $-CO_2$($C_1$-$C_4$ alkyl), -CONH($C_1$-$C_4$ alkyl), -CON($C_1$-$C_4$ alkyl)($C_1$-$C_2$ alkyl), -CN, $-NO_2$, -SO($C_1$-$C_4$ alkyl) and $-SO_2$($C_1$-$C_4$ alkyl), wherein said $C_1$-$C_4$ alkyl and $C_1$-$C_6$ alkyl groups may optionally contain one double or triple bond;

$R_2$ is $C_1$-$C_{12}$ alkyl, aryl, or -($C_1$-$C_4$ alkylene)aryl, wherein said aryl group is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, oxazolyl, or benzoxazolyl; or 3- to 8-membered cycloalkyl or -($C_1$-$C_6$ alkylene)cycloalkyl, wherein one or two of the ring carbons of said cycloalkyl group having at least 4 ring members and of those cycloalkyl moieties of said -($C_1$-$C_6$ alkylene)cycloalkyl group having at least 4 ring members may optionally be replaced by O, S, or NZ, wherein Z is hydrogen or $C_1$-$C_4$ alkyl; and wherein $R_2$ may optionally be substituted by from one to three substituents independently selected from chloro, fluoro and $C_1$-$C_4$ alkyl or by one substituent selected from bromo, iodo, $C_1$-$C_6$ alkoxy, -OCO($C_1$-$C_6$ alkyl), -S($C_1$-$C_6$ alkyl), $-CO_2$($C_1$-$C_4$ alkyl), -CN, $-NO_2$, -SO($C_1$-$C_4$ alkyl) and $-SO_2$($C_1$-$C_4$ alkyl), wherein said $C_1$-$C_{12}$ alkyl group and the $C_1$-$C_4$ alkylene moiety of said -($C_1$-$C_4$ alkylene) aryl group may optionally contain one double or triple bond; or

$-NR_1R_2$ may form a saturated 5- to 8-membered heterocyclic ring or $-CHR_1R_2$ may form a saturated 5- to 8-membered carbocyclic ring, wherein each of these rings may optionally contain one or two double bonds and wherein one or two of the carbon atoms of each of these rings may optionally be replaced by O or S;

$R_3$ is $C_1$-$C_4$ alkyl, fluoro, chloro, bromo, iodo, $-CH_2OH$, $-CH_2OCH_3$, -O($C_1$-$C_3$ alkyl), -S($C_1$-$C_3$ alkyl), or $-SO_2$ ($C_1$-$C_3$ alkyl), wherein said $C_1$-$C_3$ alkyl group may optionally contain one double or triple bond;

$R_4$ is hydrogen, $C_1$-$C_6$ alkyl, fluoro, chloro, bromo, iodo, $C_1$-$C_4$ alkoxy, amino, $-NHCH_3$, $-N(CH_3)_2$, $-CH_2OH$, $-CH_2OCH_3$, or $-SO_n$($C_1$-$C_4$ alkyl), wherein n is 0, 1, or 2, cyano, hydroxy, -CO($C_1$-$C_4$ alkyl), -CHO, or $-CO_2$ ($C_1$-$C_4$ alkyl), wherein said $C_1$-$C_4$ alkyl group may optionally contain one double or triple bond;

$R_5$ is phenyl, naphthyl, thienyl, benzothienyl, pyridyl, pyrimidyl, benzofuranyl, pyrazinyl, or benzothiazolyl, which may optionally be substituted by from one to three substituents independently selected from fluoro, chloro, $C_1$-$C_6$ alkyl and $C_1$-$C_6$ alkoxy or by one substituent selected from iodo, hydroxy, bromo, formyl, cyano, nitro, amino, trifluoromethyl, -NH($C_1$-$C_4$ alkyl), -N($C_1$-$C_6$)($C_1$-$C_2$ alkyl), $-CO_2$($C_1$-$C_4$ alkyl), -CO($C_1$-$C_4$ alkyl), -COOH, $-SO_2NH$($C_1$-$C_4$ alkyl), $-SO_2N$($C_1$-$C_4$ alkyl)($C_1$-$C_2$ alkyl), $-SO_2NH_2$, $-NHSO_2$($C_1$-$C_4$ alkyl), -S($C_1$-$C_6$ alkyl) and $-SO_2$($C_1$-$C_6$ alkyl), wherein said $C_1$-$C_4$ alkyl and $C_1$-$C_6$ alkyl groups may optionally be substituted by from one to three fluorine atoms;

$R_6$ is hydrogen, $C_1$-$C_4$ alkyl, fluoro, chloro, bromo, iodo, $-CH_2OH$, $-CH_2OCH_3$, or $C_1$-$C_4$ alkoxy;

$R_7$ is hydrogen, $C_1$-$C_4$ alkyl, fluoro, chloro, bromo, iodo, -O($C_1$-$C_4$ alkyl), cyano, $-CH_2OH$, $-CH_2O$($C_1$-$C_2$ alkyl), -CO($C_1$-$C_2$ alkyl), or $-CO_2$($C_1$-$C_2$ alkyl);

$R_{11}$ is hydrogen, hydroxy, fluoro, or methoxy; and

$R_{12}$ is hydrogen or $C_1$-$C_4$ alkyl;

with the proviso that when A is N, then

(a) B is not unsubstituted alkyl;

(b) $R_5$ is not unsubstituted phenyl or monosubstituted phenyl; and

(c) $R_3$ is not unsubstituted alkyl;

or a pharmaceutically acceptable salt thereof.

**6.** A pharmaceutical composition according to Claim 1 wherein said corticotropin-releasing factor antagonist is a compound of formula I, II, or III

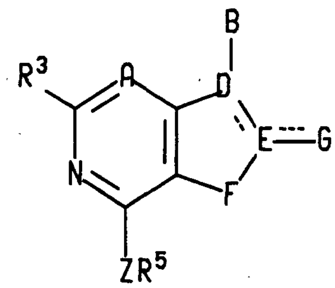

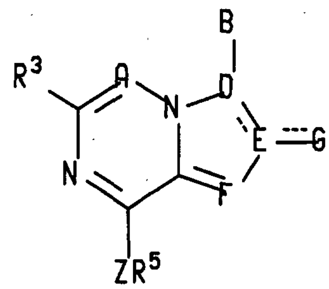

or

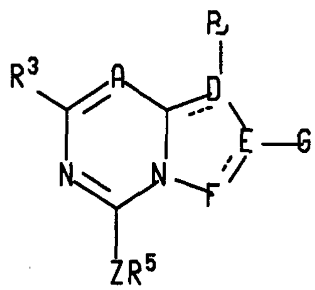

or a pharmaceutically acceptable salt thereof, wherein

the dashed lines represent optional double bonds;

A is nitrogen or $CR^7$;

B is $-NR^1R^2$, $-CR^1R^2R^{10}$, $-C(=CR^2R^{11})R^1$, $-NHCR^1R^2R^{10}$, $-OCR^1R^2R^{10}$, $-SCR^1R^2R^{10}$, $-CR^2R^{10}NHR^1$, $-CR^2R^{10}OR^1$, $-CR^2R^{10}SR^1$, or $-COR^2$;

D is nitrogen and is single bonded to all atoms to which it is attached; or D is carbon and is either double bonded to E in formulas I and II or is double bonded to the adjacent carbon atom common to both fused rings in formula III; or D is CH and is single bonded to E in formulas I and II;

E is nitrogen, CH, or carbon;

F, when single bonded to E, is oxygen, sulfur, $-CHR^4-$, or $-NR^4-$; or F, when double bonded to E, is nitrogen or $CR^4$;

G, when single bonded to E, is hydrogen, $C_1$-$C_4$ alkyl, -S($C_1$-$C_4$ alkyl), -O($C_1$-$C_4$ alkyl), -NH2, -NH($C_1$-$C_4$ alkyl), or -N($C_1$-$C_2$ alkyl)($C_1$-$C_4$ alkyl), wherein said $C_1$-$C_4$ alkyl group may optionally be substituted by one hydroxy, -O($C_1$-$C_2$ alkyl), or fluoro group; or G, when double bonded to E, is oxygen, sulfur, or -NH-; or G, when E is nitrogen and double bonded to D or F, is absent;

$R^1$ is hydrogen, $C_1$-$C_6$ alkyl optionally substituted by one or two substituents $R^8$ independently selected from hydroxy, fluoro, chloro, bromo, iodo, $C_1$-$C_4$ alkoxy, $-CF_3$, $-CO_2(C_1$-$C_4)$alkyl, -OCO($C_1$-$C_4$ alkyl), -OCON($C_1$-$C_4$ alkyl)($C_1$-$C_2$ alkyl), -NHCO($C_1$-$C_4$ alkyl), -COOH, $-CO_2(C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, $-NO_2$, -SO($C_1$-$C_4$ alkyl), $-SO_2(C_1$-$C_4$ alkyl), $-SO_2NH(C_1$-$C_4$ alkyl) and $-SO_2N(C_1$-$C_4$ alkyl)($C_1$-$C_2$ alkyl), wherein said $C_1$-$C_4$ alkyl group may optionally contain one or two double or triple bonds;

$R^2$ is $C_1$-$C_{12}$ alkyl which may optionally contain from one to three double or triple bonds, aryl, or -($C_1$-$C_4$ alkylene)aryl, wherein said aryl group and the aryl moiety of said -($C_1$-$C_4$ alkylene)aryl group are selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; or $C_3$-$C_8$ cycloalkyl or -($C_1$-$C_6$ alkylene)$C_3$-$C_8$ cycloalkyl, wherein one or two of the carbon atoms of said cycloalkyl group and of the 5-to 8-membered cycloalkyl moieties of said -($C_1$-$C_6$ alkylene)$C_3$-$C_8$ cycloalkyl group may optionally and independently be replaced by O, S, or $NZ^2$, wherein $Z^2$ is selected from hydrogen, $C_1$-$C_4$ alkyl, benzyl and $C_1$-$C_4$ alkanoyl; and wherein $R^2$ may optionally be substituted by from one to three substituents independently selected from chloro, fluoro, hydroxy and $C_1$-$C_4$ alkyl or by one substituent selected from bromo, iodo, $C_1$-$C_6$ alkoxy, -OCO($C_1$-$C_6$ alkyl), -OCON($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl)($C_1$-$C_4$ alkyl), -N($C_1$-$C_4$ alkyl)CO($C_1$-$C_4$ alkyl), -NHCO($C_1$-$C_4$ alkyl), -COOH, $-CO_2(C_1$-$C_4$ alkyl), -CONH($C_1$-$C_4$ alkyl), -CON($C_1$-$C_4$ alkyl)($C_1$-$C_2$ alkyl), -SH, -CN, $-NO_2$, -SO($C_1$-$C_4$ alkyl), $-SO_2(C_1$-$C_4$ alkyl), $-SO_2NH(C_1$-$C_4$ alkyl) and $-SO_2N(C_1$-$C_4$ alkyl)($C_1$-$C_2$ alkyl); or

$-NR^1R^2$ or $-CR^1R^2R^{10}$ may form a saturated 3- to 8-membered carbocyclic ring which may optionally contain from one to three double bonds and wherein one or two of the ring carbon atoms of such 5- to 8-membered rings may optionally and independently be replaced by O, S, or $NZ^3$, wherein $Z^3$ is hydrogen, $C_1$-$C_4$ alkyl, benzyl, or $C_1$-$C_4$ alkanoyl;

$R^3$ is hydrogen, $C_1$-$C_4$ alkyl, -O($C_1$-$C_4$ alkyl), chloro, fluoro, bromo, iodo, -CN, -S($C_1$-$C_4$ alkyl), or $-SO_2(C_1$-$C_4$ alkyl), wherein said $C_1$-$C_4$ alkyl group may optionally be substituted with one substituent $R^9$ selected from hydroxy, fluoro and $C_1$-$C_2$ alkoxy;

each $R^4$ is independently selected from hydrogen, $C_1$-$C_6$ alkyl, fluoro, chloro, bromo, iodo, hydroxy, cyano, amino, nitro, -O($C_1$-$C_4$ alkyl), -N($C_1$-$C_4$ alkyl)-($C_1$-$C_2$ alkyl), -S($C_1$-$C_4$ alkyl), -SO($C_1$-$C_4$ alkyl), $-SO_2(C_1$-$C_4)$ alkyl, -CO($C_1$-$C_4$ alkyl), -CHO and $-CO_2(C_1$-$C_4$ alkyl), wherein said $C_1$-$C_6$ alkyl and $C_1$-$C_4$ alkyl groups may optionally contain one or two double or triple bonds and may optionally be substituted by one or two substituents independently selected from hydroxy, amino, $C_1$-$C_3$ alkoxy, dimethylamino, methylamino, ethylamino, $-NHCOCH_3$, fluoro, chloro, $C_1$-$C_3$ thioalkyl, -CN, -COOH, $-CO_2(C_1$-$C_4$ alkyl), -CO($C_1$-$C_4$ alkyl) and $-NO_2$;

$R^5$ is phenyl, naphthyl, thienyl, benzothienyl, pyridyl, quinolyl, pyrazinyl, furanyl, benzofuranyl, benzothiazolyl, benzisothiazolyl, benzisoxazolyl, benzimidazolyl, indolyl, or benzoxazolyl; or $C_3$-$C_8$ cycloalkyl, wherein one or two carbon atoms of those cycloalkyl groups containing at least 5 ring members may optionally and independently be replaced by O, S, or $NZ^4$, wherein $Z^4$ is hydrogen, $C_1$-$C_4$ alkyl, or benzyl; and wherein $R^5$ is substituted by from one to four substituents $R^{12}$, wherein from one to three of said substituents may be selected independently from chloro, $C_1$-$C_6$ alkyl and -O($C_1$-$C_6$ alkyl) and one of said substituents may be selected from bromo, iodo, formyl, -CN, $-CF_3$, $-NO_2$, $-NH_2$, -NH($C_1$-$C_4$ alkyl), -N($C_1$-$C_2$ alkyl)($C_1$-$C_6$ alkyl), $-CO_2(C_1$-$C_4$ alkyl), -CO($C_1$-$C_4$ alkyl), -COOH, $-SO_2NH(C_1$-$C_4$ alkyl), $-SO_2N(C_1$-$C_2$ alkyl)($C_1$-$C_4$ alkyl), $-SO_2NH_2$, $-NHSO_2(C_1$-$C_4$ alkyl), -S($C_1$-$C_6$ alkyl) and $-SO_2(C_1$-$C_6$ alkyi), wherein said $C_1$-$C_4$ alkyl and $C_1$-$C_6$ alkyl groups may optionally be substituted by one or two substituents independently selected from fluoro, hydroxy, amino, methylamino, dimethylamino and acetyl;

$R^7$ is hydrogen, $C_1$-$C_4$ alkyl, halo, cyano, hydroxy, -O($C_1$-$C_4$ alkyl), -CO($C_1$-$C_4$ alkyl), $-CO_2(C_1$-$C_4$ alkyl), $-OCF_3$, $-CF_3$, $-CH_2OH$, or $-CH_2O(C_1$-$C_4$ alkyl);

$R^{10}$ is hydrogen, hydroxy, methoxy, or fluoro;

$R^{11}$ is hydrogen or $C_1$-$C_4$ alkyl; and

Z is -NH-, oxygen, sulfur, -N($C_1$-$C_4$ alkyl), -NCO($C_1$-$C_2$ alkyl), $-NCO_2(C_1$-$C_2$ alkyl), or $-CR^{13}R^{14}$, wherein $R^{13}$ and $R^{14}$ are independently selected from hydrogen, trifluoromethyl and methyl with the exception that one of

$R^{13}$ and $_{R}^{14}$ may be cyano;

with the provisos that

(a) in the five-membered rings of structures I, II and III, there cannot be two double bonds adjacent to each other; and

(b) when $R^4$ is attached to nitrogen, it cannot be halo, cyano, or nitro;

or a pharmaceutically acceptable salt thereof.

**7.** A pharmaceutical composition according to Claim 1 wherein said corticotropin-releasing factor antagonist is a compound of formula I

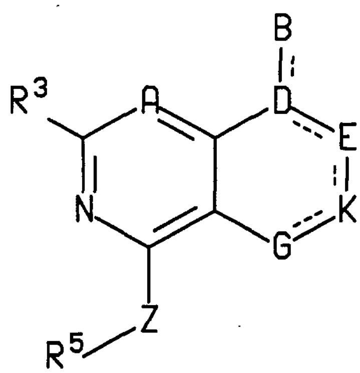

wherein

the dashed lines represent optional double bonds;

A is nitrogen or $CR^7$;

B, when single bonded to D, is $-NR^1R^2$, $-CR^1R^2R^{10}$, $-C(=CR^2R^{11})R^1$, $-NHCR^1R^2R^{10}$, $-OCR^1R^2R^{10}$, $-SCR^1R^2R^{10}$, $-CR^2R^{10}NHR^1$, $-CR^2R^{10}OR^1$, $-CR^2R^{10}SR^1$, or $-COR^2$; or B, when double bonded to D, is $-CR^1R^2$ and D is carbon;

D, when single bonded to all atoms to which it is attached, is nitrogen or $CR^4$; or D, when double bonded to E or double bonded to B, is carbon;

E is oxygen, nitrogen, sulfur, C=O, C=S, $-CR^6R^{12}-$, $-NR^6-$, or $-CR^6-$; or E is a two atom spacer, wherein one of the atoms is oxygen, sulfur, nitrogen, C=O, C=S, $-CR^6R^{12}-$, $-NR^6-$, or $CR_6$ and the other is $-CR^6R^{12}-$ or $CR^9$;

K and G, when single bonded to both adjacent ring atoms, are each independently C=O, C=S, sulfur, oxygen, $-CHR^8-$, or $-NR^8-$; or K and G, when double bonded to an adjacent ring atom, are nitrogen or $CR^8$;

the 6- or 7-membered ring that contains D, E, K and G may contain from one to three double bonds, from zero to two heteroatoms selected from oxygen, sulfur and nitrogen, and from zero to two C=O or C=S groups, wherein the carbon atoms of such groups are part of the ring and the oxygen and sulfur atoms are substituents on the ring;

$R^1$ is $C_1$-$C_6$ alkyl, optionally substituted with one or two substituents independently selected from hydroxy, fluoro, chloro, bromo, iodo, $C_1$-$C_4$ alkoxy, $CF_3$, -CO($C_1$-$C_4$alkyl), $-CO_2$($C_1$-$C_4$)alkyl, -OCO($C_1$-$C_4$ alkyl), -OCON-($C_1$-$C_4$ alkyl)($C_1$-$C_2$ alkyl), -NHCO($C_1$-$C_4$ alkyl), -COOH, $-CO_2$($C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$-($C_1$-$C_4$ alkyl)($C_1$-$C_2$ alkyl), wherein said $C_1$-$C_4$ alkyl group may optionally contain one or two double or triple bonds;

$R^2$ is $C_1$-$C_{12}$ alkyl, which may optionally contain from one to three double or triple bonds, aryl, or -($C_1$-$C_4$ alkylene)aryl, wherein said aryl group and the aryl moiety of said -($C_1$-$C_4$ alkylene)aryl group are selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; or $C_3$-$C_8$ cycloalkyl or -($C_1$-$C_6$ alkylene)$C_3$-$C_8$ cycloalkyl, wherein one or two of the carbon atoms of said cycloalkyl group and of the 5- to 8-membered cycloalkyl moieties of said -($C_1$-$C_6$ alkylene)$C_3$-$C_8$ cycioalkyl group may optionally and independently be replaced by O or S; and wherein $R^2$ may optionally be substituted by from one to three substituents independently selected from chloro, fluoro, hydroxy and $C_1$-$C_4$ alkyl or with one substituent selected from $C_1$-$C_6$ alkoxy, -OCO($C_1$-$C_6$ alkyl), -OCON($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl)($C_1$-$C_4$ alkyl), -N($C_1$-$C_4$ alkyl)CO($C_1$-$C_4$ alkyl), -NHCO-($C_1$-$C_4$ alkyl), -COOH, -$CO_2$($C_1$-$C_4$ alkyl), -CONH($C_1$-$C_4$ alkyl), -CON($C_1$-$C_4$ alkyl)($C_1$-$C_2$ alkyl), -SH, -CN, -$NO_2$, -SO($C_1$-$C_4$ alkyl), -$SO_2$($C_1$-$C_4$ alkyl), -$SO_2$NH($C_1$-$C_4$ alkyl) and -$SO_2$N($C_1$-$C_4$ alkyl)($C_1$-$C_2$ alkyl); or

-$NR^1R^2$ or -$CR^1R^2R^{10}$ may form a ring selected from saturated 3- to 8-membered rings, the 5- to 8-membered rings of which may optionally contain one or two double bonds, wherein one or two of the ring carbon atoms of such 5- to 8-membered rings may optionally and independently be replaced by O, S, or $NZ^3$, wherein $Z^3$ is hydrogen or $C_1$-$C_4$ alkyl;

$R^3$ is hydrogen, $C_1$-$C_4$ alkyl, -O($C_1$-$C_4$ alkyl), chloro, fluoro, bromo, iodo, -S($C_1$-$C_4$ alkyl), or -$SO_2$($C_1$-$C_4$ alkyl);

$R^4$ is hydrogen, $C_1$-$C_2$ alkyl, hydroxy, or fluoro;

each $R^6$, $R^8$ and $R^9$ that is attached to a carbon atom is selected independently from hydrogen, $C_1$-$C_2$ alkyl, fluoro, chloro, bromo, iodo, hydroxy, hydroxymethyl, formyl, trifluoromethyl, cyano, amino, nitro, -O($C_1$-$C_2$ alkyl), -N($C_1$-$C_2$ alkyl)($C_1$-$C_2$ alkyl), -S($C_1$-$C_2$ alkyl), -CO($C_1$-$C_2$ alkyl), -CHO and -$CO_2$($C_1$-$C_2$alkyl), wherein said $C_1$-$C_2$ alkyl group may optionally contain one double or triple bond; and each $R^6$, $R^8$, and $R^9$ that is attached to a nitrogen atom is selected independently from hydrogen and $C_1$-$C_4$ alkyl;

$R^5$ is phenyl, naphthyl, pyridyl, or pyrimidyl substituted by from two to four substituents $R^{15}$, wherein from one to three of said substituents may be selected independently from chloro, $C_1$-$C_6$ alkyl, -O($C_1$-$C_6$ alkyl) and -($C_1$-$C_6$ alkylene)O($C_1$-$C_6$ alkyl) and one of said substituents may be selected independently from bromo, iodo, formyl, cyano, trifluoromethyl, nitro, amino, -NH($C_1$-$C_4$ alkyl), -N($C_1$-$C_2$ alkyl)($C_1$-$C_6$ alkyl), -$CO_2$($C_1$-$C_4$ alkyl), -CO($C_1$-$C_4$ alkyl), -COOH, -$SO_2$NH($C_1$-$C_4$ alkyl), -$SO_2$N($C_1$-$C_2$ alkyl)($C_1$-$C_4$ alkyl), -$SO_2NH_2$, -$NHSO_2$($C_1$-$C_4$ alkyl), -S($C_1$-$C_6$ alkyl) and -$SO_2$($C_1$-$C_6$ alkyl), wherein said $C_1$-$C_4$ alkyl and $C_1$-$C_6$ alkyl groups may optionally be substituted by one or two substituents independently selected from fluoro, hydroxy, amino, methylamino, dimethylamino and acetyl;

$R^7$ is hydrogen, methyl, halo, hydroxy, methoxy, -CO($C_1$-$C_2$ alkyl), -$CO_2$($C_1$-$C_2$ alkyl), trifluoromethoxy, hydroxymethyl, trifluoromethyl, or formyl;

$R^{10}$ is hydrogen, hydroxy, methoxy, or fluoro;

$R^{11}$ is hydrogen or $C_1$-$C_4$ alkyl;

$R^{12}$ is hydrogen or methyl; and

Z is -NH-, oxygen, sulfur, -N($C_1$-$C_4$ alkyl)-, or -$CR^{13}R^{14}$-, wherein $R^{13}$ and $R^{14}$ are independently selected from hydrogen and methyl with the exception that one of $R^{13}$ and $R^{14}$ may optionally be cyano;

with the provisos that

(a) in the 6- or 7-membered rings of structures in formula I, there cannot be two double bonds adjacent to each other; and

(b) when D is carbon and is double bonded to B, then B is $CR^1$ $R^2$;

or a pharmaceutically acceptable salt thereof.

**8.** A pharmaceutical composition according to Claim 1 wherein said corticotropin-releasing factor antagonist is a compound of formula I

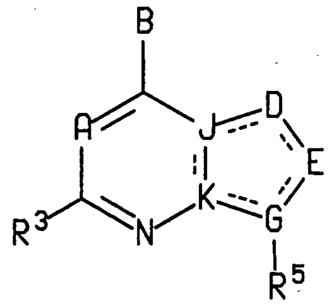

or a pharmaceutically acceptable salt thereof, wherein

the dashed lines represent optional double bonds;

A is nitrogen or $CR^7$;

B is $-NR^1R^2$, $-CR^1R^2R^{10}$, $-C(=CR^2R^{11})R^1$, $-NHCR^1R^2R^{10}$, $-OCR^1R^2R^{10}$, $-SCR^1R^2R^{10}$ $-CR^2R^{10}NHR^1$, $-CR^2R^{10}OR^1$, $-CR^2R^{10}SR^1$, or $-COR^2$;

J and K are each independently nitrogen or carbon, provided both cannot be nitrogens;

D and E are each selected independently from nitrogen, $CR^4$, C=O, C=S, oxygen, sulfur, $-CR^4R^6-$ and $-NR^8-$;

G is nitrogen or carbon;

the ring containing D, E, G, K, and J in formula I may be a saturated or unsaturated 5-membered ring, may optionally contain one or two double bonds, may optionally contain from one to three heteroatoms in the ring, and may optionally have one or two C=O or C=S groups;

$R^1$ is $C_1$-$C_6$ alkyl optionally substituted with one or two substituents independently selected from hydroxy, fluoro, chloro, bromo, iodo, -O($C_1$-$C_4$ alkyl), $-CF_3$, $-CO_2$($C_1$-$C_4$alkyl), -OCO($C_1$-$C_4$ alkyl), -OCON($C_1$-$C_4$ alkyl)($C_1$-$C_2$ alkyi), -NHCO($C_1$-$C_4$ alkyl), -COOH, $-CO_2$($C_1$-$C_4$ alkyl), -CONH($C_1$-$C_4$ alkyl), -CON($C_1$-$C_4$ alkyl)($C_1$-$C_2$ alkyl), -S($C_1$-$C_4$ alkyl), -CN, -N02, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl), wherein said $C_1$-$C_4$ alkyl group may optionally contain one or two double or triple bonds;

$R^2$ is $C_1$-$C_{12}$ alkyl, which may optionally contain from one to three double or triple bonds, aryl, or -($C_1$-$C_4$ alkylene)aryl, wherein said aryl group and the aryl moiety of said -($C_1$-$C_4$ alkylene)aryl group are selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; or $C_3$-$C_8$ cycloalkyl or -($C_1$-$C_6$ alkylene)$C_3$-$C_8$ cycloalkyl, wherein one or two of the carbon atoms of said cycloalkyl group and of the 5- to 8-membered cycloalkyl moieties of said -($C_1$-$C_6$ alkylene)$C_3$-$C_8$ cycloalkyl group may optionally and independently be replaced by O, S, or $NZ^2$, wherein $Z^2$ is selected from hydrogen, $C_1$-$C_4$ alkyl, benzyl and $C_1$-$C_4$ alkanoyl; and wherein $R^2$ may optionally be substituted by from one to three substituents independently selected from chloro, fluoro, hydroxy and $C_1$-$C_4$ alkyl or by one substituent selected from bromo, iodo, $C_1$-$C_6$ alkoxy, -OCO($C_1$-$C_6$ alkyl), -OCON($C_1$-$C_4$ alkyl)($C_1$-$C_2$ alkyl), -S($C_1$-$C_6$ alkyl), amino, -NH($C_1$-$C_2$ alkyl), -N($C_1$-$C_2$ alkyl)($C_1$-$C_4$ alkyl), -N($C_1$-$C_4$alkyl)-CO($C_1$-$C_4$ alkyl), -NHCO($C_1$-$C_4$ alkyl), -COOH, $-CO_2$($C_1$-$C_4$ alkyl), -CONH-($C_1$-$C_4$ alkyl), -CON($C_1$-$C_4$ alkyl)($C_1$-$C_2$ alkyl), -SH, -CN, $-NO_2$, -SO($C_1$-$C_4$ alkyl), $-SO_2$($C_1$-$C_4$ alkyl), $-SO_2NH$($C_1$-$C_4$ alkyl) and $-SO_2N$($C_1$-$C_4$ alkyl)($C_1$-$C_2$ alkyl); or

$-NR^1R^2$ or $-CR^1R^2R^{10}$ may form a saturated 3- to 8-membered carbocyclic ring which may optionally contain from one to three double bonds and wherein one or two of the ring carbon atoms of such 5- to 8-membered rings may optionally and independently be replaced by O, S, or $NZ^3$, wherein $Z^3$ is hydrogen, $C_1$-$C_4$ alkyl,

benzyl, or $C_1$-$C_4$ alkanoyl;

$R^3$ is hydrogen, $C_1$-$C_4$ alkyl, -O($C_1$-$C_4$ alkyl), chloro, fluoro, bromo, iodo, ($C_1$-$C_2$ alkylene)O($C_1$-$C_2$ alkyl), -($C_1$-$C_2$ alkylene)OH, or -S($C_1$-$C_4$ alkyl);

each $R^4$ is independently hydrogen, $C_1$-$C_6$ alkyl, fluoro, chloro, bromo, iodo, hydroxy, cyano, amino, -($C_1$-$C_2$ alkylene)OH, -$CF_3$, -$CH_2SCH_3$, nitro, -O($C_1$-$C_4$ alkyl), -N($C_1$-$C_4$ alkyl)($C_1$-$C_2$ alkyl), -S($C_1$-$C_4$ alkyl), -CO($C_1$-$C_4$ alkyl), -CHO, or -$CO_2$($C_1$-$C_4$ alkyl);

$R^6$ is hydrogen, methyl, or ethyl;

$R^8$ is hydrogen or $C_1$-$C_4$ alkyl;

$R^5$ is phenyl, pyridyl, pyrazinyl, pyrimidyl, or pyridazinyl, wherein each of the foregoing $R^5$ groups is substituted with from one to four substituents $R^{13}$, wherein from one to three of said substituents may be selected independently from fluoro, chloro, $C_1$-$C_6$ alkyl and -O($C_1$-$C_6$ alkyl) and one of said substituents may be selected from bromo, iodo, formyl, -OH, -($C_1$-$C_4$ alkylene)OH, -($C_1$-$C_4$ alkylene)O($C_1$-$C_2$ alkyl), -CN, -$CF_3$, -$NO_2$, -NH2, -NH($C_1$-$C_4$ alkyl), -N($C_1$-$C_2$ alkyl)($C_1$-$C_6$ alkyl), -OCO($C_1$-$C_4$ alkyl), -($C_1$-$C_4$ alkylene)O($C_1$-$C_4$ alkyl), -S($C_1$-$C_6$ alkyl), -($C_1$-$C_4$ alkylene)S($C_1$-$C_4$ alkyl), -$CO_2$($C_1$-$C_4$ alkyl), -CO($C_1$-$C_4$ alkyl), -COOH, -$SO_2$NH($C_1$-$C_4$ alkyl), -S02N-($C_1$-$C_2$ alkyl)($C_1$-$C_4$ alkyl), -$SO_2NH_2$, -$NHSO_2$($C_1$-$C_4$ alkyl), -S($C_1$-$C_6$ alkyl) and -$SO_2$($C_1$-$C_6$ alkyl), wherein said $C_1$-$C_4$ alkyl and $C_1$-$C_6$ alkyl groups may optionally contain one or two double bonds;

$R^7$ is hydrogen, $C_1$-$C_4$ alkyl, halo (e.g. chloro, fluoro, iodo, or bromo), hydroxy, -O($C_1$-$C_4$ alkyl), -CO($C_1$-$C_4$ alkyl), -$CO_2$($C_1$-$C_4$ alkyl), -$OCF_3$, -$CF_3$, -CH20H, or -$CH_2O$($C_1$-$C_2$ alkyl);

$R^{10}$ is hydrogen, hydroxy, methoxy, or fluoro; and

$R^{11}$ is hydrogen or $C_1$-$C_4$ alkyl;

with the provisos that

(a) when both J and K are carbons, D is $CR^4$ and E is nitrogen, then G cannot be nitrogen;

(b) when both J and K are carbons and both D and G are nitrogens, then E cannot be $CR^4$, C=O, or C=S;

(c) when both J and K are carbons and both D and E are carbons, then G cannot be nitrogen;

(d) when G is carbon, it must be double bonded to E; and

(e) in the ring containing J, K, D, E and G, there cannot be two double bonds adjacent to each other;

or a pharmaceutically acceptable salt thereof.

**9.** A pharmaceutical composition according to Claim 1 wherein said corticotropin-releasing factor antagonist is a compound of formula I

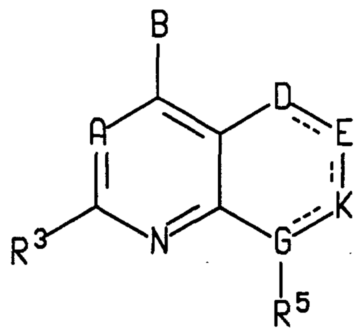

wherein

the dashed lines represent optional double bonds;

A is nitrogen or $CR^7$;

B is $-NR^1R^2$, $-CR^1R^2R^{10}$, $-C(=CR^2R^{11})R^1$, $-NHCR^1R^2R^{10}$, $-OCR^1R^2R^{10}$, $-SCR^1R^2R^{10}$, $-CR^2R^{10}NHR^1$, $-CR^2R^{10}OR^1$, $-CR^2R^{10}SR^1$, or $-COR^2$;

G, when single bonded to all atoms to which it is attached, is nitrogen or $CR^4$; or G, when double bonded to K, is carbon;

K, when double bonded to G or E, is nitrogen or $CR^6$; or K, when single bonded to both adjacent ring atoms, is oxygen, sulfur, C=O, C=S, $-CR^6R^{12}-$, or $-NR^8-$; or K is a two atom spacer, wherein one of the two ring atoms of the spacer is oxygen, sulfur, nitrogen, C=O, C=S, $-CR^6R^{12}-$, $-NR^6-$, or $CR^6$ and the other is $-CR^6R^{12}-$ or $CR^9$;

D and E, when single bonded to both adjacent ring atoms, are each independently C=O, C=S, oxygen, sulfur, $-CR^4R^6-$, or $-NR^8-$; or D and E, when double bonded to an adjacent ring atom, are nitrogen or $CR^4$;

the 6- or 7-membered ring that contains D, E, K and G may contain from one to three double bonds, from zero to two heteroatoms selected from oxygen, sulfur and nitrogen, and from zero to two C=O or C=S groups, wherein the carbon atoms of such groups are part of the ring and the oxygen and sulfur atoms are substituents on the ring;

$R^1$ is $C_1$-$C_6$ alkyl optionally substituted by one or two substituents independently selected from hydroxy, fluoro, chloro, bromo, iodo, $C_1$-$C_4$ alkoxy, $-CF_3$, $-CO(C_1$-$C_4$alkyl), $-CO_2(C_1$-$C_4$)alkyl, $-OCO(C_1$-$C_4$ alkyl), $-OCON-(C_1$-$C_4$ alkyl)($C_1$-$C_2$ alkyl), $-NHCO(C_1$-$C_4$ alkyl), -COOH, $-CO_2(C_1$-$C_4$ alkyl), $-CONH(C_1$-$C_4$ alkyl), $-CON(C_1$-$C_4$ alkyl)($C_1$-$C_2$ alkyl), $-S(C_1$-$C_4$ alkyl), -CN, $-NO_2$, $-SO(C_1$-$C_4$ alkyl), $-SO_2(C_1$-$C_4$ alkyl), $-SO_2NH(C_1$-$C_4$ alkyl) and -S02N-($C_1$-$C_4$ alkyl)($C_1$-$C_2$ alkyl), wherein said $C_1$-$C_4$ alkyl group may optionally contain one or two double or triple bonds;

$R^2$ is $C_1$-$C_{12}$ alkyl, which may optionally contain from one to three double or triple bonds, aryl, or -($C_1$-$C_4$ alkylene)aryl, wherein said aryl group and the aryl moiety of said -($C_1$-$C_4$ alkylene)aryl group are selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; or $C_3$-$C_8$ cycloalkyl or -($C_1$-$C_6$ alkylenyl)$C_3$-$C_8$ cycloalkyl, wherein one or two of the carbon atoms of said cycloalkyl group and of the 5- to 8-membered cycloalkyl moieties of said -($C_1$-$C_6$ alkylenyl)$C_3$-$C_8$ cycloalkyl group may optionally and independently be replaced by O, S, or NZ, wherein Z is hydrogen, $C_1$-$C_4$ alkyl, or benzyl; and wherein $R^2$ may optionally be substituted by from one to three substituents independently selected from chloro, fluoro, hydroxy and $C_1$-$C_4$ alkyl or with one substituent selected from $C_1$-$C_6$ alkoxy, $-OCO(C_1$-$C_6$ alkyl), $-OCON(C_1$-$C_4$ alkyl)($C_1$-$C_2$ alkyl), $-S(C_1$-$C_6$ alkyl), amino, $-NH(C_1$-$C_2$ alkyl), $-N(C_1$-$C_2$ alkyl)-($C_1$-$C_4$ alkyl), -N($C_1$-$C_4$ alkyl)CO($C_1$-$C_4$ alkyl), $-NHCO(C_1$-$C_4$ alkyl), -COOH, $-CO_2(C_1$-$C_4$ alkyl), $-CONH(C_1$-$C_4$ alkyl), -CON($C_1$-$C_4$ alkyl)($C_1$-$C_2$ alkyl), -SH, -CN, -N02, $-SO(C_1$-$C_4$ alkyl), $-SO_2(C_1$-$C_4$ alkyl), $-SO_2NH(C_1$-$C_4$ alkyl) and -$SO_2N(C_1$-$C_4$ alkyl)($C_1$-$C_2$ alkyl); or

$-NR^1R^2$ or $-CR^1R^2R^{10}$ may form a ring selected from saturated 3- to 8-membered rings, the 5- to 8-membered rings of which may optionally contain one or two double bonds and wherein one or two of the ring carbon atoms of such 5- to 8-membered rings may optionally and independently be replaced by O, S, or $NZ^2$, wherein $Z^2$ is hydrogen, benzyl, or $C_1$-$C_4$ alkyl;

$R^3$ is hydrogen, $C_1$-$C_4$ alkyl, -O($C_1$-$C_4$ alkyl), chloro, fluoro, bromo, iodo, -S($C_1$-$C_4$ alkyl), or -$SO_2$($C_1$-$C_4$ alkyl);

each $R^8$, $R^9$ and $R^{12}$ is selected independently from hydrogen and $C_1$-$C_2$ alkyl;

each $R^4$ and $R^6$, when attached to a carbon atom, is selected independently from hydrogen, $C_1$-$C_6$ alkyl, fluoro, chloro, bromo, iodo, hydroxy, -($C_1$-$C_2$ alkyl)OH, trifluoromethyl, cyano, amino, nitro, -O($C_1$-$C_4$ alkyl), -N($C_1$-$C_4$ alkyl)($C_1$-$C_2$ alkyl), -$CH_2SCH_3$, -S($C_1$-$C_4$ alkyl), -CO($C_1$-$C_4$ alkyl), -CHO and -$CO_2$($C_1$-$C_4$ alkyl), wherein said $C_1$-$C_2$ alkyl group may optionally contain one double or triple bond; or $R^6$, when attached to a nitrogen atom, is selected from hydrogen and $C_1$-$C_4$ alkyl;

$R^5$ is phenyl, naphthyl, pyridyl, or pyrimidyl substituted with from two to four substituents $R^{13}$, wherein from one to three of said substituents may be selected independently from chloro, $C_1$-$C_6$ alkyl, -O($C_1$-$C_6$ alkyl) and -($C_1$-$C_6$ alkylene)O($C_1$-$C_6$alkyl) and one of said substituents may be selected independently from bromo, iodo, formyl, cyano, trifluoromethyl, nitro, amino, -NH($C_1$-$C_4$ alkyl), -N($C_1$-$C_2$ alkyl)($C_1$-$C_6$ alkyl), -$CO_2$($C_1$-$C_4$ alkyl), -CO($C_1$-$C_4$ alkyl), -COOH, -$SO_2$NH($C_1$-$C_4$ alkyl), -$SO_2$N($C_1$-$C_2$ alkyl)($C_1$-$C_4$ alkyl), -$SO_2NH_2$, -$NHSO_2$($C_1$-$C_4$ alkyl), -($C_0$-$C_1$ alkylene)S($C_1$-$C_2$ alkyl), -($C_0$-$C_1$ alkylene)SO($C_1$-$C_2$ alkyl), -($C_0$-$C_1$ alkylene)$SO_2$-($C_1$-$C_2$ alkyl) and -($C_1$-$C_4$ alkylene)OH, wherein said $C_1$-$C_4$ alkyl and $C_1$-$C_6$ alkyl groups may optionally be substituted by one or two substituents independently selected from fluoro, hydroxy, amino, methylamino, dimethylamino and acetyl;

$R^7$ is hydrogen, methyl, halo (e.g. chloro, fluoro, iodo, or bromo), hydroxy, methoxy, -CO($C_1$-$C_2$ alkyl), -$CO_2$($C_1$-$C_2$ alkyl), hydroxymethyl, trifluoromethyl, or formyl;

$R^{10}$ is hydrogen, hydroxy, methoxy, or fluoro; and

$R^{11}$ is hydrogen or $C_1$-$C_4$ alkyl;

with the proviso that in the ring containing D, E, K and G of formula I, there cannot be two double bonds adjacent to each other;

or a pharmaceutically acceptable salt thereof.

**10.** A pharmaceutical composition according to Claim 1 wherein said corticotropin-releasing factor antagonist is a compound selected from the group consisting of

4-(1-ethylpropoxy)-3,6-dimethyl-2-(2,4,6-trimethylphenoxy)pyridine; butyl[2,5-dimethyl-7-(2,4,6-trimethylphenyl)-6,7-dihydro-5H-pyrrolo[2,3-d]pyrimidin-4-yl]ethylamine;
4-butylethylamino-2,5-dimethyl-7-(2,4,6-trimethylphenyl)-5,7-dihydro-pyrrolo[2,3-d]pyrimidin-6-one;
4-(1-ethylpropoxy)-2,5-dimethyl-6-(2,4,6-trimethylphenoxy)pyrimidine; N-butyl-N-ethyl-2,5-dimethyl-N, N-(2,4,6-trimethylphenyl)pyrimidine-4,6-diamine;
4-(1-ethylpropoxy)-3,6-dimethylpyridin-2-yl(2,4,6-trimethylphenyl)amine;
6-ethylpropylamino-2,7-dimethyl-9-(2,4,6-trimethylphenyl)-7,9-dihydropurin-8-one;
3-{(4-methylbenzyl)[3,6-dimethyl-1-(2,4,6-trimethylphenyl)-1H-pyrazolo[3,4-d]pyrimidin-4-yl]amino}propan-1-ol;
diethyl-[6-methyl-3-methylsulfanyl-1-(2,4,6-trichlorophenyl)-1H-pyrazolo[3,4-d]pyrimidin-4-yl]amine;
2-{butyl-[6-methyl-3-methylsulfanyl-1-(2,4,6-trichlorophenyl)-1H-pyrazolo[3,4-d]pyrimidin-4-yl]amino}ethanol;
dibutyl-[6-methyl-3-methylsulfanyl-1-(2,4,6-trichlorophenyl)-1H-pyrazolo[3,4-d]pyrimidin-4-yl}amine;
butylethyl[6-methyl-3-methylsulfanyl-1-(2,4,6-trichlorophenyl)-1H-pyrazolo[3,4-d]pyrimidin-4-yl]amine;
butylethyl[6-methyl-3-methylsulfonyl-1-(2,4,6-trichlorophenyl)-1H-pyrazolo[3,4-d]pyrimidin-4-yl]amine;
butylcyclopropylmethyl-[6-methyl-3-methylsulfanyl-1-(2,4,6-trichlorophenyl)-1H-pyrazolo[3,4-d]pyrimidin-4-yl]amine;
di-1-propyl-[6-methyl-3-methylsulfanyl-1-(2,4,6-trichlorophenyl)-1H-pyrazolo[3,4-d]pyrimidin-4-yl]amine;

diallyl-[6-methyl-3-methylsulfanyl-1-(2,4,6-trichlorophenyl)-1H-pyrazolo[3,4-d]pyrimidin-4-yl]amine;
butylethyl-[6-chloro-3-methylsulfanyl-1-(2,4,6-trichlorophenyl)-1H-pyrazolo[3,4-d]pyrimidin-4-yl]amine;
butylethyl-[6-methoxy-3-methylsulfanyl-1-(2,4,6-trichlorophenyl)-1H-pyrazolo[3,4-d]pyrimidin-4-yl]amine;
propylethyl-[3,6-dimethyl-1-(2,4,6-trimethylphenyl)-1H-pyrazolo[3,4-d]pyrimidin-4-yl]amine;
4-(1-ethylpropyl)-6-methyl-3-methylsulfanyl-1-(2,4,6-trimethylphenyl)-1H-pyrazolo[3,4-d]pyrimidine;
n-butylethyl-[2,5-dimethyl-7-(2,4,6-trimethylphenyl)-7H-pyrrolo[2,3-d]pyrimidin-4-yl]amine;
di-n-propyl-[2,5-dimethyl-7-(2,4,6-trimethylphenyl)-7H-pyrrolo[2,3-d]pyrimidin-4-yl]amine;
ethyl-n-propyl-[2,5-dimethyl-7-(2,4,6-trimethylphenyl)-7H-pyrrolo[2,3-d]pyrimidin-4-yl]amine;
diethyl-2,5-dimethyl-7-(2,4,6-trimethylphenyl)-7H-pyrrolo[2,3-d]pyrimidin-4-yl]amine;
n-butylethyl[2,5,6-trimethyl-7-(2,4,6-trimethylphenyl)-7H-pyrrolo[2,3-d]pyrimidin-4-yl]amine;
2-{N-n-butyl-N-[2,5-dimethyl-7-(2,4,6-trimethylphenyl)-7H-pyrrolo[2,3-d]pyrimidin-4-yl]amino}ethanol;
4-(1-ethylpropyl)-2,5,6-trimethyl-7-(2,4,6-trimethylphenyl)-7H-pyrrolo[2,3-d]pyrimidine;
n-butylethyl[2,5-dimethyl-7-(2,4-dimethylphenyl)-7H-pyrrolo[2,3-d]pyrimidin-4-yl]amine;
2,5-dimethyl-7-(2,4,6-trimethylphenyl)-7H-pyrrolo[2,3-d]pyrimidyl-4-yl]-( 1-ethylpropyl)amine;
butyl-[3,6-dimethyl-1-(2,4,6-trimethylphenyl)-1H-pyrazolo[3,4-b]pyridin-4-yl]-ethylamine;
3,6-dimethyl-1-(2,4,6-trimethylphenyl)-1H-pyrazolo[3,4,b]pyridin-4-yl(1-methoxymethylpropyl)amine;
4-(1-methoxymethylpropoxy)-3,6-dimethyl-1-(2,4,6-trimethylphenyl)-1H-pyrazolo[3,4-b]pyridine;
1-ethylpropyl[3,5,6-trimethyl-1-(2,4,6-trimethylphenyl)-1H-pyrazolo[3,4-b]pyridin-4-yl]amine;
4-(1-ethylpropoxy)-2,5-dimethyl-7-(2,4,6-trimethylphenyl)-7H-pyrrolo[2,3-b]pyridine;
4-(1-ethylpropoxy)-2,5,6-trimethyl-7-(2,4,6-trimethylphenyl)-7H-pyrrolo[2,3-b]pyridine;
4-(1-ethylpropoxy)-2,5-dimethyl-7-(2,6-dimethyl-4-bromophenyl)-7H-pyrrolo[2,3-b]pyridine;
2,5,6-trimethyl-7-(1-propylbutyl)-4-(2,4,6-trimethylphenoxy)-7H-pyrrolo[2,3-d]pyrimidine;
1-(1-ethylpropyl)-6-methyl-4-(2,4,6-trimethylphenylamino)-1,3-dihydro-imidazo[4,5-c]pyridin-2-one;
9-(1-ethylpropyl)-2-methyl-6-(2,4,6-trimethylphenylamino)-7,9-dihydro-purin-8-one;
1-(1-ethylpropyl)-6-methyl-4-(2,4,6-trimethylphenoxy)-1,3-dihydro-imidazo[4,5-c]pyridin-2-one;
1-(1-ethylpropyl)-6-methyl-4-(2,4,6-trimethylphenoxy)-1H-imidazo[4,5-c]pyridine;
1-(1-ethylpropyl)-3,6-dimethyl-4-(2,4,6-trimethylphenoxy)-1,3-dihydro-imidazo[4,5-c]pyridin-2-one;
1-(1-ethylpropyl)-3,6-dimethyl-4-(2,4,6-trimethylphenylamino)-1,3-dihydro-imidazo[4,5-c]pyridin-2-one;
1-(1-ethylpropyl)-4,7-dimethyl-5-(2,4,6-trimethylphenoxy)-1,4-dihydro-2H-pyrido[3,4-b]pyrazin-3-one;
1-(1-ethylpropyl)-4,7-dimethyl-5-(2,4,6-trimethylphenoxy)-1,2,3,4-tetrahydro-pyrido[3,4-b]pyrazine;
1-(1-ethylpropyl)-7-methyl-5-(2,4,6-trimethylphenoxy)-1,2,3,4-tetrahydropyrido[3,4-b]pyrazine;
1-(1-ethylpropyl)-7-methyl-2-oxo-5-(2,4,6-trimethylphenoxy)-1,2,3,4-tetrahydro[1,6]naphthyridine-3-carboxylic acid, methyl ester;
1-(1-ethylpropyl)-7-methyl-2-oxo-5-(2,4,6-trimethylphenoxy)-1,2,3,4-tetrahydro[1,6]naphthyridine-3-carboxylic acid, isopropyl ester;
1-(1-ethylpropyl)-7-methyl-5-(2,4,6-trimethylphenoxy)-3,4-dihydro-1H-[1,6]naphthyridin-2-one;
1-(1-ethylpropyl)-7-methyl-5-(2,4,6-trimethylphenoxy)-1,2,3,4-tetrahydro-[1,6]naphthyridine;
1-(1-ethylpropyl)-7-methyl-5-(2,4,6-trimethylphenoxy)-1,4-dihydro-2H-3-oxa-1,6-diazanaphthalene;
1-(1-ethylpropyl)-4,7-dimethyl-5-(2,4,6-trimethylphenoxy)-1,4-dihydro-2H-3-oxa-1,6-diazanaphthalene;
1-(1-ethylpropyl)-3,7-dimethyl-5-(2,4,6-trimethylphenoxy)-3,4-dihydro-1H-3-oxa[1,6]naphthyridin-2-one;
1-(1-ethylpropyl)-3,3,6-trimethyl-4-(2,4,6-trimethylphenoxy)-2,3-dihydro-1H-pyrrolo[3,2-c]pyridine;
7-(1-ethylpropoxy)-5-methyl-3-(2,4,6-trimethylphenyl)pyrazolo[1,5-a]pyrimidine;
2,5-dimethyl-3-(2,4,6-trimethylphenyl)-pyrazolo[1,5-a]pyrimidin-7-yl(1-ethyl-propyl)amine;
1-ethylpropyl[5-methyl-3-(2,4,6-trimethyl-phenyl)-pyrazolo[1,5-a]pyrimidin-7-yl]amine;
7-(1-ethylpropoxy)-2,5-dimethyl-3-(2,4,6-trimethylphenyl)pyrazolo[1,5-a]pyrimidine;
[2,5-dimethyl-3-(2,4,6-trimethylphenyl)pyrazolo[1,5-a]pyrimidin-7-yl]ethyl-propylamine;
[6-bromo-5-bromomethyl-3-(2,4,6-trimethylphenyl)-3H-[1,2,3]triazolo[4,5-b]pyridin-7-yl]-1-ethylpropylamine;
1-ethylpropyl[5-methyl-3-(2,4,6-trimethylphenyl)-3H-[1,2,3]triazolo[4,5-b]pyridin-7-yl]amine;
[6-bromo-5-methyl-3-(2,4,6-trimethylphenyl)-3H-[1,2,3]triazolo[4,5-b]pyridin-7-yl]-1-ethylpropylmethylamine;
7-(1-ethylpropoxy)-5-methyl-3-(2,4,6-trimethylphenyl)-3H-[1,2,3]triazolo[4,5-b]pyridine;
4-(1-ethylpropoxy)-2,5-dimethyl-7-(2,4,6-trimethylphenyl)-5H-pyrrolo[3,2-d]pyrimidine;
(±)-2,5-dimethyl-4-(tetrahydrofuran-3-yloxy)-7-(2,4,6-trimethylphenyl)-5H-pyrrolo[3,2-d]pyrimidine;
2,5-dimethyl-4-(S)-(tetrahydrofuran-3-yloxy)-7-(2,4,6-trimethylphenyl)-5H-pyrrolo[3,2-d]pyrimidine;
2,5-dimethyl-4-(1-propylbutoxy)-7-(2,4,6-trimethylphenyl)-5H-pyrrolo[3,2-d]pyrimidine;
4-sec-butylsulfanyl-2,5-dimethyl-7-(2,4,6-trimethylphenyl)-5H-pyrrolo[3,2-d]pyrimidine;
4-butylethylamino-2,6-dimethyl-8-(2,4,6-trimethylphenyl)-5,8-dihydro-6H-pyrido[2,3-d]pyrimidin-7-one;
8-(1-ethylpropoxy)-6-methyl-4-(2,4,6-trimethylphenyl)-3,4-dihydro-1H-pyrido[2,3-b]pyrazin-2-one;
8-(1-ethylpropoxy)-6-methyl-4-(2,4,6-trimethylphenyl)-1,2,3,4-tetrahydro-pyrido[2,3-b]pyrazine;

4-(1-ethylpropoxy)-2-methyl-8-(2,4,6-trimethylphenyl)quinoline;
5-(1-ethylpropoxy)-7-methyl-1-(2,4,6-trimethylphenyl)-1,4-dihydro-2H-3-oxa-1,8-diazanaphthalene;
5-(1-ethylpropoxy)-7-methyl-1-(2,4,6-trimethyl-phenyl)-1,2-dihydro-3-oxa-1,8--diazanaphthalen-4-one;
8-(1-ethylpropoxy)-1,6-dimethyl-4-(2,4,6-trimethyl-phenyl)-1,2,3,4-tetrahydropyrido[2,3-b]pyrazine;
1-ethylpropyl[2-methyl-8-(2,4, 6-trimethylphenyl)quinolin-4-yl]amine;
4-butylethylamino-2,6-dimethyl-8-(2,6-dimethyl-4-bromophenyl)-5,8-dihydro-6H-pyrido[2,3-d]pyrimidin-7-one;
4-butylethylamino-2-methyl-8-(2,6-dimethyl-4-bromophenyl)-5,8-dihydro-6H-pyrido[2,3-d]pyrimidin-7-one;
4-(1-ethylpropoxy)-2-methyl-8-(2,6-dimethyl-4-bromophenyl)-5,8-dihydro-6H-pyrido[2,3-d]pyrimidin-7-one;
butylethyl[2-methyl-8-(2,6-dimethyl-4-bromophenyl)-5,6,7,8-tetrahydro-pyrido[2,3-d]pyrimidin-4-yl]amine;
propylethyl[2-methyl-8-(2,6-dimethyl-4-bromophenyl)-5,6,7,8-tetrahydro-pyrido[2,3-d]pyrimidin-4-yl]amine;
diethyl[2-methyl-8-(2,6-dimethyl-4-bromophenyl)-5,6,7,8-tetrahydro-pyrido[2,3-d]pyrimidin-4-yl]amine;
1-ethylpropyl[2-methyl-8-(2,6-dimethyl-4-bromophenyl)-5,6,7,8-tetrahydropyrido[2,3-d]pyrimidin-4-yl]amine;
1-ethylpropoxy-2-methyl-8-(2,6-dimethyl-4-bromophenyl)-5, 6, 7, 8-tetrahydropyrido[2,3-d]pyrimidine;
4-(butylethylamino)-2-methyl-8-(2,4,6-trimethylphenyl)-5,8-dihydro-6H-pyrido[2,3-d]pyrimidin-7-one;
4-(1-ethylpropoxy)-2-methyl-8-(2,4,6-trimethylphenyl)-5,8-dihydro-6H-pyrido[2,3-d]pyrimidin-7-one;
butylethyl[2-methyl-8-(2,4,6-trimethyl-phenyl)-5,6,7,8-tetrahydropyrido[2,3-d]-pyrimidin-4-yl]amine;
propylethyl[2-methyl-8-(2,4,6-trimethylphenyl)-5,6,7,8-tetrahydropyrido-[2,3-d]pyrimidin-4-yl]amine;
diethyl[2-methyl-8-(2,4,6-trimethylphenyl)-5,6,7,8-tetrahydropyrido[2,3-d]-pyrimidin-4-yl]amine;
1-ethylpropyl[2-methyl-8-(2,4,6-trimethylphenyl)-5,6,7, 8-tetrahydro-pyrido[2,3-d]pyrimidin-4-yl]amine;
1-ethylpropoxy-2-methyl-8,(2,4,6-trimethylphenyl)-5,6,7,8-tetrahydro-pyrido[2,3-d]pyrimidine;
8-(1-ethylpropoxy)-6-methyl-4-(2,6-dimethyl-4-bromophenyl)-3,4-dihydro-1H-pyrido[2,3-b]pyrazin-2-one;
8-(1-ethylpropoxy)-6-methyl-4-(2,6-dimethyl-4-bromophenyl)-1,2,3,4-tetrahydropyrido[2,3-b]pyrazine;
4-(1-ethylpropoxy)-2-methyl-8-(2,6-dimethyl-4-bromophenyl)quinoline;
5-(1-ethylpropoxy)-7-methyl-1-(2,6-dimethyl-4-bromophenyl)-1,4-dihydro-2H-3-oxa-1,8-diazanaphthalene;
5-(1-ethylpropoxy)-7-methyl-1-(2,6-dimethyl-4-bromophenyl)-1,2-dihydro-3-oxa-1,8-diazanaphthalen-4-one;
8-(1-ethylpropoxy)-1,6-dimethyl-4-(2,6-dimethyl-4-bromophenyl)-1,2,3,4-tetrahydropyrido[2,3-b]pyrazine;
1-ethylpropyl[2-methyl-8-(2,6-dimethyl-4-bromophenyl)-quinolin-4-yl]-amine;
4-(butylethylamino)-2,6-dimethyl-8-(2,6-dimethyl-4-chlorophenyl)-5,8-dihydro-6H-pyrido[2,3-d]pyrimidin-7-one;
8-(1-ethylpropoxy)-6-methyl-4-(2,6-dimethyl-4-chlorophenyl)-3,4-dihydro-1H-pyrido[2,3-b]pyrazin-2-one;
8-(1-ethylpropoxy)-6-methyl-4-(2,6-dimethyl-4-chlorophenyl)-1,2,3,4-tetrahydropyrido[2,3-b]pyrazine;
4-(1-ethylpropoxy)-2-methyl-8-(2,6-dimethyl-4-chlorophenyl)quinoline;
5-(1-ethylpropoxy)-7-methyl-1-(2,6-dimethyl-4-chlorophenyl)-1,4-dihydro-2H-3-oxa-1,8-diazanaphthalene;
5-(1-ethylpropoxy)-7-methyl-1-(2,6-dimethyl-4-chlorophenyl)-1,2-dihydro-3-oxa-1,8-diazanaphthalen-4-one;
8-(1-ethylpropoxy)-1,6-dimethyl-4-(2,6-dimethyl-4-chlorophenyl)-1,2,3,4-tetrahydropyrido[2,3-b]pyrazine;
1-ethylpropyl[2-methyl-8-(2,6-dimethyl-4-chlorophenyl)quinolin-4-yl]-amine;
8-(1-hydroxymethylpropoxy)-6-methyl-4-(2,4,6-trimethylphenyl)-3,4-dihydro-1 H-pyrido[2,3-b]pyrazin-2-one;
8-(1-hydroxymethylpropylamino)-6-methyl-4-(2,4,6-trimethylphenyl)-3,4-dihydro-1H-pyrido[2,3-b]pyrazin-2-one;
8-(1-ethylpropylamino)-6-methyl-4-(2,4,6-trimethylphenyl)-3,4-dihydro-1H-pyrido[2,3-b]pyrazin-2-one;
8-diethylamino-6-methyl-4-(2,4,6-trimethylphenyl)-3,4-dihydro-1H-pyrido-[2,3-b]pyrazin-2-one;
8-ethylpropylamino-6-methyl-4-(2,4,6-trimethylphenyl)-3,4-dihydro-1H-pyrido[2,3-b]pyrazin-2-one;
8-butylethylamino-6-methyl-4-(2,4, 6-trimethylphenyl)-3,4-dihydro-1H-pyrido[2,3-b]pyrazin-2-one;
8-(1-hydroxymethylpropoxy)-6-methyl-4-(2,4,6-trimethylphenyl)-1,2,3,4-tetrahydropyrido[2,3-b]pyrazine;
8-(1-hydroxymethylpropylamino)-6-methyl-4-(2,4,6-trimethylphenyl)-1,2,3,4-tetrahydropyrido[2,3-b]pyrazine;
8-(1-ethylpropylamino)-6-methyl-4-(2,4,6-trimethylphenyl)-1,2,3,4-tetrahydropyrido[2,3-b]pyrazine;
8-diethylamino-6-methyl-4-(2,4,6-trimethylphenyl)-1,2,3,4-tetrahydro-pyrido[2,3-b]pyrazine;
8-ethylpropylamino-6-methyl-4-(2,4,6-trimethylphenyl)-1,2,3,4-tetrahydro-pyrido[2,3-b]pyrazine;
8-butylethylamino-6-methyl-4-(2,4,6-trimethylphenyl)-1,2,3,4-tetrahydro-pyrido[2,3-b]pyrazine;
4-(1-hydroxymethylpropoxy)-2-methyl-8-(2,4,6-trimethylphenyl)quinoline;
4-(1-hydroxymethylpropylamino)-2-methyl-8-(2,4,6-trimethylphenyl)quinoline;
4-(1-ethyl-propylamino)-2-methyl-8-(2,4,6-trimethylphenyl)quinoline;
4-diethylamino-2-methyl-8-(2,4,6-trimethylphenyl)quinoline;
4-(ethylpropylamino)-2-methyl-8-(2,4,6-trimethylphenyl)quinoline;
4-(butylethylamino)-2-methyl-8-(2,4,6-trimethylphenyl)quinoline;
5-(1-hydroxymethylpropoxy)-7-methyl-1-(2,4,6-trimethylphenyl)-1,4-dihydro-2H-3-oxa-1,8-diazanaphthalene;

5-(1-hydroxymethylpropylamino)-7-methyl-1-(2,4,6-trimethylphenyl)-1,4-dihydro-2H-3-oxa-1,8-diazanaphthalene;
5-(1-ethylpropylamino)-7-methyl-1-(2,4,6-trimethylphenyl)-1,4-dihydro-2H-3-oxa-1,8-diazanaphthalene;
5-diethylamino-5-methyl-1-(2,4,6-trimethylphenyl)-1,4-dihydro-2H-3-oxa-1,8-diazanaphthalene;
5-ethylpropylamino-7-methyl-1-(2,4,6-trimethyl-phenyl)-1,4-dihydro-2H-3-oxa-1,8-diazanaphthalene;
8-butylethylamino-6-methyl-4-(2,4,6-trimethylphenyl)-1,4-dihydro-2H-3-oxa-1,8-diazanaphthalene;
4-(2,4-dichlorophenyl)-5-methyl-2-[N-(1-(methoxymethyl)-1-(naphth-2-yl) methyl)-N-propylamino]thiazole;
oxalate of 4-(2,4-dichlorophenyl)-5-methyl-2-[N-(6-methoxyisoquinol-5-yl)-N-propylamino]thiazole;
oxalate of 4-(2-chloro-4-methoxyphenyl)-5-methyl-2-[N-(6-methylisoquinol-5-yl)-N-propylamino]thiazole;
4-(2-chloro-4-methoxyphenyl)-5-methyl-2-[N-(1-methoxycarbonylmethylindol-5-yl)-N-propylamino]thiazole;
oxalate of 4-(2-chloro-4-methoxyphenyl)-5-methyl-2-[N-(6-methoxyisoquinol-5-yl)-N-propylamino]thiazole;
oxalate of 4-(2-chloro-4-methoxyphenyl)-5-methyl-2-[N-(6-chloroisoquinol-5-yl)-N-propylamino]thiazole;
oxalate of 4-(2-chloro-4-methoxyphenyl)-5-methyl-2-[N-(6-methoxyisoquinol-5-yl)-N-propylamino]thiazole;
4-(2-chloro-4-methoxyphenyl)-5-methyl-2-[N-1-methoxynaphth-2-yl)-N-propylamino]thiazole;
oxalate of 4-(2-chloro-4-trifluoromethylphenyl)-5-methyl-2-[N-6-methoxyisoquinol-5-yl)-N-propylamino]thiazole;
chlorhydrate of 4-(2-chloro-4-methoxyphenyl)-5-methyl-2-[N-(2-ethoxynaphth-1-yl)-N- propylamino]thiazole;
chlorhydrate of 4-(2-chloro-4-methoxyphenyl)-5-methyl-2[N-(2,3-dimethylnaphth-1-yl)-N-propylamino]thiazole;
chlorhydrate of 4-(2-chloro-4-methoxyphenyl)-5-methyl-2-[N-(6-bromo-2-methoxynaphth-1-yl)-N-propylamino]thiazole;
chlorhydrate of 4-(2-chloro-4-methoxyphenyl)-5-methyl-2-[N-(2,6-dimethylnaphth-1-yl)-N-propylamino]thiazole;
chlorhydrate of 4-(2-chloro-4-methoxyphenyl)-5-methyl-2-[N-(1-methoxymethyl-1-(naphth-2-yl)methyl)-N-propylamino]thiazole;
chlorhydrate of 4-(2-chloro-4-methoxyphenyl)-5-methyl-2-[N-(1-(cyclopropyl)-1-(naphth-2-yl)methyl)-N-propylamino]thiazole;
3-(2,4-dichlorophenyl)-5-methyl-7(N-propyl-N-cyclopropanemethylamino)-pyrazolo[2,3-a]pyrimidine;
3-(2,4-dichlorophenyl)-5-methyl-7-(N-allyl-N-cyclopropanemethylamino)-pyrazolo[2,3-a]pyrimidine;
2-methylthio-3-(2,4-dichlorophenyl)-5-methyl-7-(N,N-diallylamino)-pyrazolo[2,3-a]pyrimidine;
2-methylthio-3-(2,4-dichlorophenyl)-5-methyl-7-(N-butyl-N-cyclopropane-methylamino)pyrazolo[2,3-a]pyrimidine;
2-methylthio-3-(2,4-dichlorophenyl)-5-methyl-7-(N-propyl-N-cyclopropane-methylamino)pyrazolo[2,3-a]pyrimidine;
2-methyl-3-(4-chlorophenyl)-5-methyl-7-(N,N-dipropylamino)pyrazolo[2,3-a] pyrimidine;
3-[6-(dimethylamino)-3-pyridinyl-2,5-dimethyl-N,N-dipropylpyrazolo[2,3-a] pyrimidin-7-amine;
3-[6-(dimethylamino)-4-methyl-3-pyridinyl]-2,5-dimethyl-N,N-dipropyl-pyrazolo[2,3-a]pyrimidine-7-amine;
3-(2,4-dimethoxyphenyl)-2,5-dimethyl-7-(N-propyl-N-methyloxyethylamino)-pyrazolo[2,3-a]pyrimidine;
7-(N-diethylamino)-2,5-dimethyl-3-(2-methyl-4-methoxyphenyl-[1,5-a]-pyrazolopyrimidine;
7-(N-(3-cyanopropyl)-N-propylamino-2,5,dimethyl-3-(2,4-dimethylphenyl)-[1,5-a]-pyrazolopyrimidine;
[3,6-dimethyl-2-(2,4,6-trimethylphenoxy)pyridin-4-yl]-1-ethylpropylamine;
[2-(4-chloro-2,6-dimethylphenoxy)-3,6-dimethylpyridin-4-yl]-1-ethylpropylamine;
cyclopropylmethyl[3-(2,4-dimethylphenyl)-2,5-dimethylpyrazolo[1,5-a]pyrimidin-7-yl]propylamine;
cyclopropylmethyl[3-(2-methyl-4-chlorophenyl)-2,5-dimethylpyrazolo[1,5-a]pyrimidin-7-yl]propylamine;
cyclopropylmethyl[3-(2,4-dichlorophenyl)-2,5-dimethylpyrazolo[1,5-a]pyrimidin-7-yl]propylamine;
[3-(2-methyl-4-chlorophenyl)-2,5-dimethylpyrazolo[1,5-a]pyrimidin-7-yl]-dipropylamine;
[2,5-dimethyl-3-(2,4-dimethylphenyl)pyrazolo[1,5-a]pyrimidin-7-yl]-1-ethylpropylamine;
[2,5-dimethyl-3-(2,4-dichlorophenyl)pyrazolo[1,5-a]pyrimidin-7-yl]1-ethylpropylamine;
4-(1-ethylpropylamino)-6-methyl-2-(2,4,6-trimethylphenoxy)nicotinic acid, methyl ester;
3-[6-(dimethylamino)-4-methyl-3-pyridinyl]-2,5-dimethyl-N-propyl-N-cyclopropylmethylpyrazolo[2,3-a]pyrimidin-7-amine; and
3-[6-(dimethylamino)-4-methyl-3-pyridinyl]-2,5-dimethyl-N-ethyl-N-cyclopropylmethyl-pyrazolo[2,3-a]pyrimidin-7-amine.

**11.** A pharmaceutical composition according to Claim 1 wherein said growth hormone secretagogue is a compound of formula IV

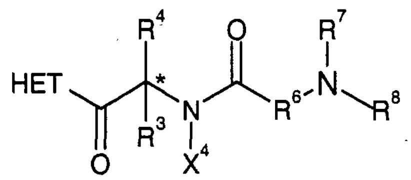

IV

or a stereoisomeric mixture thereof, a diastereomerically enriched, diastereomerically pure, enantiomerically enriched, or enantiomerically pure isomer thereof, or a prodrug of such compound, mixture, or isomer thereof, or a pharmaceutically acceptable salt of the compound, mixture, isomer, or prodrug, wherein
HET is a heterocyclic moiety selected from the group consisting of

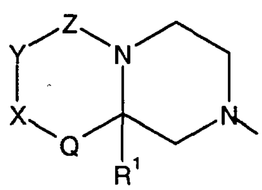
,
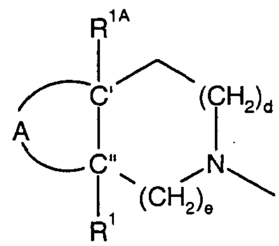
,
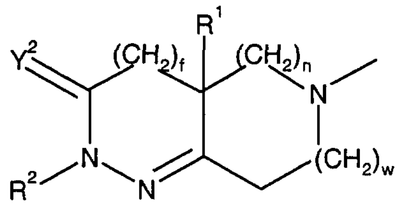
,
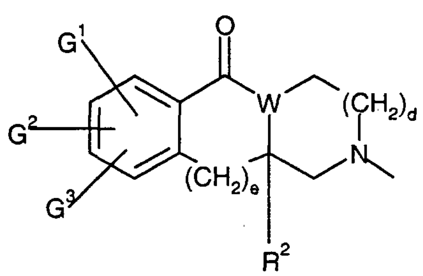
and
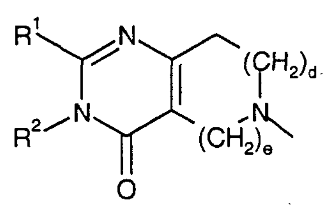

wherein

d is 0, 1, or 2;

e is 1 or 2;

f is 0 or 1;

n and w are 0, 1, or 2, provided both cannot be 0;

$Y^2$ is oxygen or sulfur;

A is a divalent radical, wherein the left hand side of the radical as shown below is connected to C" and the right hand side of the radical as shown below is connected to C', selected from the group consisting of $-NR^2CONR^2-$, $-NR^2SO_2NR^2-$, $-OCONR^2-$, $-NR^2CO_2-$, $-CONR^2CO-$, $-CONR^2C(R^9R^{10})-$, $-C(R^9R^{10})NR^2CO-$, $-C(R^9R^{10})C(R^9R^{10})C(R^9R^{10})-$, $-SO_2C(R^9R^{10})C(R^9R^{10})-$, $-C(R^9R^{10})OCO-$, $-C(R^9R^{10})OC(R^9R^{10})-$, $-NR^2COC(R^9R^{10})-$, $-OCOC(R^9R^{10})-$, $-C(R^9R^{10})CONR^2-$, $-CONR^2CO-$, $-C(R^9R^{10})CO_2-$, $-CONR^2C(R^9R^{10})C(R^9R^{10})-$, $-CO_2C(R^9R^{10})-$, $-C(R^9R^{10})C(R^9R^{10})C(R^9R^{10})C(R^9R^{10})-$, $-SO_2NR^2C(R^9R^{10})-C(R^9R^{10})-$, $-C(R^9R^{10})C(R^9R^{10})NR^2CO-$, $-C(R^9R^{10})C(R^9R^{10})OCO-$, $-NR^2CO-C(R^9R^{10})C(R^9R^{10})-$, $-NR^2SO_2C(R^9R^{10})C(R^9R^{10})-$, $-OCOC(R^9R^{10})C(R^9R^{10})-$, $-C(R^9R^{10})C(R^9R^{10})CONR^2-$, $-C(R^9R^{10})C(R^9R^{10})CO-$, $-C(R^9R^{10})NR^2CO_2-$, $-C(R^9R^{10})OCONR^2-$, $-C(R^9R^{10})NR^2CONR^2-$, $-NR^2CO_2C(R^9R^{10})-$, $-NR^2CONR^2-$, $-C(R^9R^{10})-$, $-NR^2SO_2NR^2C(R^9R^{10})-$, $-OCONR^2C(R^9R^{10})-$, $-CON=C(R^{11})NR^2-$, $-CONR^2C(R^{11})=N-$, $-C(R^9R^{10})NR^{12}C(R^9R^{10})-$, $-NR^{12}C(R^9R^{10})-$, $-NR^{12}C(R^9R^{10})-C(R^9R^{10})-$, $-CO_2C(R^9R^{10})C(R^9R^{10})-$, $-NR^2C(R^{11})=NCO-$, $-C(R^9R^{10})C(R^9R^{10})-N(R^{12})-$, $-C(R^9R^{10})NR^{12}-$, $-N=C(R^{11})NR^2CO-$, $-C(R^9R^{10})C(R^9R^{10})NR^2SO_2-$, $-C(R^9R^{10})C(R^9R^{10})SO_2NR^2-$,$-C(R^9R^{10})C(R^9R^{10})CO_2-$,$-C(R^9R^{10})SO_2C(R^9R^{10})-$, $-C(R^9R^{10})C(R^9R^{10})SO_2-$, $-OC(R^9R^{10})C(R^9R^{10})-$, $-C(R^9R^{10})C(R^9R^{10})O-$, $-C(R^9R^{10})COC(R^9R^{10})-$, $-COC(R^9R^{10})C(R^9R^{10})-$ and $-C(R^9R^{10})NR^2SO_2NR^2-$;

Q is a covalent bond or $-CH_2-$;

W is CH or N;

X is $-CR^9R^{10}-$, C=CH2, or C=O;

Y is $-CR^9R^{10}-$, -O-, or $-NR^2-$;

Z is C=O, C=S, or $SO_2$;

$G^1$ is hydrogen, halo, hydroxy, nitro, amino, cyano, phenyl, carboxyl, $-CONH_2$, $C_1$-$C_4$ alkyl optionally independently substituted by one or more phenyl groups, one or more halo atoms, or one or more hydroxy groups, $C_1$-$C_4$ alkoxy optionally independently substituted by one or more phenyl groups, one or more halo atoms, or one or more hydroxy groups, $C_1$-$C_4$ alkylthio, phenoxy, $-CO_2(C_1$-$C_4$ alkyl), -di($C_1$-$C_4$ alkyl)amino, $C_2$-$C_6$ alkenyl optionally independently substituted by one or more phenyl groups, one or more halo atoms, or one or more hydroxy groups, $C_2$-$C_6$ alkynyl optionally independently substituted by one or more phenyl groups, one or more halo atoms, or one or more hydroxy groups, $C_3$-$C_6$ cycloalkyl optionally independently substituted by one or more $C_1$-$C_4$ alkyl groups, one or more halo atoms, or one or more hydroxy groups, -($C_1$-$C_4$ alkyl)aminocarbonyl, or -di($C_1$-$C_4$ alkyl)aminocarbonyl;

$G^2$ and $G^3$ are each independently selected from the group consisting of hydrogen, halo, hydroxy, $C_1$-$C_4$ alkyl optionally independently substituted by from one to three halo atoms and $C_1$-$C_4$ alkoxy optionally independently substituted by from one to three halo atoms;

$R^1$ is hydrogen, -CN, $(CH_2)_qNX^6COX^6$, $-(CH_2)_qNX^6CO(CH_2)_tA^1$, $-(CH_2)_qNX^6SO_2(CH_2)_tA^1$, $-(CH_2)_qNX^6SO_2X^6$, $(CH_2)_qNX^6CONX^6(CH_2)_tA^1$, $-(CH_2)_qNX^6CONX^6X^6$, $-(CH_2)_qCONX^6X^6$, $-(CH_2)_qCONX^6(CH_2)_tA^1$, $-(CH_2)_qCO_2X^6$, $-(CH_2)_qCO_2(CH_2)_tA^1$, $-(CH_2)_qOX^6$, $-(CH_2)_qOCOX^6$, $-(CH_2)_qOCO(CH_2)_tA^1$, $-(CH_2)_qOCONX^6(CH_2)_tA^1$, $-(CH_2)_qOCONX^6X^6$, $-(CH_2)_qCOX^6$, $-(CH_2)_qCO(CH_2)_tA^1$, $-(CH_2)_qNX^6CO_2X^6$, $-(CH_2)_qNX^6SO_2NX^6X^6$, $-(CH_2)_qSO_mX^6$, $-(CH_2)_qSO_m(CH_2)_tA^1$, $C_1$-$C_{10}$ alkyl, $-(CH_2)_tA^1$, $-(CH_2)_q(C_3$-$C_7$ cycloalkyl), $-(CH_2)_qY^1(C_1$-$C_6$ alkyl), $(CH_2)_qY^1(CH_2)_tA^1$, or $-(CH_2)_qY^1(CH_2)_t(C_3$-$C_7$ cycloalkyl), wherein said alkyl and cycloalkyl groups are optionally substituted by $C_1$-$C_4$ alkyl, hydroxy, $C_1$-$C_4$ alkoxy, carboxyl, $-CONH_2$, $-SO_m(C_1$-$C_6$ alkyl), $-CO_2(C_1$-$C_4$ alkyl), 1H-tetrazol-5-yl, or from one to three fluorine atoms;

$Y^1$ is -O-, $-SO_m-$, $-CONX^6-$, -CH=CH-, -C≡C-, $-NX^6CO-$, $-CONX^6-$, $-CO_2-$, - $OCONX^6-$, or -OCO-;

q is 0, 1, 2, 3, or 4;

t is 0, 1, 2, or 3;

said -(CH2)q- group and $-(CH_2)_t-$ group in the definition of $R^1$ are optionally independently substituted with hydroxy, $C_1$-$C_4$ alkoxy, carboxyl, $-CONH_2$, $-SO_m(C_1$-$C_6$ alkyl), $-CO_2(C_1$-$C_4$ alkyl) ester, 1H-tetrazol-5-yl, from

one to three fluorine atoms, or one or two $C_1$-$C_4$ alkyl groups;

$R^{1A}$ is selected from the group consisting of hydrogen, F, Cl, Br, I, $C_1$-$C_6$ alkyl, phenyl($C_1$-$C_3$ alkyl)-, pyridyl ($C_1$-$C_3$ alkyl)-, thiazolyl($C_1$-$C_3$ alkyl)- and thienyl-($C_1$-$C_3$ alkyl)-, provided that $R^{1A}$ is not F, Cl, Br, or I when a heteroatom is vicinal to C";

$R^2$ is hydrogen, $C_1$-$C_8$ alkyl, -($C_0$-$C_3$ alkyl)$C_3$-$C_8$ cycloalkyl, -($C_1$-$C_4$ alkyl)$A^1$, or $A^1$, wherein said alkyl and cycloalkyl groups are optionally substituted by hydroxy, -$CO_2X^6$, -$CONX^6X^6$, -$NX^6X^6$, -$SO_m$($C_1$-$C_6$ alkyl), -$COA^1$, -$COX^6$, -$CF_3$, -CN, or from one to three independently selected halo atoms;

$R^3$ is selected from the group consisting of $A^1$, $C_1$-$C_{10}$ alkyl, -($C_1$-$C_6$ alkyl)$A^1$, -($C_1$-$C_6$ alkyl)$C_3$-$C_7$ cycloalkyl, -($C_1$-$C_5$ alkyl)$X^1$($C_1$-$C_5$ alkyl), -($C_1$-$C_5$ alkyl)$X^1$-($C_0$-$C_5$ alkyl)$A^1$ and -($C_1$-$C_5$ alkyl)$X^1$($C_1$-$C_5$ alkyl)$C_3$-$C_7$ cycloalkyl, wherein said alkyl groups are optionally substituted by -$SO_m$($C_1$-$C_6$ alkyl), -$CO_2X^3$, from one to five independently selected halo atoms, or from one to three independently selected -$OX^3$ groups;

$X^1$ is -O-, -$SO_m$-, -$NX^2$CO-, -$CONX^2$-, -OCO-, -$CO_2$-, -$CX^2$=$CX^2$-, -$NX^2CO_2$-, -$OCONX^2$-, or -C≡C-;

$R^4$ is hydrogen, $C_1$-$C_6$ alkyl, or $C_3$-$C_7$ cycloalkyl; or $R^4$ taken together with $R^3$ and the carbon atom to which they are attached form a $C_5$-$C_7$ cycloalkyl group, a $C_5$-$C_7$ cycloalkenyl group, a partially saturated or fully saturated 4- to 8-membered ring having from one to four heteroatoms independently selected from the group consisting of oxygen, sulfur and nitrogen, or a bicyclic ring system consisting of a partially saturated or fully saturated 5- or 6-membered ring fused to a partially saturated, fully unsaturated, or fully saturated 5- or 6-membered ring, optionally having from one to four heteroatoms independently selected from the group consisting of oxygen, sulfur, and nitrogen;

$X^4$ is hydrogen or $C_1$-$C_6$ alkyl; or $X^4$ taken together with $R^4$ and the nitrogen atom to which $X^4$ is attached and the carbon atom to which $R^4$ is attached form a 5- to 7-membered ring;

$R^6$ is a bond or is

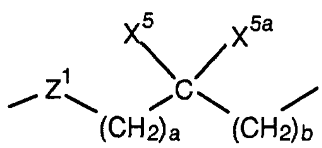

wherein a and b are each independently 0, 1, 2, or 3;

$X^5$ and $X^{5a}$ are each independently selected from the group consisting of hydrogen, -$CF_3$, $A^1$ and $C_1$-$C_6$ alkyl optionally substituted by $A^1$, -$OX^2$, -$SO_m$($C_1$-$C_6$ alkyl), -$CO_2X^2$, $C_3$-$C_7$ cycloalkyl, -$NX^2X^2$ and -$CONX^2X^2$; or the carbon bearing $X^5$ or $X^{5a}$ forms one or two alkylene bridges with the nitrogen atom bearing $R^7$ and $R^8$, wherein each alkylene bridge contains from one to five carbon atoms, provided that when one alkylene bridge is formed, then only one of $X^5$ or $X^{5a}$ is on the carbon atom and only one of $R^7$ or $R^8$ is on the nitrogen atom, and further provided that when two alkylene bridges are formed, then $X^5$ and $X^{5a}$ cannot be on the carbon atom and $R^7$ and $R^8$ cannot be on the nitrogen atom; or $X^5$ taken together with $X^{5a}$ and the carbon atom to which they are attached form a partially saturated or fully saturated 3- to 7-membered ring or a partially saturated or fully saturated 4- to 8-membered ring having from one to four heteroatoms independently selected from the group consisting of oxygen, sulfur and nitrogen; or $X^5$ taken together with $X^{5a}$ and the carbon atom to which they are attached form a bicyclic ring system consisting of a partially saturated or fully saturated 5- or 6-membered ring, optionally having one or two heteroatoms independently selected from the group consisting of nitrogen, sulfur and oxygen, fused to a partially saturated, fully saturated, or fully unsaturated 5- or 6-membered ring, optionally having from one to four heteroatoms independently selected from the group consisting of oxygen, sulfur and nitrogen;

$Z^1$ is a bond, -O-, or -$NX^2$-, provided that when a and b are both 0, then $Z^1$ is not -O- or -$NX^2$-;

$R^7$ and $R^8$ are each independently hydrogen or $C_1$-$C_6$ alkyl optionally independently substituted by $A^1$, $-CO_2(C_1$-$C_6$ alkyl), $-SO_m(C_1$-$C_6$ alkyl), from one to five halo atoms, from one to three hydroxy groups, from one to three $-OCO(C_1$-$C_{10}$ alkyl) groups, or from one to three $C_1$-$C_6$ alkoxy groups; or $R^7$ and $R^8$ can be taken together to form $-(CH_2)_rL(CH_2)_r-$, wherein L is $-CX^2X^2-$, $-SO_m-$, or $-NX^2-$;

$R^9$ and $R^{10}$ are each independently selected from the group consisting of hydrogen, fluoro, hydroxy and $C_1$-$C_5$ alkyl, optionally independently substituted by from one to five halo atoms;

$R^{11}$ is selected from the group consisting of $C_1$-$C_5$ alkyl and phenyl optionally substituted by from one to three substituents each independently selected from the group consisting of $C_1$-$C_5$ alkyl, halo and $C_1$-$C_5$ alkoxy;

$R^{12}$ is selected from the group consisting of $C_1$-$C_5$ alkylsulfonyl, $C_1$-$C_5$ alkanoyl and $C_1$-$C_5$ alkyl, wherein said alkyl groups are optionally independently substituted by from one to five halo atoms;

$A^1$ for each occurrence is independently selected from the group consisting of $C_5$-$C_7$ cycloalkenyl, phenyl, a partially saturated, fully saturated, or fully unsaturated 4- to 8-membered ring optionally having from one to four heteroatoms independently selected from the group consisting of oxygen, sulfur and nitrogen and a bicyclic ring system consisting of a partially saturated, fully unsaturated, or fully saturated 5- or 6-membered ring, optionally having from one to four heteroatoms independently selected from the group consisting of oxygen, sulfur and nitrogen, fused to a partially saturated, fully saturated, or fully unsaturated 5- or 6-membered ring, optionally having from one to four heteroatoms independently selected from the group consisting of oxygen, sulfur and nitrogen;

$A^1$ for each occurrence is independently optionally substituted on one or, if $A^1$ is a bicyclic ring system, both rings, with up to three substituents, each substituent independently selected from the group consisting of -F, -Cl, -Br, -I, $-OCF_3$, -OCF2H, $-CF_3$, $-CH_3$, $-OCH_3$, $-OX^6$, $-CONX^6X^6$, $-CO_2X^6$, oxo, $C_1$-$C_6$ alkyl, nitro, cyano, benzyl, $-SO_m(C_1$-$C_6$ alkyl), 1H-tetrazol-5-yl, phenyl, phenoxy, phenylalkyloxy, halophenyl, methylenedioxy, $-NX^6X^6$, $-NX^6COX^6$, $-SO_2NX^6X^6$, $-NX^6SO_2$phenyl, $NX^6SO_2X^6$, $-CONX^{11}X^{12}$, $-SO_2NX^{11}X^{12}$, $-NX^6SO_2X^{12}$, $-NX^6CONX^{11}X^{12}$, $-NX^6SO_2NX^{11}X^{12}$, $-NX^6COX^{12}$, imidazolyl, thiazolyl and tetrazolyl, provided that if $A^1$ is optionally substituted by methylenedioxy, then it can only be substituted with one methylenedioxy, wherein $X^{11}$ is hydrogen or $C_1$-$C_6$ alkyl optionally independently substituted by phenyl, phenoxy, $C_1$-$C_6$ alkoxycarbonyl, $-SO_m(C_1$-$C_6$ alkyl), from one to five halo atoms, from one to three hydroxy groups, from one to three $C_1$-$C_{10}$ alkanoyloxy groups, or from one to three $C_1$-$C_6$ alkoxy groups, and wherein $X^{12}$ is hydrogen, $C_1$-$C_6$ alkyl, phenyl, thiazolyl, imidazolyl, furyl, or thienyl, provided that when $X^{12}$ is not hydrogen, the $X^{12}$ group is optionally substituted by from one to three substituents independently selected from the group consisting of -Cl, -F, $-CH_3$, $-OCH_3$, $-OCF_3$ and $-CF_3$; or $X^{11}$ and $X^{12}$ are taken together to form $(CH_2)_rL^1(CH_2)_r-$, wherein $L^1$ is $-CX^2X^2-$, -O-, $-SO_m-$, or $-NX^2-$;

r for each occurrence is independently 1, 2, or 3;

$X^2$ for each occurrence is independently hydrogen, optionally substituted $C_1$-$C_6$ alkyl, or optionally substituted $C_3$-$C_7$ cycloalkyl, wherein said optionally substituted $C_1$-$C_6$ alkyl group and said optionally substituted $C_3$-$C_7$ cycloalkyl group are optionally independently substituted by $-SO_m(C_1$-$C_6$ alkyl), $-CO_2X^3$, from one to five halo atoms, or from one to three $-OX^3$ groups;

$X^3$ for each occurrence is independently hydrogen or $C_1$-$C_6$ alkyl;

$X^6$ for each occurrence is independently hydrogen, optionally substituted $C_1$-$C_6$ alkyl, halogenated $C_2$-$C_6$ alkyl, optionally substituted $C_3$-$C_7$ cycloalkyl, or halogenated $C_3$-$C_7$ cycloalkyl, wherein said optionally substituted $C_1$-$C_6$ alkyl group and said optionally substituted $C_3$-$C_7$ cycloalkyl group are optionally independently mono- or disubstituted with $C_1$-$C_4$ alkyl, hydroxy, $C_1$-$C_4$ alkoxy, carboxyl, $-CONH_2$, $-SO_m(C_1$-$C_6$ alkyl), $-CO_2(C_1$-$C_4$ alkyl), or 1H-tetrazol-5-yl; or when there are two $X^6$ groups on one atom and both $X^6$ are independently $C_1$-$C_6$ alkyl, the two $C_1$-$C_6$ alkyl groups may be optionally joined and, together with the atom to which the two $X^6$ groups are attached, form a 4- to 9-membered ring optionally having O, S, or $NX^7$ as a ring member, wherein $X^7$ is hydrogen or $C_1$-$C_6$ alkyl optionally substituted by hydroxy;

m for each occurrence is independently 0,1, or 2;

with the provisos that

(a) $X^6$ and $X^{12}$ cannot be hydrogen when attached to CO or $SO_2$ in the form $-COX^6$, $-COX^{12}$, $-SO_2X^6$, or $-SO_2X^{12}$; and

(b) when $R^6$ is a bond, then L is $-NX^2-$ and each r in the definition $-(CH_2)_rL(CH_2)_r-$ is independently 2 or 3.

**12.** A pharmaceutical composition according to claim 11 wherein said growth hormone secretagogue is

2-amino-N-[2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide;
2-amino-N-[1-(R)-(2,4-difluorobenzyloxymethyl)-2-oxo-2-(3-oxo-3a-(R)-pyridin-2-ylmethyl)-2-(2,2,2-trifluoroethyl)-2,3,3a,4,6,7-hexahydropyrazolo-[4,3-c]pyridin-5-yl)ethyl]-2-methylpropionamide;
2-amino-N-{1(R)-benzyloxymethyl-2-[1,3-dioxo-8a(S)-pyridin-2-ylmethyl-2-(2,2,2-trifluoroethyl)hexahydroimidazo[1,5-a]pyrazin-7-yl]-2-oxoethyl}-2-methylpropionamide;
N-(1(R)-{[1,2-dihydro-1-methanesulfonylspiro(3H-indole-3,4'-piperidin)-1'-yl]carbonyl}-2-(phenylmethyloxy)ethyl)-2-amino-2-methylpropanamide;

or a prodrug of any thereof or a pharmaceutically acceptable salt of any of said compounds or prodrugs.

**13.** A pharmaceutical composition according to any of Claims 1 to 12 for use as a medicament.

**14.** The use of a corticotrophin releasing factor antagonist in the manufacture of a medicament combined with a growth hormone secretagogue or growth hormone for the treatment or prevention of osteoporosis or frailty associated with aging or obesity.

**15.** The use of a corticotrophin releasing factor antagonist in the manufacture of a medicament combined with a growth hormone secretagogue or growth hormone for the treatment or prevention of a cardiovascular or heart-related disease.

**16.** The use of a corticotrophin releasing factor antagonist in the manufacture of a medicament combined with a growth hormone secretagogue or growth hormone for accelerating bone fracture repair, attenuating protein catabolic response after a major operation, reducing cachexia and protein loss due to chronic illness, accelerating wound healing, or accelerating the recovery of burn patients or of patients having undergone major surgery.